(19) **Europäisches Patentamt / European Patent Office / Office européen des brevets**

(11) **EP 1 696 960 B1**

(12) **EUROPEAN PATENT SPECIFICATION**

(45) Date of publication and mention
of the grant of the patent:
**08.09.2010 Bulletin 2010/36**

(51) Int Cl.:
***A61K 47/00*** (2006.01)   ***A61K 9/00*** (2006.01)

(21) Application number: **04815393.6**

(86) International application number:
**PCT/US2004/043312**

(22) Date of filing: **17.12.2004**

(87) International publication number:
**WO 2005/065714 (21.07.2005 Gazette 2005/29)**

(54) **INTRANASAL ADMINISTRATION OF GLUCOSE-REGULATING PEPTIDES**

INTRANASALE VERABREICHUNG VON GLUCOSE-REGULIERENDEN PEPTIDEN

ADMINISTRATION INTRANASALE DE PEPTIDES REGULANT LE GLUCOSE

(84) Designated Contracting States:
**AT BE BG CH CY CZ DE DK EE ES FI FR GB GR HU IE IS IT LI LT LU MC NL PL PT RO SE SI SK TR**

(30) Priority: **26.12.2003 US 532337 P**

(43) Date of publication of application:
**06.09.2006 Bulletin 2006/36**

(73) Proprietor: **Amylin Pharmaceuticals, Inc.**
**San Diego, CA 92121 (US)**

(72) Inventors:
• **QUAY, Steven, C.**
**Edmonds, WA 98026-9224 (US)**

• **COSTANTINO, Henry, R.**
**Woodinville, WA 98072 (US)**

(74) Representative: **MacLean, Martin Robert et al**
**Mathys & Squire LLP**
**120 Holborn**
**London**
**EC1N 2SQ (GB)**

(56) References cited:
**WO-A-01/15666       WO-A-01/54741**
**WO-A-93/18785       WO-A-2004/103396**
**US-A1- 2003 092 606**

EP 1 696 960 B1

SEQUENCE LISTING

<110> Quay. Steven C.
Costantino. Henry R.

<120> INTRANASAL ADMINISTRATION OF GLUCOSE-REGULATING PEPTIDES

<130> 03-14PCT

<150> 60/532.337
<151> 2003-12-26

<160> 47

<170> FastSEQ for Windows Version 4.0

<210> 1
<211> 39
<212> PRT
<213> Gila

<400> 1

His Ser Asp Gly Thr Phe Thr Ser Asp Leu Ser Lys Gln Met Glu Glu
1         5         10        15

Glu Ala Val Arg Leu Phe Ile Glu Trp Leu Lys Asn Gly Gly Pro Ser
20         25         30

Ser Gly Ala Pro Pro Pro Ser
35


<210> 2
<211> 39
<212> PRT
<213> Gila

<400> 2

His Gly Glu Gly Thr Phe Thr Ser Asp Leu Ser Lys Gln Met Glu Glu
1         5         10        15

Glu Ala Val Arg Leu Phe Ile Glu Trp Leu Lys Asn Gly Gly Pro Ser
20         25         30

Ser Gly Ala Pro Pro Pro Ser
35


<210> 3
<211> 30
<212> PRT
<213> Homo sapiens

<400> 3

His Ala Glu Gly Thr Phe Thr Ser Asp Val Ser Ser Tyr Leu Glu Gly


1         5         10        15

Gln Ala Ala Lys Glu Phe Ile Ala Trp Leu Val Lys Gly Arg
20         25         30


<210> 4
<211> 31
<212> PRT
<213> Homo sapiens

<400> 4

His Ala Glu Gly Thr Phe Thr Ser Asp Val Ser Ser Tyr Leu Glu Gly
1               5               10              15
Gln Ala Ala Lys Glu Phe Ile Ala Trp Leu Val Lys Gly Arg Gly
          20              25              30

<210> 5
<211> 37
<212> PRT
<213> Homo sapiens

<400> 5

Lys Cys Asn Thr Ala Thr Cys Ala Thr Gln Arg Leu Ala Asn Phe Leu
1               5               10              15
Val His Ser Ser Asn Asn Phe Gly Ala Ile Leu Ser Ser Thr Asn Val
          20              25              30
Gly Ser Asn Thr Tyr
      35

<210> 6
<211> 37
<212> PRT
<213> Homo sapiens

<400> 6

Lys Cys Asn Thr Ala Thr Cys Ala Thr Gln Arg Leu Ala Asn Phe Leu
1               5               10              15
Val His Ser Ser Asn Asn Phe Gly Ala Ile Leu Ser Ser Thr Asn Val
          20              25              30
Gly Ser Asn Thr Tyr
      35

<210> 7
<211> 37
<212> PRT
<213> Homo sapiens

<400> 7

Lys Cys Asn Thr Ala Thr Cys Ala Thr Gln Arg Leu Ala Asn Phe Leu
1               5               10              15

Ile Arg Ser Ser Asn Asn Leu Gly Ala Ile Leu Ser Pro Thr Asn Val
          20              25              30
Gly Ser Asn Thr Tyr
      35

<210> 8
<211> 37
<212> PRT
<213> Homo sapiens

<400> 8

Lys Cys Asn Thr Ala Thr Cys Ala Thr Gln Arg Leu Ala Asn Phe Leu
1               5               10              15
Val Arg Thr Ser Asn Asn Leu Gly Ala Ile Leu Ser Pro Thr Asn Val
            20              25              30
Gly Ser Asn Thr Tyr
            35

<210> 9
<211> 37
<212> PRT
<213> Homo sapiens

<400> 9

Lys Cys Asn Thr Ala Thr Cys Ala Thr Gln Arg Leu Ala Asn Phe Leu
1               5               10              15
Val Arg Ser Ser Asn Asn Leu Gly Pro Val Leu Pro Pro Thr Asn Val
            20              25              30
Gly Ser Asn Thr Tyr
            35

<210> 10
<211> 37
<212> PRT
<213> Homo sapiens

<400> 10

Lys Cys Asn Thr Ala Thr Cys Ala Thr Gln Arg Leu Ala Asn Phe Leu
1               5               10              15
Val His Ser Asn Asn Asn Leu Gly Pro Val Leu Ser Pro Thr Asn Val
            20              25              30
Gly Ser Asn Thr Tyr
            35

<210> 11
<211> 37
<212> PRT
<213> Homo sapiens

<400> 11

Lys Cys Asn Thr Ala Thr Cys Ala Thr Gln Arg Leu Thr Asn Phe Leu
1        5         10         15

Val Arg Ser Ser His Asn Leu Gly Ala Ala Leu Leu Pro Thr Asp Val
       20       25       30

Gly Ser Asn Thr Tyr
   35

<210> 12
<211> 36
<212> PRT
<213> Homo sapiens

<400> 12

Cys Asn Thr Ala Thr Cys Ala Thr Gln Arg Leu Ala Asn Phe Leu Val
1        5         10         15

His Ser Ser Asn Asn Phe Gly Ala Ile Leu Ser Ser Thr Asn Val Gly
       20       25       30

Ser Asn Thr Tyr
   35

<210> 13
<211> 37
<212> PRT
<213> Homo sapiens

<400> 13

Lys Cys Asn Thr Ala Thr Cys Ala Thr Gln Arg Leu Ala Asn Phe Leu
1        5         10         15

Val His Ser Ser Asn Asn Phe Gly Ala Ile Leu Pro Ser Thr Asn Val
       20       25       30

Gly Ser Asn Thr Tyr
   35

<210> 14
<211> 37
<212> PRT
<213> Homo sapiens

<400> 14

Lys Cys Asn Thr Ala Thr Cys Ala Thr Gln Arg Leu Ala Asn Phe Leu
1        5         10         15

Val His Ser Ser Asn Asn Phe Gly Pro Ile Leu Pro Pro Thr Asn Val
       20       25       30

Gly Ser Asn Thr Tyr
   35

<210> 15
<211> 37

<212> PRT
<213> Homo sapiens

<400> 15

Lys Cys Asn Thr Ala Thr Cys Ala Thr Gln Arg Leu Ala Asn Phe Leu
1               5               10              15
Val Arg Ser Ser Asn Asn Phe Gly Pro Ile Leu Pro Ser Thr Asn Val
            20              25              30
Gly Ser Asn Thr Tyr
            35

<210> 16
<211> 36
<212> PRT
<213> Homo sapiens

<400> 16

Cys Asn Thr Ala Thr Cys Ala Thr Gln Arg Leu Ala Asn Phe Leu Val
1               5               10              15
His Arg Ser Asn Asn Phe Gly Pro Ile Leu Pro Ser Thr Asn Val Gly
            20              25              30
Ser Asn Thr Tyr
            35

<210> 17
<211> 37
<212> PRT
<213> Homo sapiens

<400> 17

Lys Cys Asn Thr Ala Thr Cys Ala Thr Gln Arg Leu Ala Asn Phe Leu
1               5               10              15
Val His Ser Ser Asn Asn Phe Gly Pro Val Leu Pro Pro Thr Asn Val
            20              25              30
Gly Ser Asn Thr Tyr
            35

<210> 18
<211> 37
<212> PRT
<213> Homo sapiens

<400> 18

Lys Cys Asn Thr Ala Thr Cys Ala Thr Gln Arg Leu Ala Asn Phe Leu
1       5          10          15

Val Arg Ser Ser Asn Asn Phe Gly Pro Ile Leu Pro Pro Thr Asn Val
     20         25         30

Gly Ser Asn Thr Tyr
     35

<210> 19
<211> 36
<212> PRT
<213> Homo sapiens

<400> 19

Cys Asn Thr Ala Thr Cys Ala Thr Gln Arg Leu Ala Asn Phe Leu Val
1       5          10          15

Arg Ser Ser Asn Asn Phe Gly Pro Ile Leu Pro Pro Ser Asn Val Gly
     20         25         30

Ser Asn Thr Tyr
     35

<210> 20
<211> 36
<212> PRT
<213> Homo sapiens

<400> 20

Cys Asn Thr Ala Thr Cys Ala Thr Gln Arg Leu Ala Asn Phe Leu Val
1       5          10          15

His Ser Ser Asn Asn Phe Gly Pro Ile Leu Pro Pro Ser Asn Val Gly
     20         25         30

Ser Asn Thr Tyr
     35

<210> 21
<211> 37
<212> PRT
<213> Homo sapiens

<400> 21

Lys Cys Asn Thr Ala Thr Cys Ala Thr Gln Arg Leu Ala Asn Phe Leu
1       5          10          15

Val His Ser Ser Asn Asn Leu Gly Pro Val Leu Pro Pro Thr Asn Val
     20         25         30

Gly Ser Asn Thr Tyr
     35

<210> 22
<211> 37

<212> PRT
<213> Homo sapiens

<400> 22

Lys Cys Asn Thr Ala Thr Cys Ala Thr Gln Arg Leu Ala Asn Phe Leu
1               5                   10                  15
Val His Ser Ser Asn Asn Leu Gly Pro Val Leu Pro Ser Thr Asn Val
                20                  25                  30

Gly Ser Asn Thr Tyr
                35

<210> 23
<211> 36
<212> PRT
<213> Homo sapiens

<400> 23

Cys Asn Thr Ala Thr Cys Ala Thr Gln Arg Leu Ala Asn Phe Leu Val
1               5                   10                  15
His Ser Ser Asn Asn Leu Gly Pro Val Leu Pro Ser Thr Asn Val Gly
                20                  25                  30
Ser Asn Thr Tyr
                35

<210> 24
<211> 37
<212> PRT
<213> Homo sapiens

<400> 24

Lys Cys Asn Thr Ala Thr Cys Ala Thr Gln Arg Leu Ala Asn Phe Leu
1               5                   10                  15
Val Arg Ser Ser Asn Asn Leu Gly Pro Val Leu Pro Ser Thr Asn Val
                20                  25                  30
Gly Ser Asn Thr Tyr
                35

<210> 25
<211> 37
<212> PRT
<213> Homo sapiens

<400> 25

Lys Cys Asn Thr Ala Thr Cys Ala Thr Gln Arg Leu Ala Asn Phe Leu
1                5              10              15
Val Arg Ser Ser Asn Asn Leu Gly Pro Ile Leu Pro Pro Thr Asn Val
            20              25              30
Gly Ser Asn Thr Tyr
        35

<210> 26
<211> 37
<212> PRT
<213> Homo sapiens

<400> 26

Lys Cys Asn Thr Ala Thr Cys Ala Thr Gln Arg Leu Ala Asn Phe Leu

1                5              10              15
Val Arg Ser Ser Asn Asn Leu Gly Pro Ile Leu Pro Ser Thr Asn Val
            20              25              30
Gly Ser Asn Thr Tyr
        35

<210> 27
<211> 37
<212> PRT
<213> Homo sapiens

<400> 27

Lys Cys Asn Thr Ala Thr Cys Ala Thr Gln Arg Leu Ala Asn Phe Leu
1                5              10              15
Ile His Ser Ser Asn Asn Leu Gly Pro Ile Leu Pro Pro Thr Asn Val
            20              25              30
Gly Ser Asn Thr Tyr
        35

<210> 28
<211> 37
<212> PRT
<213> Homo sapiens

<400> 28

```
Lys Cys Asn Thr Ala Thr Cys Ala Thr Gln Arg Leu Ala Asn Phe Leu
1               5               10              15
Val Ile Ser Ser Asn Asn Phe Gly Pro Ile Leu Pro Pro Thr Asn Val
                20              25              30
Gly Ser Asn Thr Tyr
                35
```

<210> 29
<211> 36
<212> PRT
<213> Homo sapiens

<400> 29

```
Cys Asn Thr Ala Thr Cys Ala Thr Gln Arg Leu Ala Asn Phe Leu Ile
1               5               10              15
His Ser Ser Asn Asn Leu Gly Pro Ile Leu Pro Pro Thr Asn Val Gly
                20              25              30
Ser Asn Thr Tyr
                35
```

<210> 30
<211> 37
<212> PRT
<213> Homo sapiens

<400> 30

```
Lys Cys Asn Thr Ala Thr Cys Ala Thr Gln Arg Leu Ala Asn Phe Leu
1               5               10              15
Ile Arg Ser Ser Asn Asn Leu Gly Ala Ile Leu Ser Ser Thr Asn Val
                20              25              30
Gly Ser Asn Thr Tyr
                35
```

<210> 31
<211> 37
<212> PRT
<213> Homo sapiens

<400> 31

```
Lys Cys Asn Thr Ala Thr Cys Ala Thr Gln Arg Leu Ala Asn Phe Leu
1               5               10              15
Ile Arg Ser Ser Asn Asn Leu Gly Ala Val Leu Ser Pro Thr Asn Val
                20              25              30
Gly Ser Asn Thr Tyr
                35
```

<210> 32
<211> 37
<212> PRT

<213> Homo sapiens

<400> 32

Lys Cys Asn Thr Ala Thr Cys Ala Thr Gln Arg Leu Ala Asn Phe Leu
1               5               10              15
Ile Arg Ser Ser Asn Asn Leu Gly Pro Val Leu Pro Pro Thr Asn Val
            20              25              30
Gly Ser Asn Thr Tyr
        35


<210> 33
<211> 37
<212> PRT
<213> Homo sapiens

<400> 33

Lys Cys Asn Thr Ala Thr Cys Ala Thr Gln Arg Leu Thr Asn Phe Leu
1               5               10              15
Val His Ser Ser His Asn Leu Gly Ala Ala Leu Leu Pro Thr Asp Val
            20              25              30
Gly Ser Asn Thr Tyr
        35


<210> 34
<211> 37
<212> PRT
<213> Homo sapiens

<400> 34

Lys Cys Asn Thr Ala Thr Cys Ala Thr Gln Arg Leu Thr Asn Phe Leu
1               5               10              15
Val His Ser Ser His Asn Leu Gly Ala Ala Leu Ser Pro Thr Asp Val
            20              25              30
Gly Ser Asn Thr Tyr
        35


<210> 35
<211> 36
<212> PRT
<213> Homo sapiens

<400> 35

Cys Asn Thr Ala Thr Cys Ala Thr Gln Arg Leu Thr Asn Phe Leu Val
1               5               10              15
His Ser Ser His Asn Leu Gly Ala Val Leu Pro Ser Thr Asp Val Gly
            20              25              30
Ser Asn Thr Tyr
        35

<210> 36
<211> 37
<212> PRT
<213> Homo sapiens

<400> 36

```
                    Lys Cys Asn Thr Ala Thr Cys Ala Thr Gln Arg Leu Thr Asn Phe Leu
                    1           5           10          15
                    Val Arg Ser Ser His Asn Leu Gly Ala Ala Leu Ser Pro Thr Asp Val
                            20          25          30
                    Gly Ser Asn Thr Tyr
                            35
```

<210> 37
<211> 37
<212> PRT
<213> Homo sapiens

<400> 37

```
                    Lys Cys Asn Thr Ala Thr Cys Ala Thr Gln Arg Leu Thr Asn Phe Leu
                    1           5           10          15
                    Val Arg Ser Ser His Asn Leu Gly Ala Ile Leu Pro Pro Thr Asp Val
                            20          25          30
                    Gly Ser Asn Thr Tyr
                            35
```

<210> 38
<211> 37
<212> PRT
<213> Homo sapiens

<400> 38

```
                    Lys Cys Asn Thr Ala Thr Cys Ala Thr Gln Arg Leu Thr Asn Phe Leu
                    1           5           10          15
                    Val Arg Ser Ser His Asn Leu Gly Pro Ala Leu Pro Pro Thr Asp Val
                            20          25          30
                    Gly Ser Asn Thr Tyr
                            35
```

<210> 39
<211> 37
<212> PRT
<213> Homo sapiens

<400> 39

Lys Asp Asn Thr Ala Thr Lys Ala Thr Gln Arg Leu Ala Asn Phe Leu
1               5               10              15
Val His Ser Ser Asn Asn Phe Gly Ala Ile Leu Ser Ser Thr Asn Val
            20              25              30
Gly Ser Asn Thr Tyr
        35


<210> 40
<211> 37
<212> PRT
<213> Homo sapiens

<400> 40


Ala Cys Asn Thr Ala Thr Cys Ala Thr Gln Arg Leu Ala Asn Phe Leu
1               5               10              15
Val His Ser Ser Asn Asn Phe Gly Ala Ile Leu Ser Ser Thr Asn Val
            20              25              30
Gly Ser Asn Thr Tyr
        35


<210> 41
<211> 37
<212> PRT
<213> Homo sapiens

<400> 41


Ser Cys Asn Thr Ala Thr Cys Ala Thr Gln Arg Leu Ala Asn Phe Leu
1               5               10              15
Val His Ser Ser Asn Asn Phe Gly Ala Ile Leu Ser Ser Thr Asn Val




                    20              25              30
        Gly Ser Asn Thr Tyr
                35


<210> 42
<211> 37
<212> PRT
<213> Homo sapiens

<400> 42


Lys Cys Asn Thr Ala Thr Cys Ala Thr Gln Arg Leu Ala Asn Phe Leu
1               5               10              15
Val His Ser Ser Asn Asn Phe Gly Ala Ile Leu Ser Pro Thr Asn Val
            20              25              30
Gly Ser Asn Thr Tyr
        35

<210> 43
<211> 37
<212> PRT
<213> Homo sapiens

<400> 43

```
Lys Cys Asn Thr Ala Thr Cys Ala Thr Gln Arg Leu Ala Asn Phe Leu
1               5                   10                  15
Val His Ser Ser Asn Asn Phe Gly Pro Ile Leu Pro Ser Thr Asn Val
            20                  25                  30
Gly Ser Asn Thr Tyr
            35
```

<210> 44
<211> 36
<212> PRT
<213> Homo sapiens

<400> 44

```
Cys Asn Thr Ala Thr Cys Ala Thr Gln Arg Leu Ala Asn Phe Leu Val
1               5                   10                  15
His Ser Ser Asn Asn Phe Gly Pro Ile Leu Pro Ser Thr Asn Val Gly
            20                  25                  30
Ser Asn Thr Tyr
            35
```

<210> 45
<211> 36
<212> PRT
<213> Homo sapiens

<400> 45

```
Cys Asn Thr Ala Thr Cys Ala Thr Gln Arg Leu Ala Asn Phe Leu Val
1               5                   10                  15
His Ser Ser Asn Asn Phe Gly Pro Val Leu Pro Pro Ser Asn Val Gly
            20                  25                  30
Ser Asn Thr Tyr
            35
```

<210> 46
<211> 37
<212> PRT
<213> Homo sapiens

<400> 46

```
Lys Cys Asn Thr Ala Thr Cys Ala Thr Gln Arg Leu Ala Asn Phe Leu
1           5              10              15
Val His Ser Ser Asn Asn Phe Gly Ala Ile Leu Ser Ser Thr Asn Val
              20            25            30
Gly Ser Asn Thr Tyr
      35
```

<210> 47
<211> 37
<212> PRT
<213> Homo sapiens

<400> 47

```
Lys Cys Asn Thr Ala Thr Cys Ala Thr Gln Arg Leu Ala Asn Phe Leu
1           5              10              15
Val His Ser Ser Asn Asn Phe Gly Pro Ile Leu Pro Pro Thr Asn Val
              20            25            30
Gly Ser Asn Thr Tyr
      35
```

**Description**

Glucose-regulating peptides are a class of peptides that have been shown to have therapeutic potential in the treatment of insulin dependent diabetes mellitus (IDDM), gestational diabetes or non insulin-dependent diabetes mellitus (NIDDM), the treatment of obesity and the treatment of dyslipidemia. See U.S. Patent No. 6,506,724, U.S. Patent Application Publication No. 20030036504A1, European Patent No. EP1083924B1, International Patent Application Publication No. WO 98/30231A1 and International Patent Application No. WO 00/73331A2. These peptides include glucagons-like peptide, GLP, e.g. GLP-1, the exendins, especially exendin-4, also known as exenatide, and amylin peptides and amylin analogs such as pramlintide. However, to date these peptides have only been administered to humans by injection.

Thus, there is a need to develop modes of administration of these peptides other than by injection.

**Brief Description of the Drawings**

FIG. 1 TER before and after 1 hour incubation for Fluorescein-exenatide formulation #1 (formulation with transmucosal excipients) and #2 (saline formulation) compared to PBS control and Triton-X control.

FIG. 2 shows MTT data for Fluorescein-exenatide formulation #1 (formulation with transmucosal excipients) and #2 (saline formulation) compared to PBS control and Triton-X control.

FIG. 3 shows LDH data for Fluorescein -exenatide formulation #1 (formulation with transmucosal excipients) and #2 (saline formulation) compared to PBS control and Triton-X control.

FIG. 4 shows Permeation data for Fluorescein-exenatide formulation #1 (formulation with transmucosal excipients) and #2 (saline formulation).

**Description of the Invention**

The present invention fulfills the foregoing needs and satisfies additional objects and advantages by providing a composition as set forth in the claims, together with a corresponding therapeutic use as set forth in the claims. In certain aspects of the invention, the glucose-regulating peptide is delivered in formulations to the intranasal mucosa so that at least about 10%, preferably 15% most preferably 20% or more of the glucose-regulating peptide contained within the dose is delivered to the systemic circulation or in other words is bioavailable. The bioavailability of the GRP is the fraction of the dose that reaches the systemic system wherein if the drug is administered intravenously, the bioavailability is 100%. Preferably the glucose-regulating peptide is a pharmaceutically acceptable salt of exendin-4, pramlintide or GLP-1 and the mammal is a human. Pharmaceutically-acceptable salts include inorganic acid salts, organic amine salts, organic acid salts, alkaline earth metal salts and mixtures thereof. Suitable examples of pharmaceutically-acceptable salts include, but are not limited to, halide, glucosamine, alkyl glucosamine, sulfate, hydrochloride, carbonate, hydrobromide, N, N'-dibenzylethylene-diamine, triethanolamine, diethanolamine, trimethylamine, triethylamine, pyridine, picoline, dicyclohexylamine; phosphate, sulfate, sulfonate, benzoate, acetate, salicylate, lactate, tartate, citrate, mesylate, gluconate, tosylate, maleate, fumarate, stearate and mixtures thereof.

In another embodiment of the present invention, an intranasal glucose-regulating peptide formulation is with transmucosal excipients is provided that results in a permeation of the glucose-regulating peptide in an *in vitro* tissue permeation assay at least 10 fold, preferably at least 50 fold, most preferably 100 fold greater then the permeation of the glucose-regulating peptide when present in a saline formulation consisting of water, the glucose-regulating peptide, sodium chloride and a buffer, wherein both formulations have identical pHs and osmolarity, and where both formuations are tested under the same *in vitro* tissue permeation assay conditions. An example of a suitable *in vitro* tissue permeation assay is the "I*ncreased permeability of .Fluorescein -labeled Exenatide across a Cellular Barrier using Permeation Enhancers*" described in Example 12.

The present invention is also directed to an intranasal formulation of a glucose-regulating peptide that is substantially free of proteins or polypeptides that stabilize the formulation. In particular, the preferred formulation is free of such proteins as albumin, and collagen-derived proteins such as gelatin.

In other aspects of the present invention a transmucosal glucose-regulating peptide formulation is comprised of a glucose-regulating peptide, water and a solubilizing agent having a pH of 2-8. In a preferred embodiment, the solubilization agent is a cyclodextrin.

In another embodiment of the present invention a transmucosal glucose-regulating peptide formulation is comprised of a glucose-regulating peptide, water, a solubilizing agent, preferably a cyclodextrin, and at least one polyol, preferably 2 polyols. In alternate embodiments the formulation may contain one or all of the following: a chelating agent, a surface-acting agent and a buffering agent.

In another embodiment of the present invention the formulation is comprised of a glucose-regulating peptide, water, chelating agent and a solubilization agent.

In another embodiment of the present invention the formulation is comprised of a glucose-regulating peptide, water and a chelating agent having a pH of 2-8.

In another embodiment of the present invention the formulation is comprised of a glucose-regulating peptide, water, chelating agent and at least one polyol, such as mannitol, lactose or sorbito, and preferably two polyols. Additional embodiments may include one or more of the following: a surface-active agent, a solubilizing agent and a buffering agent.

In another embodiment of the present invention the formulation is comprised of a glucose-regulating peptide, water, and at least two polyols, such as lactose and sorbitol. Additional agents, which can be added to the formulation, include, but are not limited to, a solubilization agent, a chelating agent, one or more buffering agents and a surface-acting agent.

The enhancement of intranasal delivery of a glucose-regulating peptide agonist according to the methods and compositions of the invention allows for the effective pharmaceutical use of these agents to treat a variety of diseases such as diabetes and obesity in mammalian subjects.

The present invention fills this need by providing for a liquid or dehydrated glucose-regulating peptide formulation wherein the formulation is substantially free of a stabilizer that is a polypeptide or a protein. The liquid glucose-regulating peptide (GRP) formulation is comprised of water, GRP and at least one of the following additives selected from the group consisting of polyols, surface-active agents, solubilizing agents and chelating agents. The pH of the formulation is preferably 2 to about 8.0, referably 4.0 to about 6.0, most preferably about 4.5 $\pm$ 0.5.

Another embodiment of the present invention is an aqueous glucose-regulating formulation of the present invention is comprised of water, a glucose-regulating peptide, a polyol and a surface-active agent wherein the formulation has a pH of about 2 to about 8, and the formulation is substantially free of a stabilizer that is a protein or polypeptide.

Another embodiment of the present invention is an aqueous glucose-regulating peptide formulation comprised of water, glucose-regulating peptide, a polyol and a solubilizing agent wherein the formulation has a pH of about 2.0 to about 8, and the formulation is substantially free of a stabilizer that is a protein or polypeptide.

Another embodiment of the present invention is an aqueous glucose-regulating peptide formulation comprised of water, glucose-regulating peptide, a solubilizing agent and a surface-active agent wherein the formulation has a pH of about 2.0 to about 8, and the formulation is substantially free of a stabilizer that is a protein or polypeptide.

Another embodiment of the invention is a aqueous glucose-regulating peptide formulation comprised of water, a glucose-regulating peptide, a solubilizing agent, a polyol and a surface-active agent wherein the formulation has a pH of about 2.0 to about 8, and the formulation is substantially free of a stabilizer that is a protein or polypeptide.

In another aspect of the present invention, the stable aqueous formulation is dehydrated to produce a dehydrated glucose-regulating peptide formulation comprised of glucose-regulating peptide and at least one of the following additives selected from the group consisting of polyols, surface-active agents, solubilizing agents and chelating agents, wherein said dehydrated glucose-regulating peptide formulation is substantially free of a stabilizer that is a protein or polypeptide such as albumin, collagen or collagen-derived protein such as gelatin. The dehydration can be achieved by various means such as lyophilization, spray-drying, salt-induced precipitation and drying, vacuum drying, rotary evaporation, or supercritical $CO_2$ precipitation.

In one embodiment, the dehydrated glucose-regulating peptide is comprised of glucose-regulating peptide, a polyol and a solubilizing agent, wherein the formulation is substantially free of a stabilizer that is a polypeptide or a protein.

In another embodiment, the dehydrated glucose-regulating peptide formulation is comprised of a glucose-regulating peptide, a polyol, and a surface-active agent wherein the glucose-regulating peptide formulation is substantially free of a stabilizer that is a protein or polypeptide.

In another embodiment, the dehydrated glucose-regulating peptide formulation is comprised of a glucose-regulating peptide, a surface-active agent, and a solubilizing agent wherein the glucose-regulating peptide formulation is substantially free of a stabilizer that is a protein or polypeptide.

In another embodiment of the present invention, the dehydrated glucose-regulating peptide formulation is comprised of a glucose-regulating peptide, a polyol, a surface-active agent and a solubilizing agent wherein the glucose-regulating peptide formulation is substantially free of a stabilizer that is a protein or polypeptide.

Any solubilizing agent can be used but a preferred one is selected from the group consisting of hydroxypropyl-β-cyclodextran, sulfobutylether-β-cyclodextran, methyl-β-cyclodextrin and chitosan.

Generally a polyol is selected from the group consisting of lactose, sorbitol, trehalose, sucrose, mannitol, mannose and maltose and derivatives and homologs thereof.

A satisfactory surface-active agent is selected from the group consisting of L-α-phosphatidylcholine didecanoyl (DDPC), polysorbate 20 (Tween 20), polysorbate 80 (Tween 80), polyethylene glycol (PEG), cetyl alcohol, polyvinylpyrolidone (PVP), polyvinyl alcohol (PVA), lanolin alcohol, and sorbitan monooleate.

In a preferred formulation, the glucose-regulating peptide formulation is also comprised of a chelating agent such as ethylene diamine tetraacetic acid (EDTA) or ethylene glycol tetraacetic acid (EGTA). Also a preservative such as chlorobutanol or benzylkonium chloride can be added to the formulation to inhibit microbial growth.

The pH is generally regulated by a pH control agent such as a buffer system such as for example, sodium citrate and citric acid, or sodium tartarate and tartaric acid or, sodium phosphate monobasic and sodium phosphate dibasic, or sodium acetate and acetic acid or succinic acid and sodium hydroxide.

The present invention also comprehends a formulation wherein the concentration of the glucose-regulating peptide is 0.1-15.0 mg/mL, preferably 1.0 - 5.0 mg/mL and the pH of the aqueous solution is 2 - 8 preferably about 4.5 ± 0.5.

The present invention further includes glucose-regulating peptide formulation wherein the concentration of the polyol is between about 0.1% and 10% (w/v) and additionally wherein the concentration of the polyol is in the range from about 0.1% to about 3% (w/v).

The present invention also includes a formulation containing a surface-actve agent, wherein the concentration of the surface-active agent is between about 0.00001% and about 5%(w/v), preferably between about 0.0002% and about 0.1% (w/v).

The present invention also includes a formulation containing a solubilization agent, wherein the concentration of the solubilzation agent is 1% - 10% (w/v) more preferably 1% to 5% (w/v).

The finished solution can be filtered and freeze-dried, lyophilized, using methods well known to one of ordinary skill in the art, and by following the instructions of the manufacturer of the lyophilizing equipment. This produces a dehydrated glucose-regulating peptide formulation substantially free of a stabilizer that is a protein.

In a preferred embodiment, the glucose-regulating peptide formulation is further comprised of at least one excipient selected from the group consisting of a surface-active agent, a solubilization agent, a polyol, and a chelating agent. Preferably the glucose-regulating peptide is a amylin peptide, an GLP-1 or a exendin peptide.

In another embodiment of the present invention a glucose-regulating petide formulation is provided that is capable of raising the amount of the glucose-regulating peptide in the plasma of a mammal by at least 10, 20 40, 60, 80 or more pmoles per mL of plasma when 100 μL or less of the formulation is administered intranasally in a single administration to said mammal.

In exemplary embodiments, the uses and compositions of the present invention provide for therapeutically effective mucosal delivery of the glucose-regulating peptide for prevention or treatment of obesity and eating disorders in mammalian subjects. In one aspect of the invention, pharmaceutical formulations suitable for intranasal administration are provided that comprise a therapeutically effective amount of a glucose-regulating peptide and one or more intranasal delivery-enhancing agents as described herein, which formulations are effective in a nasal mucosal delivery method of the invention to prevent the onset or progression of obesity or eating disorders in a mammalian subject. Nasal mucosal delivery of a therapeutically effective amount of a glucose-regulating peptide agonist and one or more intranasal delivery-enhancing agents yields elevated therapeutic levels of the glucose-regulating peptide agonist in the subject.

The enhanced uses and compositions of the present invention provide for therapeutically effective mucosal delivery of a glucose-regulating peptide for prevention or treatment of a variety of diseases and conditions in mammalian subjects. glucose-regulating peptide can be administered via a variety of mucosal routes, for example by contacting the glucose-regulating peptide to a nasal mucosal epithelium, a bronchial or pulmonary mucosal epithelium, the oral buccal surface or the oral and small intestinal mucosal surface. In exemplary embodiments, the methods and compositions are directed to or formulated for intranasal delivery (e.g., nasal mucosal delivery or intranasal mucosal delivery).

The foregoing mucosal glucose-regulating peptide formulations, preparative methods and uses of the invention provide improved mucosal delivery of a glucose-regulating peptide to mammalian subjects. These compositions and uses can involve combinatorial formulation or coordinate administration of one or more glucose-regulating peptides with one or more mucosal delivery-enhancing agents. Among the mucosal delivery-enhancing agents to be selected from to achieve these formulations and methods are (A) solubilization agents; (B) charge modifying agents; (C) pH control agents; (D) degradative enzyme inhibitors; (E) mucolytic or mucus clearing agents; (F) ciliostatic agents; (G) membrane penetration-enhancing agents (e.g., (i) a surfactant, (ii) a bile salt, (iii) a phospholipid or fatty acid additive, mixed micelle, liposome, or carrier, (iv) an alcohol, (v) an enamine, (iv) an NO donor compound, (vii) a long-chain amphipathic molecule (viii) a small hydrophobic penetration enhancer; (ix) sodium or a salicylic acid derivative; (x) a glycerol ester of acetoacetic acid (xi) a cyclodextrin or beta-cyclodextrin derivative, (xii) a medium-chain fatty acid, (xiii) a chelating agent, (xiv) an amino acid or salt thereof, (xv) an N-acetylamino acid or salt thereof, (xvi) an enzyme degradative to a selected membrane component, (xvii) an inhibitor of fatty acid synthesis, (xviii) an inhibitor of cholesterol synthesis; or (xiv) any combination of the membrane penetration enhancing agents of (i)-(xviii)); (H) modulatory agents of epithelial junction physiology, such as nitric oxide (NO) stimulators, chitosan, and chitosan derivatives; (I) vasodilator agents; (J) selective transport-enhancing agents; and (K) stabilizing delivery vehicles, carriers, supports or complex-forming species with which the glucose-regulating peptide (s) is/are effectively combined, associated, contained, encapsulated or bound to stabilize the active agent for enhanced mucosal delivery.

In various embodiments of the invention, a glucose-regulating peptide is combined with one, two, three, four or more of the mucosal delivery-enhancing agents recited in (A)-(K), above. These mucosal delivery-enhancing agents may be admixed, alone or together, with the glucose-regulating peptide, or otherwise combined therewith in a pharmaceutically acceptable formulation or delivery vehicle. Formulation of a glucose-regulating peptide with one or more of the mucosal delivery-enhancing agents according to the teachings herein (optionally including any combination of two or more mucosal delivery-enhancing agents selected from (A)-(K) above) provides for increased bioavailability of the glucose-regulating binding peptide following delivery thereof to a mucosal surface of a mammalian subject.

The present invention further provides for the use of a glucose-regulating peptide for the production of medicament for the transmucosal, administration of a glucose-regulating peptide for treating hyperglycemia, diabetes mellitus, dyslipidemia, suppressing apetite, promoting weight loss, decreasing food intake, or treating obesity in a mammal.

A mucosally effective dose of a glucose-regulating peptide within the pharmaceutical formulations of the present invention comprises, for example, between about 0.001 pmol to about 100 pmol per kg body weight, between about 0.01 pmol to about 10 pmol per kg body weight, or between about 0.1 pmol to about 5 pmol per kg body weight. In further exemplary embodiments, dosage of amylin is between about 0.5 pmol to about 1.0 pmol per kg body weight. In a preferred embodiment an intranasal dose will range from 0.1-100 $\mu$g/kg, or about 7 - 7000 $\mu$g, more preferably 0.5 -10 $\mu$g/kg, or 35 to 700 $\mu$g. More specific doses the intranasal GRP will range from 20 $\mu$g, 50 $\mu$g, 100 $\mu$g, 150 $\mu$g, 200 $\mu$g to 400 $\mu$g. The pharmaceutical formulations of the present invention may be administered one or more times per day, or 3 times per week or once per week for between one week and at least 96 weeks or even for the life of the individual patient or subject. In certain embodiments, the pharmaceutical formulations of the invention are administered one or more times daily, two times daily, four times daily, six times daily, or eight times daily.

Intranasal delivery-enhancing agents are employed which enhance delivery of amylin into or across a nasal mucosal surface. For passively absorbed drugs, the relative contribution of paracellular and transcellular pathways to drug transport depends upon the pKa, partition coefficient, molecular radius and charge of the drug, the pH of the luminal environment in which the drug is delivered, and the area of the absorbing surface. The intranasal delivery-enhancing agent of the present invention may be a pH control agent. The pH of the pharmaceutical formulation of the present invention is a factor affecting absorption of amylin via paracellular and transcellular pathways to drug transport. In one embodiment, the pharmaceutical formulation of the present invention is pH adjusted to between about pH 2 to 8. In a further embodiment, the pharmaceutical formulation of the present invention is pH adjusted to between about pH 3.0 to 6.0. In a further embodiment, the pharmaceutical formulation of the present invention is pH adjusted to between about pH 4.0 to 6.0. Generally, the pH is 4.5 ±0.5.

As noted above, the present invention provides improved therapeutic uses and compositions for mucosal delivery of glucose-regulating peptide to mammalian subjects for treatment or prevention of a variety of diseases and conditions. Examples of appropriate mammalian subjects for treatment and prophylaxis according to the methods of the invention include, but are not restricted to, humans and non-human primates, livestock species, such as horses, cattle, sheep, and goats, and research and domestic species, including dogs, cats, mice, rats, guinea pigs, and rabbits.

In order to provide better understanding of the present invention, the following definitions are provided:

**Exendins and Exendin Agonists**

Exendins are peptides that were first isolated from the salivary secretions of the Gilamonster, a lizard found in Arizona, and the Mexican Beaded Lizard. Exendin-3 is present in the salivary secretions of *Heloderma horridum,* and exendin-4 is present in the salivary secretions of *Heloderma suspectum* [Eng, J., et al., J. Biol. Chem., 265:20259-62 (1990); Eng., J., et al., J. Biol. Chem., 267:7402-05 (1992)]. The exendins have some sequence similarity to several members of the glucagon-like peptide family, with the highest homology, 53%, being to GLP-1[7-36]NH$_2$ [Goke, et al., J. Biol. Chem., 268:19650-55, (1993)]. GLP-1[7-36]NH$_2$, also known as proglucagon[78-107] and most commonly as "GLP-1," has an insulinotropic effect, stimulating insulin secretion; GLP-1 also inhibits glucagon secretion [Orskov, et al., Diabetes, 42:658-61 (1993); D'Alessio, et al., J. Clin. Invest., 97:133-38 (1996)]. GLP-1 is reported to inhibit gastric emptying [Williams B, et al., J Clin Encocrinol Metab 81: (1): 327-32 (1996); Wettergren A, et al., Dig Dis Sci 38: (4): 665-73 (1993)], and gastric acid secretion. [Schjoldager B T, et al., Dig Dis Sci 34 (5): 703-8, (1989); O'Halloran D J, et al., J Endocrinol 126 (1): 169-73 (1990); Wettergren A, et al., Dig Dis Sci 38: (4): 665-73 (1993)]. GLP-1 [7-37], which has an additional glycine residue at its carboxy terminus, also stimulates insulin secretion in humans [Orskov, et al., Diabetes, 42:658-61 (1993)]. A transmembrane G-protein adenylate-cyclase-coupled receptor believed to be responsible for the insulinotropic effect of GLP-1 is reported to have been cloned from a .beta.-cell line [Thorens, Proc. Natl. Acad Sci. USA 89:8641-45 (1992)].

The present invention is directed to therapeutic uses for treating gestational diabetes mellitus comprising the intranasal administration of an exendin, for example:

Exendin-3

His Ser Asp Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser (SEQ ID NO:1),

or

Exenatide (exendin-4)

His Gly Glu Gly Thr Phe Thr Ser Asp Leu Ser Lys Gln Met Glu Glu Glu Ala Val Arg Leu Phe Ile Glu Trp Leu Lys Asn Gly Gly Pro Ser Ser Gly Ala Pro Pro Pro Ser wherein the C-terminus serine is amidated (SEQ ID NO: 2), or other compounds which effectively bind to the receptor at which exendin exerts its actions which are beneficial in the treatment of gestational diabetes mellitus. The use of exendin-3 and exendin-4 as insulinotrophic agents for the treatment of diabetes mellitus and the prevention of hyperglycemia has been disclosed in U.S. Pat. No. 5,424,286. Exendins have also been shown to be useful in the modulation of triglyceride levels and to treat dyslipidemia.

**Glucagon-like Peptides (GLP)**

The amino acid sequence of GLP-1 is given i.a. by Schmidt et al. (Diabetologia 28 704-707 (1985). Human GLP-1 is a 37 amino acid residue peptide originating from preproglucagon which is synthesised, i.a. in the L-cells in the distal ileum, in the pancreas and in the brain. Processing of preproglucagon to GLP-1(7-36)amide, GLP-1(7-37) and GLP-2 occurs mainly in the L-cells. Although the interesting pharmacological properties of GLP-1(7-37) and analogues thereof have attracted much attention in recent years only little is known about the structure of these molecules. The secondary structure of GLP-1 in micelles has been described by Thorton et al. (Biochemistry 33 3532-3539 (1994)), but in normal solution, GLP-1 is considered a very flexible molecule.

GLP-1 and analogues of GLP-1 and fragments thereof are useful i.a. in the treatment of Type 1 and Type 2 diabetes and obesity.

WO 87/06941 discloses GLP-1 fragments, including GLP-1(7-37), and functional derivatives thereof and to their use as an insulinotropic agent.

WO 90/11296 discloses GLP-1 fragments, including GLP-1(7-36), and functional derivatives thereof which have an insulinotropic activity which exceeds the insulinotropic activity of GLP-1(1-36) or GLP-1(1-37) and to their use as insulinotropic agents.

The amino acid sequence of GLP-1(7-36) is :

```
His-Ala-Glu-Gly-Thr-Phe-Thr-Ser-Asp-Val-Ser-

Ser-Tyr-Leu-Glu-Gly-Gln-Ala-Ala-Lys-Glu-Phe-

Ile-Ala-Trp-Leu-Val-Lys-Gly-Arg (SEQ ID NO: 3)
```

and GLP-1(7-37) is

```
His-Ala-Glu-Gly-Thr-Phe-Thr-Ser-Asp-Val-Ser-

Ser-Tyr-Leu-Glu-Gly-Gln-Ala-Ala-Lys-Glu-Phe-

Ile-Ala-Trp-Leu-Val-Lys-Gly-Arg-Gly (SEQ ID NO: 4)
```

WO 91/11457 discloses analogues of the active GLP-1 peptides 7-34, 7-35, 7-36, and 7-37 which can also be useful as GLP-1 moieties.

EP 0708179-A2 (Eli Lilly & Co.) discloses GLP-1 analogues and derivatives that include an N-terminal imidazole group and optionally an unbranched $C_6$ -$C_{10}$ acyl group in attached to the lysine residue in position 34.

EP 0699686-A2 (Eli Lilly & Co.) discloses certain N-terminal truncated fragments of GLP-1 that are reported to be biologically active.

**Amylin Peptides**

KCNTATCATQRLANFLVHSSNNFGAILSSTNVGSNTY (SEQ ID NO: 5)

Agonists of amylin include:

```
Lys Cys Asn Thr Ala Thr Cys Ala Thr Gln Arg Leu Ala Asn Phe Leu
Val His Ser Ser Asn Asn Phe Gly Ala Ile Leu Ser Ser Thr Asn Val
 Gly Ser Asn Thr Tyr (SEQ ID NO: 6);
```

```
Lys Cys Asn Thr Ala Thr Cys Ala Thr Gln Arg Leu Ala Asn Phe Leu
Ile Arg Ser Ser Asn Asn Leu Gly Ala Ile Leu Ser Pro Thr Asn Val
Gly Ser Asn Thr Tyr (SEQ ID NO: 7);
```

```
Lys Cys Asn Thr Ala Thr Cys Ala Thr Gln Arg Leu Ala Asn Phe Leu
Val Arg Thr Ser Asn Asn Leu Gly Ala Ile Leu Ser Pro Thr Asn Val
Gly Ser Asn Thr Tyr (SEQ ID NO: 8);
```

```
Lys Cys Asn Thr Ala Thr Cys Ala Thr Gln Arg Leu Ala Asn Phe Leu
Val Arg Ser Ser Asn Asn Leu Gly Pro Val Leu Pro Pro Thr Asn Val
Gly Ser Asn Thr Tyr (SEQ ID NO: 9);
```

```
Lys Cys Asn Thr Ala Thr Cys Ala Thr Gln Arg Leu Ala Asn Phe Leu
Val His Ser Asn Asn Asn Leu Gly Pro Val Leu Ser Pro Thr Asn Val
Gly Ser Asn Thr Tyr (SEQ ID NO: 10);
```

```
Lys Cys Asn Thr Ala Thr Cys Ala Thr Gln Arg Leu Thr Asn Phe Leu
Val Arg Ser Ser His Asn Leu Gly Ala Ala Leu Leu Pro Thr Asp Val
Gly Ser Asn Thr Tyr (SEQ ID NO: 11);
```

Cys Asn Thr Ala Thr Cys Ala Thr Gln Arg Leu Ala Asn Phe Leu Val
His Ser Ser Asn Asn Phe Gly Ala Ile Leu Ser Ser Thr Asn Val Gly
Ser Asn Thr Tyr (SEQ ID NO: 12);

Lys Cys Asn Thr Ala Thr Cys Ala Thr Gln Arg Leu Ala Asn Phe Leu
Val His Ser Ser Asn Asn Phe Gly Ala Ile Leu Pro Ser Thr Asn Val
Gly Ser Asn Thr Tyr (SEQ ID NO: 13);

Lys Cys Asn Thr Ala Thr Cys Ala Thr Gln Arg Leu Ala Asn Phe Leu
Val His Ser Ser Asn Asn Phe Gly Pro Ile Leu Pro Pro Thr Asn Val
Gly Ser Asn Thr Tyr (SEQ ID NO: 14);

Lys Cys Asn Thr Ala Thr Cys Ala Thr Gln Arg Leu Ala Asn Phe Leu
Val Arg Ser Ser Asn Asn Phe Gly Pro Ile Leu Pro Ser Thr Asn Val
Gly Ser Asn Thr Tyr (SEQ ID NO: 15);

Cys Asn Thr Ala Thr Cys Ala Thr Gln Arg Leu Ala Asn Phe Leu Val
His Arg Ser Asn Asn Phe Gly Pro Ile Leu Pro Ser Thr Asn Val
Gly Ser Asn Thr Tyr (SEQ ID NO: 16);

Lys Cys Asn Thr Ala Thr Cys Ala Thr Gln Arg Leu Ala Asn Phe Leu
Val His Ser Ser Asn Asn Phe Gly Pro Val Leu Pro Pro Thr Asn Val
Gly Ser Asn Thr Tyr (SEQ ID NO: 17);

8

Lys Cys Asn Thr Ala Thr Cys Ala Thr Gln Arg Leu Ala Asn Phe Leu

Val Arg Ser Ser Asn Asn Phe Gly Pro Ile Leu Pro Pro Thr Asn Val

Gly Ser Asn Thr Tyr (SEQ ID NO: 18);

Cys Asn Thr Ala Thr Cys Ala Thr Gln Arg Leu Ala Asn Phe Leu Val

Arg Ser Ser Asn Asn Phe Gly Pro Ile Leu Pro Pro Ser Asn Val Gly

Ser Asn Thr Tyr (SEQ ID NO: 19);

Cys Asn Thr Ala Thr Cys Ala Thr Gln Arg Leu Ala Asn Phe Leu Val

His Ser Ser Asn Asn Phe Gly Pro Ile Leu Pro Pro Ser Asn Val Gly

Ser Asn Thr Tyr (SEQ ID NO: 20);

Lys Cys Asn Thr Ala Thr Cys Ala Thr Gln Arg Leu Ala Asn Phe Leu

Val His Ser Ser Asn Asn Leu Gly Pro Val Leu Pro Pro Thr Asn Val

Gly Ser Asn Thr Tyr (SEQ ID NO: 21);

Lys Cys Asn Thr Ala Thr Cys Ala Thr Gln Arg Leu Ala Asn Phe Leu

Val His Ser Ser Asn Asn Leu Gly Pro Val Leu Pro Ser Thr Asn Val

Gly Ser Asn Thr Tyr (SEQ ID NO: 22);

Cys Asn Thr Ala Thr Cys Ala Thr Gln Arg Leu Ala Asn Phe Leu Val

His Ser Ser Asn Asn Leu Gly Pro Val Leu Pro Ser Thr Asn Val Gly

Ser Asn Thr Tyr (SEQ ID NO: 23);

Lys Cys Asn Thr Ala Thr Cys Ala Thr Gln Arg Leu Ala Asn Phe Leu
Val Arg Ser Ser Asn Asn Leu Gly Pro Val Leu Pro Ser Thr Asn Val
Gly Ser Asn Thr Tyr (SEQ ID NO: 24);

Lys Cys Asn Thr Ala Thr Cys Ala Thr Gln Arg Leu Ala Asn Phe Leu
Val Arg Ser Ser Asn Asn Leu Gly Pro Ile Leu Pro Pro Thr Asn Val
Gly Ser Asn Thr Tyr (SEQ ID NO: 25);

Lys Cys Asn Thr Ala Thr Cys Ala Thr Gln Arg Leu Ala Asn Phe Leu
Val Arg Ser Ser Asn Asn Leu Gly Pro Ile Leu Pro Ser Thr Asn Val
Gly Ser Asn Thr Tyr (SEQ ID NO: 26);

Lys Cys Asn Thr Ala Thr Cys Ala Thr Gln Arg Leu Ala Asn Phe Leu
Ile His Ser Ser Asn Asn Leu Gly Pro Ile Leu Pro Pro Thr Asn Val
Gly Ser Asn Thr Tyr (SEQ ID NO: 27);

Lys Cys Asn Thr Ala Thr Cys Ala Thr Gln Arg Leu Ala Asn Phe Leu
Val Ile Ser Ser Asn Asn Phe Gly Pro Ile Leu Pro Pro Thr Asn Val
Gly Ser Asn Thr Tyr (SEQ ID NO: 28);

Cys Asn Thr Ala Thr Cys Ala Thr Gln Arg Leu Ala Asn Phe Leu Ile
His Ser Ser Asn Asn Leu Gly Pro Ile Leu Pro Pro Thr Asn Val Gly
Ser Asn Thr Tyr (SEQ ID NO: 29);

Lys Cys Asn Thr Ala Thr Cys Ala Thr Gln Arg Leu Ala Asn Phe Leu
Ile Arg Ser Ser Asn Asn Leu Gly Ala Ile Leu Ser Ser Thr Asn Val
Gly Ser Asn Thr Tyr (SEQ ID NO: 30);

Lys Cys Asn Thr Ala Thr Cys Ala Thr Gln Arg Leu Ala Asn Phe Leu
Ile Arg Ser Ser Asn Asn Leu Gly Ala Val Leu Ser Pro Thr Asn Val
Gly Ser Asn Thr Tyr (SEQ ID NO: 31);

Lys Cys Asn Thr Ala Thr Cys Ala Thr Gln Arg Leu Ala Asn Phe Leu
Ile Arg Ser Ser Asn Asn Leu Gly Pro Val Leu Pro Pro Thr Asn Val
Gly Ser Asn Thr Tyr (SEQ ID NO: 32);

Lys Cys Asn Thr Ala Thr Cys Ala Thr Gln Arg Leu Thr Asn Phe Leu
Val His Ser Ser His Asn Leu Gly Ala Ala Leu Leu Pro Thr Asp Val
Gly Ser Asn Thr Tyr (SEQ ID NO: 33);

Lys Cys Asn Thr Ala Thr Cys Ala Thr Gln Arg Leu Thr Asn Phe Leu
Val His Ser Ser His Asn Leu Gly Ala Ala Leu Ser Pro Thr Asp Val
Gly Ser Asn Thr Tyr (SEQ ID NO: 34);

Cys Asn Thr Ala Thr Cys Ala Thr Gln Arg Leu Thr Asn Phe Leu Val
His Ser Ser His Asn Leu Gly Ala Val Leu Pro Ser Thr Asp Val Gly
Ser Asn Thr Tyr (SEQ ID NO: 35);

Lys Cys Asn Thr Ala Thr Cys Ala Thr Gln Arg Leu Thr Asn Phe Leu
Val Arg Ser Ser His Asn Leu Gly Ala Ala Leu Ser Pro Thr Asp Val
Gly Ser Asn Thr Tyr (SEQ ID NO: 36);

Lys Cys Asn Thr Ala Thr Cys Ala Thr Gln Arg Leu Thr Asn Phe Leu
Val Arg Ser Ser His Asn Leu Gly Ala Ile Leu Pro Pro Thr Asp Val
Gly Ser Asn Thr Tyr (SEQ ID NO: 37);

Lys Cys Asn Thr Ala Thr Cys Ala Thr Gln Arg Leu Thr Asn Phe Leu

Val Arg Ser Ser His Asn Leu Gly Pro Ala Leu Pro Pro Thr Asp Val

Gly Ser Asn Thr Tyr (SEQ ID NO: 38);

Lys Asp Asn Thr Ala Thr Lys Ala Thr Gln Arg Leu Ala Asn Phe Leu

Val His Ser Ser Asn Asn Phe Gly Ala Ile Leu Ser Ser Thr Asn Val

Gly Ser Asn Thr Tyr (SEQ ID NO: 39);

Ala Cys Asn Thr Ala Thr Cys Ala Thr Gln Arg Leu Ala Asn Phe Leu

Val His Ser Ser Asn Asn Phe Gly Ala Ile Leu Ser Ser Thr Asn Val

Gly Ser Asn Thr Tyr (SEQ ID NO: 40);

Ser Cys Asn Thr Ala Thr Cys Ala Thr Gln Arg Leu Ala Asn Phe Leu

Val His Ser Ser Asn Asn Phe Gly Ala Ile Leu Ser Ser Thr Asn Val

Gly Ser Asn Thr Tyr (SEQ ID NO: 41);

Lys Cys Asn Thr Ala Thr Cys Ala Thr Gln Arg Leu Ala Asn Phe Leu

Val His Ser Ser Asn Asn Phe Gly Ala Ile Leu Ser Pro Thr Asn Val

Gly Ser Asn Thr Tyr (SEQ ID NO: 42);

Lys Cys Asn Thr Ala Thr Cys Ala Thr Gln Arg Leu Ala Asn Phe Leu

Val His Ser Ser Asn Asn Phe Gly Pro Ile Leu Pro Ser Thr Asn Val

Gly Ser Asn Thr Tyr (SEQ ID NO: 43);

Cys Asn Thr Ala Thr Cys Ala Thr Gln Arg Leu Ala Asn Phe Leu Val

His Ser Ser Asn Asn Phe Gly Pro Ile Leu Pro Ser Thr Asn Val Gly

Ser Asn Thr Tyr (SEQ ID NO: 44);

Cys Asn Thr Ala Thr Cys Ala Thr Gln Arg Leu Ala Asn Phe Leu Val

His Ser Ser Asn Asn Phe Gly Pro Val Leu Pro Pro Ser Asn Val Gly

Ser Asn Thr Tyr (SEQ ID NO: 45)

Lys Cys Asn Thr Ala Thr Cys Ala Thr Gln Arg Leu Ala Asn Phe Leu

Val His Ser Ser Asn Asn Phe Gly Ala Ile Leu Ser Ser Thr Asn Val

Gly Ser Asn Thr Tyr (SEQ ID NO: 46); and

Lys Cys Asn Thr Ala Thr Cys Ala Thr Gln Arg Leu Ala Asn Phe Leu

Val His Ser Ser Asn Asn Phe Gly Pro Ile Leu Pro Pro Thr Thr Asn

Val Gly Ser Asn Thr Tyr (SEQ ID NO: 47),

wherein the C-terminus tyrosine of SEQ ID NO: 47 is amidated. The amidated SEQ ID NO: 47 is also called pramlintide acetate. Pramlintide acetate also has a disulfide bond between the cysteines at positions 2 and 7.

According to the present invention the glucose-regulating peptides also include the free bases, acid addition salts or metal salts, such as potassium or sodium salts of the peptides, and amylin peptides that have been modified by such processes as amidation, glycosylation, acylation, sulfation, phosphorylation, acetylation, cyclization and other well known covalent modification methods.

Thus, according to the present invention, the above-described peptides are incorporated into formulations suitable for transmucosal delivery, especially intranasal delivery.

### Mucosal Delivery Enhancing Agents

"Mucosal delivery enhancing agents" are defined as chemicals and other excipients that, when added to a formulation comprising water, salts and/or common buffers and glucose-regulating peptide (the control formulation) produce a formulation that produces a significant increase in transport of glucose-regulating peptide across a mucosa as measured by the maximum blood, serum, or cerebral spinal fluid concentration ($C_{max}$) or by the area under the curve, AUC, in a plot of concentration versus time. A mucosa includes the nasal, oral, intestional, buccal, bronchopulmonary, vaginal, and rectal mucosal surfaces and in fact includes all mucus-secreting membranes lining all body cavities or passages that communicate with the exterior. Mucosal delivery enhancing agents are sometimes called carriers.

### Endotoxin-free formulation

"Endotoxin-free formulation" means a formulation which contains a glucose-regulating peptide and one or more mucosal delivery enhancing agents that is substantially free of endotoxins and/or related pyrogenic substances. Endotoxins include toxins that are confined inside a microorganism and are released only when the microorganisms are broken down or die. Pyrogenic substances include fever-inducing, thermostable substances (glycoproteins) from the outer membrane of bacteria and other microorganisms. Both of these substances can cause fever, hypotension and shock if administered to humans. Producing formulations that are endotoxin-free can require special equipment, expert artisians, and can be significantly more expensive than making formulations that are not endotoxin-free. Because intravenous administration of GLP or amylin simultaneously with infusion of endotoxin in rodents has been shown to prevent the hypotension and even death associated with the administration of endotoxin alone (US Patent 4,839,343), producing endotoxin-free formulations of these therapeutic agents would not be expected to be necessary for non-parental (non-injected) administration.

## Non-infused Administration

"Non-infused administration" means any method of delivery that does not involve an injection directly into an artery or vein, a method which forces or drives (typically a fluid) into something and especially to introduce into a body part by means of a needle, syringe or other invasive method. Non-infused administration includes subcutaneous injection, intramuscular injection, intraparitoneal injection and the non-injection methods of delivery to a mucosa.

Improved methods and compositions for mucosal administration of glucose-regulating peptide to mammalian subjects optimize glucose-regulating peptide dosing schedules. The present invention provides mucosal delivery of glucose-regulating peptide formulated with one or more mucosal delivery-enhancing agents wherein glucose-regulating peptide dosage release is substantially normalized and/or sustained for an effective delivery period of glucose-regulating peptide release ranges from approximately 0.1 to 2.0 hours; 0.4 to 1.5 hours; 0.7 to 1.5 hours; or 0.8 to 1.0 hours; following mucosal administration. The sustained release of glucose-regulating peptide achieved may be facilitated by repeated administration of exogenous gtucosc-regulating peptides utilizing compositions of the present invention.

Improved compositions and therapeutic uses for mucosal administration of glucose-regulating peptide to mammalian subjects optimize glucose-regulating peptide dosing schedules. The present invention provides improved mucosal (e.g., nasal) delivery of a formulation comprising glucose-regulating peptide in combination with one or more mucosal delivery-enhancing agents and an optional sustained release-enhancing agent or agents. Mucosal delivery-enhancing agents of the present invention yield an effective increase in delivery, e.g., an increase in the maximal plasma concentration ($C_{max}$) to enhance the therapeutic activity of mucosally-administered glucose-regulating peptide. A second factor affecting therapeutic activity of glucose-regulating peptide in the blood plasma and CNS is residence time (RT). Sustained release-enhancing agents, in combination with intranasal delivery-enhancing agents, increase $C_{max}$ and increase residence time (RT) of glucose-regulating peptide. Polymeric delivery vehicles and other agents and methods of the present invention that yield sustained release-enhancing formulations, for example, polyethylene glycol (PEG), are disclosed herein. The present invention provides an improved glucose-regulating peptide dosage form for treatment of symptoms related to obesity, colon cancer, exendin cancer, or breast cancer in mammalian subjects.

Within the mucosal delivery formulations and therapeutic uses of the invention, the glucose-regulating peptide is frequently combined or coordinately administered with a suitable carrier or vehicle for mucosal delivery. As used herein, the term "carrier" means a pharmaceutically acceptable solid or liquid filler, diluent or encapsulating material. A water-containing liquid carrier can contain pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents or other biocompatible materials. A tabulation of ingredients listed by the above categories, can be found in the U.S Pharmacopeia National Formulary, 1857-1859, (1990). Some examples of the materials which can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen free water, isotonic saline; Ringer's solution, ethyl alcohol and phosphate buffer solutions, as well as other non toxic compatible substances used in pharmaceutical formulations. Wetting agents, emulsifiers and lubricants such as sodium lauryl sulfate and magnesium stearate, as well as coloring agents, release agents, coating agents, sweetening, flavoring and perfuming agents, preservatives and antioxidants can also be present in the compositions, according to the desires of the formulator. Examples of pharmaceutically acceptable antioxidants include water soluble antioxidants such as ascorbic acid, cysteine hydrochloride, sodium bisulfite, sodium metabisulfite, sodium sulfite and the like; oil-soluble antioxidants such as ascorbyl palmitate, butylated hydroxyanisole (BHA), butylated hydroxytoluene (BHT), lecithin, propyl gallate, alpha-tocopherol and the like; and metal-chelating agents such as citric acid, ethylenediamine tetraacetic acid (EDTA), sorbitol, tartaric acid, phosphoric acid and the like. The amount of active ingredient that can be combined with the carrier materials to produce a single dosage form will vary depending upon the particular mode of administration.

Within the mucosal delivery compositions and therapeutic uses of the invention, various delivery-enhancing agents are employed which enhance delivery of glucose-regulating peptide into or across a mucosal surface. In this regard, delivery of glucose-regulating peptide across the mucosal epithelium can occur "transcellularly" or "paracellularly". The extent to which these pathways contribute to the overall flux and bioavailability of the glucose-regulating peptide depends upon the environment of the mucosa, the physico-chemical properties the active agent, and on the properties of the mucosal epithelium. Paracellular transport involves only passive diffusion, whereas transcellular transport can occur by passive, facilitated or active processes. Generally, hydrophilic, passively transported, polar solutes diffuse through the paracellular route, while more lipophilic solutes use the transcellular route. Absorption and bioavailability (e.g., as reflected by a permeability coefficient or physiological assay), for diverse, passively and actively absorbed solutes, can be readily evaluated, in terms of both paracellular and transcellular delivery components, for any selected glucose-regulating peptide within the invention. For passively absorbed drugs, the relative contribution of paracellular and transcellular pathways to drug transport depends upon the pKa, partition coefficient, molecular radius and charge of the drug, the pH of the luminal environment in which the drug is delivered, and the area of the absorbing surface. The paracellular route represents a relatively small fraction of accessible surface area of the nasal mucosal epithelium. In general terms, it has been reported that cell membranes occupy a mucosal surface area that is a thousand times greater than the area occupied by the paracellular spaces. Thus, the smaller accessible area, and the size- and charge-based discrimination against macromolecular permeation would suggest that the paracellular route would be a generally less favorable route than transcellular delivery for drug transport. Surprisingly, the methods and compositions of the invention provide for significantly enhanced transport of biotherapeutics into and across mucosal epithelia via the paracellular route. Therefore, the methods and compositions of the invention successfully target both paracellular and transcellular routes, alternatively or within a single method or composition.

As used herein, "mucosal delivery-enhancing agents" include agents which enhance the release or solubility (e.g., from a formulation delivery vehicle), diffusion rate, penetration capacity and timing, uptake, residence time, stability, effective half life, peak or sustained concentration levels, clearance and other desired mucosal delivery characteristics (e.g., as measured at the site of delivery, or at a selected target site of activity such as the bloodstream or central nervous system) of glucose-regulating peptide or other biologically active compound(s). Enhancement of mucosal delivery can thus occur by any of a variety of mechanisms, for example by increasing the diffusion, transport, persistence or stability of glucose-regulating peptide, increasing membrane fluidity, modulating the availability or action of calcium and other ions that regulate intracellular or paracellular permeation, solubilizing mucosal membrane components (e.g., lipids), changing non-protein and protein sulfhydryl levels in mucosal tissues, increasing water flux across the mucosal surface, modulating epithelial junctional physiology, reducing the viscosity of mucus overlying the mucosal epithelium, reducing mucociliary clearance rates, and other mechanisms.

As used herein, a "mucosally effective amount of glucose-regulating peptide" contemplates effective mucosal delivery of glucose-regulating peptide to a target site for drug activity in the subject that may involve a variety of delivery or transfer routes. For example, a given active agent may find its way through clearances between cells of the mucosa and reach an adjacent vascular wall, while by another route the agent may, either passively or actively, be taken up into mucosal cells to act within the cells or be discharged or transported out of the cells to reach a secondary target site, such as the systemic circulation. The methods and compositions of the invention may promote the translocation of active agents along one or more such alternate routes, or may act directly on the mucosal tissue or proximal vascular tissue to promote absorption or penetration of the active agent(s). The promotion of absorption or penetration in this context is not limited to these mechanisms.

As used herein "peak concentration ($C_{max}$) of glucose-regulating peptide in a blood plasma", "area under concentration vs. time curve (AUC) of glucose-regulating peptide in a blood plasma", "time to maximal plasma concentration ($t_{max}$) of glucose-regulating peptide in a blood plasma" are pharmacokinetic parameters known to one skilled in the art. Laursen et al., Eur. J. Endocrinology, 135: 309-315, 1996. The "concentration vs. time curve" measures the concentration of glucose-regulating peptide in a blood serum of a subject vs. time after administration of a dosage of glucose-regulating peptide to the subject either by intranasal, intramuscular, subcutaneous, or other parenteral route of administration. "$C_{max}$" is the maximum concentration of glucose-regulating peptide in the blood serum of a subject following a single dosage of glucose-regulating peptide to the subject. "$t_{max}$" is the time to reach maximum concentration of glucose-regulating peptide in a blood serum of a subject following administration of a single dosage of glucose-regulating peptide to the subject.

As used herein, "area under concentration vs. time curve (AUC) of glucose-regulating peptide in a blood plasma" is calculated according to the linear trapezoidal rule and with addition of the residual areas. A decrease of 23% or an increase of 30% between two dosages would be detected with a probability of 90% (type II error $\beta$ = 10%). The "delivery rate" or "rate of absorption" is estimated by comparison of the time ($t_{max}$) to reach the maximum concentration ($C_{max}$). Both $C_{max}$ and $t_{max}$ are analyzed using non-parametric methods. Comparisons of the pharmacokinetics of intramuscular, subcutaneous, intravenous and intranasal glucose-regulating peptide administrations were performed by analysis of variance (ANOVA). For pair wise comparisons a Bonferroni-Holmes sequential procedure is used to evaluate significance. The dose-response relationship between the three nasal doses is estimated by regression analysis. $P < 0.05$ is considered significant. Results are given as mean values +/- SEM.

While the mechanism of absorption promotion may vary with different mucosal delivery-enhancing agents of the invention, useful reagents in this context will not substantially adversely affect the mucosal tissue and will be selected according to the physicochemical characteristics of the particular glucose-regulating peptide or other active or delivery-enhancing agent. In this context, delivery-enhancing agents that increase penetration or permeability of mucosal tissues will often result in some alteration of the protective permeability barrier of the mucosa. For such delivery-enhancing agents to be of value within the invention, it is generally desired that any significant changes in permeability of the mucosa be reversible within a time frame appropriate to the desired duration of drug delivery. Furthermore, there should be no substantial, cumulative toxicity, nor any permanent deleterious changes induced in the barrier properties of the mucosa with long-term use.

Within certain aspects of the invention, absorption-promoting agents for coordinate administration or combinatorial formulation with glucose-regulating peptide of the invention are selected from small hydrophilic molecules, including but not limited to, dimethyl sulfoxide (DMSO), dimethylformamide, ethanol, propylene glycol, and the 2-pyrrolidones. Alternatively, long-chain amphipathic molecules, for example, deacylmethyl sulfoxide, azone, sodium laurylsulfate, oleic acid, and the bile salts, may be employed toenhance mucosal penetration of the glucose-regulating peptide. In additional aspects, surfactants (e.g., polysorbates) are employed as adjunct compounds, processing agents, or formulation additives to enhance intranasal delivery of the glucose-regulating peptide. Agents such as DMSO, polyethylene glycol, and ethanol can, if present in sufficiently high concentrations in delivery environment (e.g., by pre-administration or incorporation in a therapeutic formulation), enter the aqueous phase of the mucosa and alter its solubilizing properties, thereby enhancing the partitioning of the glucose-regulating peptide from the vehicle into the mucosa.

Additional mucosal delivery-enhancing agents that are useful within the coordinate administration and processing methods and combinatorial formulations of the invention include, but are not limited to, mixed micelles; enamines; nitric oxide donors (e.g., S-nitroso-N-acetyl-DL-penicillamine, NOR1, NOR4--which are preferably co-administered with an NO scavenger such as carboxy-PITO or doclofenac sodium); sodium salicylate; glycerol esters of acetoacetic acid (e.g., glyceryl-1,3-diacetoacetate or 1,2-isopropylideneglycerine-3-acetoacetate); and other release-diffusion or intra- or trans-epithelial penetration-promoting agents that are physiologically compatible for mucosal delivery. Other absorption-promoting agents are selected from a variety of carriers, bases and excipients that enhance mucosal delivery, stability, activity or trans-epithelial penetration of the glucose-regulating peptide. These include, *inter asia*, cyclodextrins and β-cyclodextrin derivatives (e.g., 2-hydroxypropyl-β-cyclodextrin and heptakis(2,6-di-O-methyl-β-cyclodextrin). These compounds, optionally conjugated with one or more of the active ingredients and further optionally formulated in an oleaginous base, enhance bioavailability in the mucosal formulations of the invention. Yet additional absorption-enhancing agents adapted for mucosal delivery include medium-chain fatty acids, including mono- and diglycerides (e.g., sodium caprate--extracts of coconut oil, Capmul), and triglycerides (e.g., amylodextrin, Estaram 299, Miglyol 810).

The mucosal therapeutic and prophylactic compositions of the present invention may be supplemented with any suitable penetration-promoting agent that facilitates absorption, diffusion, or penetration of glucose-regulating peptide across mucosal barriers. The penetration promoter may be any promoter that is pharmaceutically acceptable. Thus, in more detailed aspects of the invention compositions are provided that incorporate one or more penetration-promoting agents selected from sodium salicylate and salicylic acid derivatives (acetyl salicylate, choline salicylate, salicylamide, etc.); amino acids and salts thereof (e.g. monoaminocarboxlic acids such as glycine, alanine, phenylalanine, proline, hydroxyproline, etc.; hydroxyamino acids such as serene; acidic amino acids such as aspartic acid, glutamic acid, etc; and basic amino acids such as lysine etc-inclusive of their alkali metal or alkaline earth metal salts); and N-acetylamino acids (N-acetylalanino, N-acetylphenylalanine, N-acetylserine, N-acetylglycine, N-acetyllysine, N-acetylglutamic acid, N-acetylproline, N-acetylhydroxyproline, etc.) and their salts (alkali metal salts and alkaline earth metal salts). Also provided as penetration-promoting agents within the methods and compositions of the invention are substances which are generally used as emulsifiers (e.g. sodium oleyl phosphate, sodium lauryl phosphate, sodium lauryl sulfate, sodium myristyl sulfate, polyoxyethylene alkyl ethers, polyoxyethylene alkyl esters, etc.), caproic acid, lactic acid, malic acid and citric acid and alkali metal salts thereof, pyrrolidonecarboxylic acids, alkylpyrrolidonecarboxylic acid esters, N-alkylpyrrolidones, proline acyl esters, and the like.

Within various aspects of the invention, improved nasal mucosal delivery formulations and therapeutic uses are provided that allow delivery of glucose-regulating peptide and other therapeutic agents within the invention across mucosal barriers between administration and selected target sites. Certain formulations are specifically adapted for a selected target cell, tissue or organ, or even a particular disease state. In other aspects, formulations and therapeutic uses provide for efficient, selective endo- or transcytosis of glucose-regulating peptide specifically routed along a defined intracellular or intercellular pathway. Typically, the glucose-regulating peptide is efficiently loaded at effective concentration levels in a carrier or other delivery vehicle, and is delivered and maintained in a stabilized form, e.g., at the nasal mucosa and/or during passage through intracellular compartments and membranes to a remote target site for drug action (e.g., the blood stream or a defined tissue, organ, or extracellular compartment). The glucose-regulating peptide may be provided in a delivery vehicle or otherwise modified (e.g., in the form of a prodrug), wherein release or activation of the glucose-regulating peptide is triggered by a physiological stimulus (e.g. pH change, lysosomal enzymes, etc.) Often, the glucose-regulating peptide is pharmacologically inactive until it reaches its target site for activity. In most cases, the glucose-regulating peptide and other formulation components are non-toxic and non-immunogenic. In this context, carriers and other formulation components are generally selected for their ability to be rapidly degraded and excreted under physiological conditions. At the same time, formulations are chemically and physically stable in dosage form for effective storage.

## Peptide and Protein Analogs and Mimetics

Included within the definition of biologically active peptides and proteins for use within the invention are natural or synthetic, therapeutically or prophylactically active, peptides (comprised of two or more covalently linked amino acids), proteins, peptide or protein fragments, peptide or protein analogs, and chemically modified derivatives or salts of active peptides or proteins. A wide variety of useful analogs and mimetics of glucose-regulating peptide are contemplated for use within the invention and can be produced and tested for biological activity according to known methods. Often, the peptides or proteins of glucose-regulating peptide or other biologically active peptides or proteins for use within the invention are muteins that are readily obtainable by partial substitution, addition, or deletion of amino acids within a naturally occurring or native (e.g., wild-type, naturally occurring mutant, or allelic variant) peptide or protein sequence. Additionally, biologically active fragments of native peptides or proteins are included. Such mutant derivatives and fragments substantially retain the desired biological activity of the native peptide or proteins. In the case of peptides or proteins having carbohydrate chains, biologically active variants marked by alterations in these carbohydrate species are also included within the invention.

As used herein, the term "conservative amino acid substitution" refers to the general interchangeability of amino acid residues having similar side chains. For example, a commonly interchangeable group of amino acids having aliphatic side chains is alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Examples of conservative substitutions include the substitution of a non-polar (hydrophobic) residue such as isoleucine, valine, leucine or methionine for another. Likewise, the present invention contemplates the substitution of a polar (hydrophilic) residue such as between arginine and lysine, between glutamine and asparagine, and between threonine and serine. Additionally, the substitution of a basic residue such as lysine, arginine or histidine for another or the substitution of an acidic residue such as aspartic acid or glutamic acid for another is also contemplated. Exemplary conservative amino acids substitution groups are: valine-leucine-isoleucine, phenylalanine-tyrosine, lysine-arginine, alanine-valine, and asparagine-glutamine. By aligning a peptide or protein analog optimally with a corresponding native peptide or protein, and by using appropriate assays, e.g., adhesion protein or receptor binding assays, to determine a selected biological activity, one can readily identify operable peptide and protein analogs for use within the methods and compositions of the invention. Operable peptide and protein analogs are typically specifically immunoreactive with antibodies raised to the corresponding native peptide or protein.

An approach for stabilizing solid protein formulations of the invention is to increase the physical stability of purified, e.g., lyophilized, protein. This will inhibit aggregation via hydrophobic interactions as well as via covalent pathways that may increase as proteins unfold. Stabilizing formulations in this context often include polymer-based formulations, for example a biodegradable hydrogel formulation/delivery system. As noted above, the critical role of water in protein structure, function, and stability is well known. Typically, proteins are relatively stable in the solid state with bulk water removed. However, solid therapeutic protein formulations may become hydrated upon storage at elevated humidities or during delivery from a sustained release composition or device. The stability of proteins generally drops with increasing hydration. Water can also play a significant role in solid protein aggregation, for example, by increasing protein flexibility resulting in enhanced accessibility of reactive groups, by providing a mobile phase for reactants, and by serving as a reactant in several deleterious processes such as beta-elimination and hydrolysis.

Protein preparations containing between about 6% to 28% water are the most unstable. Below this level, the mobility of bound water and protein internal motions are low. Above this level, water mobility and protein motions approach those of full hydration. Up to a point, increased susceptibility toward solid-phase aggregation with increasing hydration has been observed in several systems. However, at higher water content, less aggregation is observed because of the dilution effect.

In accordance with these principles, an effective method for stabilizing peptides and proteins against solid-state aggregation for mucosal delivery is to control the water content in a solid formulation and maintain the water activity in the formulation at optimal levels. This level depends on the nature of the protein, but in general, proteins maintained below their "monolayer" water coverage will exhibit superior solid-state stability.

A variety of additives, diluents, bases and delivery vehicles are provided within the invention that effectively control water content to enhance protein stability. These reagents and carrier materials effective as anti-aggregation agents in this sense include, for example, polymers of various functionalities, such as polyethylene glycol, dextran, diethylaminoethyl dextran, and carboxymethyl cellulose, which significantly increase the stability and reduce the solid-phase aggregation of peptides and proteins admixed therewith or linked thereto. In some instances, the activity or physical stability of proteins can also be enhanced by various additives to aqueous solutions of the peptide or protein drugs. For example, additives, such as polyols (including sugars), amino acids, proteins such as collagen and gelatin, and various salts may be used.

Certain additives, in particular sugars and other polyols, also impart significant physical stability to dry, e.g., lyophilized proteins. These additives can also be used within the invention to protect the proteins against aggregation not only during lyophilization but also during storage in the dry state. For example sucrose and Ficoll 70 (a polymer with sucrose units) exhibit significant protection against peptide or protein aggregation during solid-phase incubation under various conditions. These additives may also enhance the stability of solid proteins embedded within polymer matrices.

Yet additional additives, for example sucrose, stabilize proteins against solid-state aggregation in humid atmospheres at elevated temperatures, as may occur in certain sustained-release formulations of the invention. Proteins such as gelatin and collagen also serve as stabilizing or bulking agents to reduce denaturation and aggregation of unstable proteins in this context. These additives can be incorporated into polymeric melt processes and compositions within the invention. For example, polypeptide microparticles can be prepared by simply lyophilizing or spray drying a solution containing various stabilizing additives described above. Sustained release of unaggregated peptides and proteins can thereby be obtained over an extended period of time.

Various additional preparative components and methods, as well as specific formulation additives, are provided herein which yield formulations for mucosal delivery of aggregation-prone peptides and proteins, wherein the peptide or protein is stabilized in a substantially pure, unaggregated form using a solubilization agent. A range of components and additives are contemplated for use within these methods and formulations. Exemplary of these solubilization agents are cyclodextrins (CDs), which selectively bind hydrophobic side chains of polypeptides. These CDs have been found to bind to hydrophobic patches of proteins in a manner that significantly inhibits aggregation.. This inhibition is selective with respect to both the CD and the protein involved. Such selective inhibition of protein aggregation provides additional advantages within the intranasal delivery methods and compositions of the invention. Additional agents for use in this context include CD dimers, trimers and tetramers with varying geometries controlled by the linkers that specifically block aggregation of peptides and protein. Yet solubilization agents and methods for incorporation within the invention involve the use of peptides and peptide mimetics to selectively block protein-protein interactions. In one aspect, the specific binding of hydrophobic side chains reported for CD multimers is extended to proteins via the use of peptides and peptide mimetics that similarly block protein aggregation. A wide range of suitable methods and anti-aggregation agents are available for incorporation within the compositions and procedures of the invention.

### Charge Modifying and pH Control Agents and Methods

To improve the transport characteristics of biologically active agents (including glucose-regulating peptide, other active peptides and proteins, and macromolecular and small molecule drugs) for enhanced delivery across hydrophobic mucosal membrane barriers, the invention also provides techniques and reagents for charge modification of selected biologically active agents or delivery-enhancing agents described herein. In this regard, the relative permeabilities of macromolecules is generally be related to their partition coefficients. The degree of ionization of molecules, which is dependent on the $pK_a$ of the molecule and the pH at the mucosal membrane surface, also affects permeability of the molecules. Permeation and partitioning of biologically active agents, including glucose-regulating peptide and analogs of the invention, for mucosal delivery may be facilitated by charge alteration or charge spreading of the active agent or permeabilizing agent, which is achieved, for example, by alteration of charged functional groups, by modifying the pH of the delivery vehicle or solution in which the active agent is delivered, or by coordinate administration of a charge- or pH-altering reagent with the active agent.

Consistent with these general teachings, mucosal delivery of charged macromolecular species, including glucose-regulating peptide and other biologically active peptides and proteins, within the methods and compositions of the invention is substantially improved when the active agent is delivered to the mucosal surface in a substantially un-ionized, or neutral, electrical charge state.

Certain glucose-regulating peptide and other biologically active peptide and protein components of mucosal formulations for use within the invention will be charge modified to yield an increase in the positive charge density of the peptide or protein. These modifications extend also to cationization of peptide and protein conjugates, carriers and other delivery forms disclosed herein. Cationization offers a convenient means of altering the biodistribution and transport properties of proteins and macromolecules within the invention. Cationization is undertaken in a manner that substantially preserves the biological activity of the active agent and limits potentially adverse side effects, including tissue damage and toxicity.

### Degradative Enzyme Inhibitory Agents and Methods

Another excipient that may be included in a trans-mucosal preparation is a degradative enzyme inhibitor. Exemplary mucoadhesive polymer-enzyme inhibitor complexes that are useful within the mucosal delivery formulations and methods of the invention include, but are not limited to: Carboxymethylcellulose-pepstatin (with anti-pepsin activity); Poly(acrylic acid)-Bowman-Birk inhibitor (anti-chymotrypsin); Poly(acrylic acid)-chymostatin (anti-chymotrypsin); Poly(acrylic acid)-elastatinal (anti-elastase); Carboxymethylcellulose-elastatinal (anti-elastase); Polycarbophil-elastatinal (anti-elastase); Chitosan-antipain (anti-trypsin); Poly(acrylic acid)-bacitracin (anti-aminopeptidase N); Chitosan-EDTA (anti-aminopeptidase N, anti-carboxypeptidase A); Chitosan-EDTA-antipain (and-trypsin, anti-chymotrypsin, anti-elastase). As described in further detail below, certain embodiments of the invention will optionally incorporate a novel chitosan derivative or chemically modified form of chitosan. One such novel derivative for use within the invention is denoted as a β-[1→4]-2-guanidino-2-deoxy-D-glucose polymer (poly-GuD).

Any inhibitor that inhibits the activity of an enzyme to protect the biologically active agent(s) may be usefully employed in the compositions and therapeutic uses of the invention. Useful enzyme inhibitors for the protection of biologically active proteins and peptides include, for example, soybean trypsin inhibitor, exendin trypsin inhibitor, chymotrypsin inhibitor and trypsin and chrymotrypsin inhibitor isolated from potato (solanum tuberosum L.) tubers. A combination or mixtures of inhibitors may be employed. Additional inhibitors of proteolytic enzymes for use within the invention include ovomucoid-enzyme, gabaxate mesylate, alphalantitrypsin, aprotinin, amastatin, bestatin, puromycin, bacitracin, leupepsin, alpha2-macroglobulin, pepstatin and egg white or soybean trypsin inhibitor. These and other inhibitors can be used alone or in combination. The inhibitor(s) may be incorporated in or bound to a carrier, e.g., a hydrophilic polymer, coated on the surface of the dosage form which is to contact the nasal mucosa, or incorporated in the superficial phase of the surface, in combination with the biologically active agent or in a separately administered (e.g., pre-administered) formulation.

The amount of the inhibitor, e.g., of a proteolytic enzyme inhibitor that is optionally incorporated in the compositions of the invention will vary depending on (a) the properties of the specific inhibitor, (b) the number of functional groups present in the molecule (which may be reacted to introduce ethylenic unsaturation necessary for copolymerization with hydrogel forming monomers), and (c) the number of lectin groups, such as glycosides, which are present in the inhibitor molecule. It may also depend on the specific therapeutic agent that is intended to be administered. Generally speaking, a useful amount of an enzyme inhibitor is from about 0.1 mg/ml to about 50 mg/ml, often from about 0.2 mg/ml to about 25 mg/ml, and more commonly from about 0.5 mg/ml to 5 mg/ml of the of the formulation (i.e., a separate protease inhibitor formulation or combined formulation with the inhibitor and biologically active agent).

In the case of trypsin inhibition, suitable inhibitors may be selected from, e.g., aprotinin, BBI, soybean trypsin inhibitor, chicken ovomucoid, chicken ovoinhibitor, human exendin trypsin inhibitor, camostat mesilate, flavonoid inhibitors, antipain, leupeptin, p-aminobenzamidine, AEBSF, TLCK (tosyllysine chloromethylketone), APMSF, DFP, PMSF, and poly(acrylate) derivatives. In the case of chymotrypsin inhibition, suitable inhibitors may be selected from, e.g., aprotinin, BBI, soybean trypsin inhibitor, chymostatin, benzyloxycarbonyl-Pro-Phe-CHO, FK-448, chicken ovoinhibitor, sugar biphenylboxonic acids complexes, DFP, PMSF, β-phenylpropionate, and poly(acrylate) derivatives. In the case of elastase inhibition, suitable inhibitors may be selected from, e.g., elastatinal, methoxysuccinyl-Ala-Ala-Pro-Val-chloromethylketone (MPCMK), BBI, soybean trypsin inhibitor, chicken ovoinhibitor, DFP, and PMSF.

Additional enzyme inhibitors for use within the invention are selected from a wide range of non-protein inhibitors that vary in their degree of potency and toxicity. As described in further detail below, immobilization of these adjunct agents to matrices or other delivery vehicles, or development of chemically modified analogues, may be readily implemented to reduce or even eliminate toxic effects, when they are encountered. Among this broad group of candidate enzyme inhibitors for use within the invention are organophosphorous inhibitors, such as diisopropylfluorophosphate (DFP) and phenylmethylsulfonyl fluoride (PMSF), which are potent, irreversible inhibitors of serine proteases (e.g., trypsin and chymotrypsin). The additional inhibition of acetylcholinesterase by these compounds makes them highly toxic in uncontrolled delivery settings. Another candidate inhibitor, 4-(2-Aminoethyl)-benzenesulfonyl fluoride (AEBSF), has an inhibitory activity comparable to DFP and PMSF, but it is markedly less toxic. (4-Aminophenyl)-methanesulfonyl fluoride hydrochloride (APMSF) is another potent inhibitor of trypsin, but is toxic in uncontrolled settings. In contrast to these inhibitors, 4-(4-isopropylpiperadinocarbonyl)phenyl1,2,3,4-tetrahydro-1-naphthoate methanesulphonate (FK-448) is a low toxic substance, representing a potent and specific inhibitor of chymotrypsin. Further representatives of this non-protein group of inhibitor candidates, and also exhibiting low toxic risk, are camostat mesilate (N,N'-dimethyl carbamoylmethyl-p-(p '-guanidino-benzoyloxy)phenylacetate methane-sulphonate).

Yet another type of enzyme inhibitory agent for use within the therapeutic uses and compositions of the invention are amino acids and modified amino acids that interfere with enzymatic degradation of specific therapeutic compounds. For use in this context, amino acids and modified amino acids are substantially non-toxic and can be produced at a low cost. However, due to their low molecular size and good solubility, they are readily diluted and absorbed in mucosal environments. Nevertheless, under proper conditions, amino acids can act as reversible, competitive inhibitors of protease enzymes. Certain modified amino acids can display a much stronger inhibitory activity. A desired modified amino acid in this context is known as a 'transition-state' inhibitor. The strong inhibitory activity of these compounds is based on their structural similarity to a substrate in its transition-state geometry, while they are generally selected to have a much higher affinity for the active site of an enzyme than the substrate itself. Transition-state inhibitors are reversible, competitive inhibitors. Examples of this type of inhibitor are α-aminoboronic acid derivatives, such as boro-leucine, boro-valine and boro-alanine. The boron atom in these derivatives can form a tetrahedral boronate ion that is believed to resemble the transition state of peptides during their hydrolysis by aminopeptidases. These amino acid derivatives are potent and reversible inhibitors of aminopeptidases and it is reported that boro-leucine is more than 100-times more effective in enzyme inhibition than bestatin and more than 1000-times more effective than puromycin. Another modified amino acid for which a strong protease inhibitory activity has been reported is N-acetylcysteine, which inhibits enzymatic activity of aminopeptidase N. This adjunct agent also displays mucolytic properties that can be employed within the methods and compositions of the invention to reduce the effects of the mucus diffusion barrier.

Still other useful enzyme inhibitors for use within the coordinate administration methods and combinatorial formulations of the invention may be selected from peptides and modified peptide enzyme inhibitors. An important representative of this class of inhibitors is the cyclic dodecapeptide, bacitracin, obtained from Bacillus licheniformis. In addition to these types of peptides, certain dipeptides and tripeptides display weak, non-specific inhibitory activity towards some protease. By analogy with amino acids, their inhibitory activity can be improved by chemical modifications. For example, phosphinic acid dipeptide analogues are also 'transition-state' inhibitors with a strong inhibitory activity towards aminopeptidases. They have reportedly been used to stabilize nasally administered leucine enkephalin. Another example of a transition-state analogue is the modified pentapeptide pepstatin, which is a very potent inhibitor of pepsin. Structural analysis of pepstatin, by testing the inhibitory activity of several synthetic analogues, demonstrated the major structure-function characteristics of the molecule responsible for the inhibitory activity. Another special type of modified peptide includes inhibitors with a terminally located aldehyde function in their structure. For example, the sequence benzyloxycarbonyl-Pro-Phe-CHO, which fulfills the known primary and secondary specificity requirements of chymotrypsin, has been found to be a potent reversible inhibitor of this target proteinase. The chemical structures of further inhibitors with a terminally located aldehyde function, e.g. antipain, leupeptin, chymostatin and elastatinal, are also known in the art, as are the structures of other known, reversible, modified peptide inhibitors, such as phosphoramidon, bestatin, puromycin and amastatin.

Due to their comparably high molecular mass, polypeptide protease inhibitors are more amenable than smaller compounds to concentrated delivery in a drug-carrier matrix. Additional agents for protease inhibition within the formulations and methods of the invention involve the use of complexing agents. These agents mediate enzyme inhibition by depriving the intranasal environment (or preparative or therapeutic composition) of divalent cations, which are co-factors for many proteases. For instance, the complexing agents EDTA and DTPA as coordinately administered or combinatorially formulated adjunct agents, in suitable concentration, will be sufficient to inhibit selected proteases to thereby enhance intranasal delivery of biologically active agents according to the invention. Further representatives of this class of inhibitory agents are EGTA, 1,10-phenanthroline and hydroxychinoline. In addition, due to their propensity to chelate divalent cations, these and other complexing agents are useful within the invention as direct, absorption-promoting agents.

As noted in more detail elsewhere herein, it is also contemplated to use various polymers, particularly mucoadhesive polymers, as enzyme inhibiting agents within the coordinate administration, multi-processing and/or combinatorial formulation methods and compositions of the invention. For example, poly(acrylate) derivatives, such as poly(acrylic acid) and polycarbophil, can affect the activity of various proteases, including trypsin, chymotrypsin. The inhibitory effect of these polymers may also be based on the complexation of divalent cations such as $Ca^{2+}$ and $Zn^{2+}$. It is further contemplated that these polymers may serve as conjugate partners or carriers for additional enzyme inhibitory agents, as described above. For example, a chitosan-EDTA conjugate has been developed and is useful within the invention that exhibits a strong inhibitory effect towards the enzymatic activity of zinc-dependent proteases. The mucoadhesive properties of polymers following covalent attachment of other enzyme inhibitors in this context are not expected to be substantially compromised, nor is the general utility of such polymers as a delivery vehicle for biologically active agents within the invention expected to be diminished. On the contrary, the reduced distance between the delivery vehicle and mucosal surface afforded by the mucoadhesive mechanism will minimize presystemic metabolism of the active agent, while the covalently bound enzyme inhibitors remain concentrated at the site of drug delivery, minimizing undesired dilution effects of inhibitors as well as toxic and other side effects caused thereby. In this manner, the effective amount of a coordinately administered enzyme inhibitor can be reduced due to the exclusion of dilution effects.

Exemplary mucoadhesive polymer-enzyme inhibitor complexes that are useful within the mucosal formulations and methods of the invention include, but are not limited to: Carboxymethylcellulose-pepstatin (with anti-pepsin activity); Poly(acrylic acid)-Bowman-Birk inhibitor (anti-chymotrypsin); Poly(acrylic acid)-chymostatin (anti-chymotrypsin); Poly(acrylic acid)-elastatinal (anti-elastase); Carboxymethylcellulose-elastatinal (anti-elastase); Polycarbophil-elastatinal (anti-elastase); Chitosan-antipain (anti-trypsin); Poly(acrylic acid)-bacitracin (anti-aminopeptidase N); Chitosan-EDTA (anti-aminopeptidase N, anti-carboxypeptidase A); Chitosan-EDTA-antipain (anti-trypsin, anti-chymotrypsin, anti-elastase).

**Mucolytic and Mncus-Clearing Agents and Methods**

Effective delivery of biotherapeutic agents via intranasal administration must take into account the decreased drug transport rate across the protective mucus lining of the nasal mucosa, in addition to drug loss due to binding to glycoproteins of the mucus layer. Normal mucus is a viscoelastic, gel-like substance consisting of water, electrolytes, mucins, macromolecules, and sloughed epithelial cells. It serves primarily as a cytoprotective and lubricative covering for the underlying mucosal tissues. Mucus is secreted by randomly distributed secretory cells located in the nasal epithelium and in other mucosal epithelia. The structural unit of mucus is mucin. This glycoprotein is mainly responsible for the viscoelastic nature of mucus, although other macromolecules may also contribute to this property. In airway mucus, such macromolecules include locally produced secretory IgA, IgM, IgE, lysozyme, and bronchotransferrin, which also play an important role in host defense mechanisms.

The coordinate administration therapeutic uses of the instant invention optionally incorporate effective mucolytic or mucus-clearing agents, which serve to degrade, thin or clear mucus from intranasal mucosal surfaces to facilitate absorption of intranasally administered biotherapeutic agents. Within these uses, a mucolytic or mucus-clearing agent is coordinately administered as an adjunct compound to enhance intranasal delivery of the biologically active agent. Alternatively, an effective amount of a mucolytic or mucus-clearing agent is incorporated as a processing agent within a multi-processing method of the invention, or as an additive within a combinatorial formulation of the invention, to provide an improved formulation that enhances intranasal delivery of biotherapeutic compounds by reducing the barrier effects of intranasal mucus.

A variety of mucolytic or mucus-clearing agents are available for incorporation within the therapeutic uses and compositions of the invention. Based on their mechanisms of action, mucolytic and mucus clearing agents can often be classified into the following groups: proteases (e.g., pronase, papain) that cleave the protein core of mucin glycoproteins; sulfhydryl compounds that split mucoprotein disulfide linkages; and detergents (e.g., Triton X-100, Tween 20) that break non-covalent bonds within the mucus. Additional compounds in this context include, but are not limited to, bile salts and surfactants, for example, sodium deoxycholate, sodium taurodeoxycholate, sodium glycocholate, and lysophosphatidylcholine.

The effectiveness of bile salts in causing structural breakdown of mucus is in the order deoxycholate > taurocholate > glycocholate. Other effective agents that reduce mucus viscosity or adhesion to enhance intranasal delivery according to the invention include, e.g., short-chain fatty acids, and mucolytic agents that work by chelation, such as N-acylcollagen peptides, bile acids, and saponins (the latter function in part by chelating $Ca^{2+}$ and/or $Mg^{2+}$ which play an important role in maintaining mucus layer structure).

Additional mucolytic agents for use within the therapeutic uses and compositions of the invention include N-acetyl-L-cysteine (ACS), a potent mucolytic agent that reduces both the viscosity and adherence of bronchopulmonary mucus and is reported to modestly increase nasal bioavailability of human growth hormone in anesthetized rats (from 7.5 to 12.2%). These and other mucolytic or mucus-clearing agents are contacted with the nasal mucosa, typically in a concentration range of about 0.2 to 20 mM, coordinately with administration of the biologically active agent, to reduce the polar viscosity and/or elasticity of intranasal mucus.

Still other mucolytic or mucus-clearing agents may be selected from a range of glycosidase enzymes, which are able to cleave glycosidic bonds within the mucus glycoprotein. α-amylase and β-amylase are representative of this class of enzymes, although their mucolytic effect may be limited. In contrast, bacterial glycosidases which allow these microorganisms to permeate mucus layers of their hosts.

For combinatorial use with most biologically active agents within the invention, including peptide and protein therapeutics, non-ionogenic detergents are generally also useful as mucolytic or mucus-clearing agents. These agents typically will not modify or substantially impair the activity of therapeutic polypeptides.

**Ciliostatic Agents and Methods**

Because the self-cleaning capacity of certain mucosal tissues (e.g., nasal mucosal tissues) by mucociliary clearance is necessary as a protective function (e.g., to remove dust, allergens, and bacterial), it has been generally considered that this function should not be substantially impaired by mucosal medications. Mucociliary transport in the respiratory tract is a particularly important defense mechanism against infections. To achieve this function, ciliary beating in the nasal and airway passages moves a layer of mucus along the mucosa to removing inhaled particles and microorganisms.

Ciliostatic agents find use within the methods and compositions of the invention to increase the residence time of mucosally (e.g., intranasally) administered glucose-regulating peptide, analogs and mimetics, and other biologically active agents disclosed herein. In particular, the delivery these agents within the methods and compositions of the invention is significantly enhanced in certain aspects by the coordinate administration or combinatorial formulation of one or more ciliostatic agents that function to reversibly inhibit ciliary activity of mucosal cells, to provide for a temporary, reversible increase in the residence time of the mucosally administered active agent(s). For use within these aspects of the invention, the foregoing ciliostatic factors, either specific or indirect in their activity, are all candidates for successful employment as ciliostatic agents in appropriate amounts (depending on concentration, duration and mode of delivery) such that they yield a transient (i.e., reversible) reduction or cessation of mucociliary clearance at a mucosal site of administration to enhance delivery of glucose-regulating peptide, analogs.and mimetics, and other biologically active agents disclosed herein, without unacceptable adverse side effects.

Within more detailed aspects, a specific ciliostatic factor is employed in a combined formulation or coordinate administration protocol with one or more glucose-regulating peptide proteins, analogs and mimetics, and/or other biologically active agents disclosed herein. Various bacterial ciliostatic factors isolated and characterized in the literature may be employed within these embodiments of the invention. Ciliostatic factors from the bacterium *Pseudomonas aeruginosa* include a phenazine derivative, a pyo compound (2-alkyl-4-hydroxyquinolines), and a rhamnolipid (also known as a hemolysin). The pyo compound produced ciliostasis at concentrations of 50 $\mu$g/ml and without obvious ultrastructural lesions. The phenazine derivative also inhibited ciliary motility but caused some membrane disruption, although at substantially greater concentrations of 400 $\mu$g/ml. Limited exposure of tracheal explants to the rhamnolipid resulted in ciliostasis, which is associated with altered ciliary membranes. More extensive exposure to rhamnolipid is associated with removal of dynein arms from axonemes.

**Surface Active Agents and Methods**

One or more membrane penetration-enhancing agents may be employed within a mucosal delivery therapeutic use or formulation of the invention to enhance mucosal delivery of glucose-regulating peptide proteins, analogs and mimetics, and other biologically active agents disclosed herein. Membrane penetration enhancing agents in this context can be selected from: (i) a surfactant, (ii) a bile salt, (iii) a phospholipid additive, mixed micelle, liposome, or carrier, (iv) an alcohol, (v) an enamine, (vi) an NO donor compound, (vii) a long-chain amphipathic molecule (viii) a small hydrophobic penetration enhancers; (ix) sodium or a salicylic acid derivative; (x) a glycerol ester of acetoacetic acid (xi) a clyclodextrin or beta-cyclodextrin derivative, (xii) a medium-chain fatty acid, (xiii) a chelating agent, (xiv) an amino acid or salt thereof, (xv) an N-acetylamino acid or salt thereof, (xvi) an enzyme degradative to a selected membrane component, (xvii) an inhibitor of fatty acid synthesis, or (xviii) an inhibitor of cholesterol synthesis; or (xix) any combination of the membrane penetration enhancing agents recited in (i)-(xix).

Certain surface-active agents are readily incorporated within the mucosal delivery formulations and therapeutic uses of the invention as mucosal absorption enhancing agents. These agents, which may be coordinately administered or combinatorially formulated with glucose-regulating peptide proteins, analogs and mimetics, and other biologically active agents disclosed herein, may be selected from a broad assemblage of known surfactants. Surfactants, which generally fall into three classes: (1) nonionic polyoxyethylene ethers; (2) bile salts such as sodium glycocholate (SGC) and deoxycholate (DOC); and (3) derivatives of fusidic acid such as sodium taurodihydrofusidate (STDHF). The mechanisms of action of these various classes of surface-active agents typically include solubilization of the biologically active agent. For proteins and peptides which often form aggregates, the surface active properties of these absorption promoters can allow interactions with proteins such that smaller units such as surfactant coated monomers may be more readily maintained in solution. Examples of other surface-active agents are L-$\alpha$-Phosphatidylchollne Didecanoyl (DDPC) polysorbate 80 and polysorbate 20.These monomers are presumably more transportable units than aggregates. A second potential mechanism is the protection of the peptide or protein from proteolytic degradation by proteases in the mucosal environment. Both bile salts and some fusidic acid derivatives reportedly inhibit proteolytic degradation of proteins by nasal homogenates at concentrations less than or equivalent to those required to enhance protein absorption. This protease inhibition may be especially important for peptides with short biological half-lives.

**Degradation Enzymes and Inhibitors of Fatty Acid and Cholesterol Synthesis**

In related aspects of the invention, glucose-regulating peptide proteins, analogs and mimetics, and other biologically active agents for mucosal administration may be formulated or coordinately administered with a penetration enhancing agent selected from a degradation enzyme, or a metabolic stimulatory agent or inhibitor of synthesis of fatty acids, sterols or other selected epithelial barrier components, U.S. Patent No. 6,190,894. For example, degradative enzymes such as phospholipase, hyaluronidase, neuraminidase, and chondroitinase may be employed to enhance mucosal penetration of glucose-regulating peptide proteins, analogs and mimetics, and other biologically active agent without causing irreversible damage to the mucosal barrier. In one embodiment, chondroitinase is employed within a therapeutic use or composition as provided herein to alter glycoprotein or glycolipid constituents of the permeability barrier of the mucosa, thereby enhancing mucosal absorption of glucose-regulating peptide proteins, analogs and mimetics, and other biologically active agents disclosed herein.

With regard to inhibitors of synthesis of mucosal barrier constituents, it is noted that free fatty acids account for 20-25% of epithelial lipids by weight. Two rate-limiting enzymes in the biosynthesis of free fatty acids are acetyl CoA carboxylase and fatty acid synthetase. Through a series of steps, free fatty acids are metabolized into phospholipids. Thus, inhibitors of free fatty acid synthesis and metabolism for use within the methods and compositions of the invention include, but are not limited to, inhibitors of acetyl CoA carboxylase such as 5-tetradecyloxy-2-furancarboxylic acid (TOFA);

inhibitors of fatty acid synthetase; inhibitors of phospholipase A such as gomisin A, 2-(p-amylcinnamyl)amino-4-chlorobenzoic acid, bromophenacyl bromide, monoalide, 7,7-dimethyl-5,8-eicosadienoic acid, nicergoline, cepharanthine, nicardipine, quercetin, dibutyryl-cyclic AMP, R-24571, N-oleoylethanolamine, N-(7-nitro-2,1,3-benzoxadiazol-4-yl) phosphostidyl serine, cyclosporine A, topical anesthetics, including dibucaine, prenylamine, retinoids, such as all-trans and 13-cis-retinoic acid, W-7, trifluoperazine, R-24571 (calmidazolium), 1-hexadocyl-3-trifluoroethyl glycero-sn-2-phosphomenthol (MJ33); calcium channel blockers including nicardipine, verapamil, diltiazem, nifedipine, and nimodipine; antimalarials including quinacrine, mepacrine, chloroquine and hydroxychloroquine; beta blockers including propanalol and labetalol; calmodulin antagonists; EGTA; thimersol; glucocorticosteroids including dexamethasone and prednisolone; and nonsteroidal antiinflammatory agents including indomethacin and naproxen.

Free sterols, primarily cholesterol, account for 20-25% of the epithelial lipids by weight. The rate limiting enzyme in the biosynthesis of cholesterol is 3-hydroxy-3-methylglutaryl (HMG) CoA reductase. Inhibitors of cholesterol synthesis for use within the methods and compositions of the invention include, but are not limited to, competitive inhibitors of (HMG) CoA reductase, such as simvastatin, lovastatin, fluindostatin (fluvastatin), pravastatin, mevastatin, as well as other HMG CoA reductase inhibitors, such as cholesterol oleate, cholesterol sulfate and phosphate, and oxygenated sterols, such as 25-OH-- and 26-OH-- cholesterol; inhibitors of squalene synthetase; inhibitors of squalene epoxidase; inhibitors of DELTA7 or DELTA24 reductases such as 22,25-diazacholesterol, 20,25-diazacholestenol, AY9944, and triparanol.

Each of the inhibitors of fatty acid synthesis or the sterol synthesis inhibitors may be coordinately administered or combinatorially formulated with one or more glucose-regulating peptide proteins, analogs and mimetics, and other biologically active agents disclosed herein to achieve enhanced epithelial penetration of the active agent(s). An effective concentration range for the sterol inhibitor in a therapeutic or adjunct formulation for mucosal delivery is generally from about 0.0001 % to about 20% by weight of the total, more typically from about 0.01% to about 5%.

**Nitric Oxide Donor Agents and Methods**

A nitric oxide (NO) donor is selected as a membrane penetration-enhancing agent to enhance mucosal delivery of one or more glucose-regulating peptide proteins, analogs and mimetics, and other biologically active agents disclosed herein. Various NO donors are known in the art and are useful in effective concentrations within the methods and formulations of the invention. Exemplary NO donors include, but are not limited to, nitroglycerine, nitropruside, NOC5 [3-(2-hydroxy-1-(methylethyl)-2-nitrosohydrazino)-1-propanamine], NOC12 [*N*-ethyl-2-(1-ethyl-hydroxy-2-nitrosohydrazino)-ethanamine], SNAP [S-nitroso-N-acetyl-DL-penicillamine], NORI and NOR4. Within the therapeutic uses and compositions of the invention, an effective amount of a selected NO donor is coordinately administered or combinatorially formulated with one or more glucose-regulating peptide proteins, analogs and mimetics, and/or other biologically active agents disclosed herein, into or through the mucosal epithelium.

**Agents for Modulating Epithelial Junction Structure and/or Physiology**

The present invention provides pharmaceutical composition that contains one or more glucose-regulating peptide proteins, in combination with mucosal delivery enhancing agents disclosed herein formulated in a pharmaceutical preparation for mucosal delivery.

The permeabilizing agent reversibly enhances mucosal epithelial paracellular transport, typically by modulating epithelial junctional structure and/or physiology at a mucosal epithelial surface in the subject. This effect typically involves inhibition by the permeabilizing agent of homotypic or heterotypic binding between epithelial membrane adhesive proteins of neighboring epithelial cells. Target proteins for this blockade of homotypic or heterotypic binding can be selected from various related junctional adhesion molecules (JAMs), occludins, or claudins. Examples of this are antibodies, antibody fragments or single-chain antibodies that bind to the extracellular domains of these proteins.

In yet additional detailed embodiments, the invention describes permeabilizing peptides and peptide analogs and mimetics for enhancing mucosal epithelial paracellular transport. The subject peptides and peptide analogs and mimetics typically work within the compositions and therapeutic uses of the invention by modulating epithelial junctional structure and/or physiology in a mammalian subject. In certain embodiments, the peptides and peptide analogs and mimetics effectively inhibit homotypic and/or heterotypic binding of an epithelial membrane adhesive protein selected from a junctional adhesion molecule (JAM), occludin, or claudin.

One such agent that has been extensively studied is the bacterial toxin from *Vibrio cholerae* known as the "zonula occludens toxin" (ZOT). This toxin mediates increased intestinal mucosal permeability and causes disease symptoms including diarrhea in infected subjects. Fasano et al, Proc. Nat. Acad. Sci., U.S.A., 8:5242-5246 (1991). When tested on rabbit ileal mucosa, ZOT increased the intestinal permeability by modulating the structure of intercellular tight junctions. More recently, it has been found that ZOT is capable of reversibly opening tight junctions in the intestinal mucosa. It has also been reported that ZOT is capable of reversibly opening tight junctions in the nasal mucosa. U.S.

23

Pat No. 5,908,825.

Within the therapeutic uses and compositions described in the invention, ZOT, as well as various analogs and mimetics of ZOT that function as agonists or antagonists of ZOT activity, are useful for enhancing intranasal delivery of biologically active agents-by increasing paracellular absorption into and across the nasal mucosa. In this context, ZOT typically acts by causing a structural reorganization of tight junctions marked by altered localization of the junctional protein ZO1. Within these aspects of the invention, ZOT is coordinately administered or combinatorially formulated with the biologically active agent in an effective amount to yield significantly enhanced absorption of the active agent, by reversibly increasing nasal mucosal permeability without substantial adverse side effects

## Vasodilator Agents and Methods

Yet another class of absorption-promoting agents that shows beneficial utility within the coordinate administration and combinatorial formulation methods and compositions of the invention are vasoactive compounds, more specifically vasodilators. These compounds function within the invention to modulate the structure and physiology of the submucosal vasculature; increasing the transport rate of glucose-regulating peptide, analogs and mimetics, and other biologically active agents into or through the mucosal epithelium and/or to specific target tissues or compartments (e.g., the systemic circulation or central nervous system.).

Vasodilator agents which can be used within the invention typically cause submucosal blood vessel relaxation by either a decrease in cytoplasmic calcium, an increase in nitric oxide (NO) or by inhibiting myosin light chain kinase. They are generally divided into 9 classes: calcium antagonists, potassium channel openers, ACE inhibitors, angiotensin-II receptor antagonists, $\alpha$-adrenergic and imidazole receptor antagonists, $\beta1$ -adrenergic agonists, phosphodiesterase inhibitors, eicosanoids and NO donors.

Despite chemical differences, the pharmacokinetic properties of calcium antagonists are similar. Absorption into the systemic circulation is high, and these agents therefore undergo considerable first-pass metabolism by the liver, resulting in individual variation in pharmacokinetics. Except for the newer drugs of the dihydropyridine type (amlodipine, felodipine, isradipine, nilvadipine, nisoldipine and nitrendipine), the half-life of calcium antagonists is short. Therefore, to maintain an effective drug concentration for many of these may require delivery by multiple dosing, or controlled release formulations, as described elsewhere herein. Treatment with the potassium channel opener minoxidil may also be limited in manner and level of administration due to potential adverse side effects.

ACE inhibitors prevent conversion of angiotensin-I to angiotensin-II and are most effective when renin production is increased. Since ACE is identical to kininase-II, which inactivates the potent endogenous vasodilator bradykinin, ACE inhibition causes a reduction in bradykinin degradation. ACE inhibitors provide the added advantage of cardioprotective and cardioreparative effects, by preventing and reversing cardiac fibrosis and ventricular hypertrophy in animal models. The predominant elimination pathway of most ACE inhibitors is via renal excretion. Therefore, renal impairment is associated with reduced elimination and a dosage reduction of 25 to 50% is recommended in patients with moderate to severe renal impairment.

With regard to NO donors; these compounds are particularly useful within the invention for their additional effects on mucosal permeability. In addition to the above-noted NO donors, complexes of NO with nucleophiles called NO/nucleophiles, or NONOates, spontaneously and nonenzymatically release NO when dissolved in aqueous solution at physiologic pH. . In contrast, nitro vasodilators such as nitroglycerin require specific enzyme activity for NO release. NONOates release NO with a defined stoichiometry and at predictable rates ranging from <3 minutes for diethylamine/NO to approximately 20 hours for diethylenetriamine/NO (DETANO).

Within certain therapeutic uses and compositions of the invention, a selected vasodilator agent may be coordinately administered (e.g., systemically or intranasally, simultaneously or in combinatorially effective temporal association) or combinatorially formulated with one or more glucose-regulating peptide, and other biologically active agent(s) in an amount effective to enhance the mucosal absorption of the active agent(s) to reach a target tissue or compartment in the subject (e.g., the liver, hepatic portal vein, CNS tissue or fluid, or blood plasma).

## Selective Transport-Enhancing Agents

The compositions and therapeutic uses of the invention optionally incorporate a selective transport-enhancing agent that facilitates transport of one or more biologically active agents. These transport-enhancing agents may be employed in a combinatorial formulation or coordinate administration protocol with one or more of the glucose-regulating peptide proteins, analogs and mimetics disclosed herein, to coordinately enhance delivery of one or more additional biologically active agent(s) across mucosal transport barriers, to enhance mucosal delivery of the active agent(s) to reach a target tissue or compartment in the subject (e.g., the mucosal epithelium, liver, CNS tissue or fluid, or blood plasma). Alternatively, the transport-enhancing agents may be employed in a combinatorial formulation or coordinate administration protocol to directly enhance mucosal delivery of one or more of the glucose-regulating peptide proteins, analogs and mimetics, with or without enhanced delivery of an additional biologically active agent

Exemplary selective transport-enhancing agents for use within this aspect of the invention include, but are not limited to, glycosides, sugar-containing molecules, and binding agents such as lectin binding agents, which are known to interact specifically with epithelial transport barrier components. For example, specific "bioadhesive" ligands, including various plant and bacterial lectins, which bind to cell surface sugar moieties by receptor-mediated interactions can be employed as carriers or conjugated transport mediators for enhancing mucosal, e.g., nasal delivery of biologically active agents within the invention. Certain bioadhesive ligands for use within the invention will mediate transmission of biological signals to epithelial target cells that trigger selective uptake of the adhesive ligand by specialized cellular transport processes (endocytosis or transcytosis). These transport mediators can therefore be employed as a "carrier system" to stimulate or direct selective uptake of one or more glucose-regulating peptide proteins, analogs and mimetics, and other biologically active agent(s) into and/or through mucosal epithelia. These and other selective transport-enhancing agents significantly enhance mucosal delivery of macromolecular biopharmaceuticals (particularly peptides, proteins, oligonucleotides and polynucleotide vectors) within the invention. Lectins are plant proteins that bind to specific sugars found on the surface of glycoproteins and glycolipids of eukaryotic cells. Concentrated solutions of lectins have a 'mucotractive' effect, and various studies have demonstrated rapid receptor mediated endocytocis (RME) of lectins and lectin conjugates (e.g., concanavalin A conjugated with colloidal gold particles) across mucosal surfaces. Additional studies have reported that the uptake mechanisms for lectins can be utilized for intestinal drug targeting *in vivo.* In certain of these studies, polystyrene nanoparticles (500 nm) were covalently coupled to tomato lectin and reported yielded improved systemic uptake after oral administration to rats.

In addition to plant lectins, microbial adhesion and invasion factors provide a rich source of candidates for use as adhesive/selective transport carriers within the mucosal delivery therapeutic uses and compositions of the invention. Two components are necessary for bacterial adherence processes, a bacterial 'adhesin' (adherence or colonization factor) and a receptor on the host cell surface. Bacteria causing mucosal infections need to penetrate the mucus layer before attaching themselves to the epithelial surface. This attachment is usually mediated by bacterial fimbriae or pilus structures, although other cell surface components may also take part in the process. Adherent bacteria colonize mucosal epithelia by multiplication and initiation of a series of biochemical reactions inside the target cell through signal transduction mechanisms (with or without the help of toxins). Associated with these invasive mechanisms, a wide diversity of bioadhesive proteins (e.g., invasin, internalin) originally produced by various bacteria and viruses are known. These allow for extracellular attachment of such microorganisms with an impressive selectivity for host species and even particular target tissues. Signals transmitted by such receptor-ligand interactions trigger the transport of intact, living microorganisms into, and eventually through, epithelial cells by endo- and transcytotic processes. Such naturally occurring phenomena may be harnessed (e.g., by complexing biologically active agents such as glucose-regulating peptide with an adhesin) according to the teachings herein for enhanced delivery of biologically active compounds into or across mucosal epithelia and/or to other designated target sites of drug action.

Various bacterial and plant toxins that bind epithelial surfaces in a specific, lectin-like manner are also useful within the methods and compositions of the invention. For example, diptheria toxin (DT) enters host cells rapidly by RME. Likewise, the B subunit of then *E.coli* heat labile toxin binds to the brush border of intestinal epithelial cells in a highly specific, lectin-like manner. Uptake of this toxin and transcytosis to the basolateral side of the enterocytes has been reported *in vivo* and *in vitro.* Other researches have expressed the transmembrane domain of diphtheria toxin in E. coli as a maltose-binding fusion protein and coupled it chemically to high-Mw poly-L-lysine. The resulting complex is successfully used to mediate internalization of a reporter gene *in vitro.* In addition to these examples, *Staphylococcus aureus* produces a set of proteins (e.g., staphylococcal enterotoxin A (SEA), SEB, toxic shock syndrome toxin 1 (TSST-1) which act both as superantigens and toxins. Studies relating to these proteins have reported dose-dependent, facilitated transcytosis of SEB and TSST- I in Caco-2 cells.

Viral haemagglutinins comprise another type of transport agent to facilitate mucosal delivery of biologically active agents within the therapeutic uses and compositions of the invention. The initial step in many viral infections is the binding of surface proteins (haemagglutinins) to mucosal cells. These binding proteins have been identified for most viruses, including rotaviruses, varicella zoster virus, semliki forest virus, adenoviruses, potato leafroll virus, and reovirus. These and other exemplary viral hemagglutinins can be employed in a combinatorial formulation (e.g., a mixture or conjugate formulation) or coordinate administration protocol with one or more of the glucose-regulating peptide, analogs and mimetics disclosed herein, to coordinately enhance mucosal delivery of one or more additional biologically active agent(s). Alternatively, viral hemagglutinins can be employed in a combinatorial formulation or coordinate administration protocol to directly enhance mucosal delivery of one or more of the glucose-regulating peptide proteins, analogs and mimetics, with or without enhanced delivery of an additional biologically active agent.

A variety of endogenous, selective transport-mediating factors are also available for use within the invention. Mammalian cells have developed an assortment of mechanisms to facilitate the internalization of specific substrates and target these to defined compartments. Collectively, these processes of membrane deformations are termed 'endocytosis' and comprise phagocytosis, pinocytosis, receptor-mediated endocytosis (clathrin-mediated RME), and potocytosis (non-clathrin-mediated RME). RME is a highly specific cellular biologic process by which, as its name implies, various ligands bind to cell surface receptors and are subsequently internalized and trafficked within the cell. In many cells the process of endocytosis is so active that the entire membrane surface is internalized and replaced in less than a half hour. Two classes of receptors are proposed based on their orientation in the cell membrane; the amino terminus of Type I receptors is located on the extracellular side of the membrane, whereas Type II receptors have this same protein tail in the intracellular milieu.

Still other embodiments of the invention describe transferrin as a carrier or stimulant of RME of mucosally delivered biologically active agents. Transferrin, an 80 kDa iron-transporting glycoprotein, is efficiently taken up into cells by RME. Transferrin receptors are found on the surface of most proliferating cells, in elevated numbers on erythroblasts and on many kinds of tumors. The transcytosis of transferrin (Tf) and transferrin conjugates is reportedly enhanced in the presence of Brefeldin A (BFA), a fungal metabolite. In other studies, BFA treatment has been reported to rapidly increase apical endocytosis of both ricin and HRP in MDCK cells. Thus, BFA and other agents that stimulate receptor-mediated transport can be employed within the therapeutic uses of the invention as combinatorially formulated (e.g., conjugated) and/or coordinately administered agents to enhance receptor-mediated transport of biologically active agents, including glucose-regulating peptide proteins.

## Polymeric DeUvery Vehicles and Methods

Within certain aspects of the invention, glucose-regulating peptide proteins, other biologically active agents disclosed herein, and delivery-enhancing agents as described above, may be individually or combinatorially, incorporated within a mucosally (e.g., nasally) administered formulation that includes a biocompatible polymer functioning as a carrier or base. Such polymer carriers include polymeric powders, matrices or microparticulate delivery vehicles, among other polymer forms. The polymer can be of plant, animal, or synthetic origin. Often the polymer is crosslinked. Additionally, in these delivery systems the glucose-regulating peptide, can be functionalized in a manner where it can be covalently bound to the polymer and rendered inseparable from the polymer by simple washing. In other embodiments, the polymer is chemically modified with an inhibitor of enzymes or other agents which may degrade or inactivate the biologically active agent(s) and/or delivery enhancing agent(s): In certain formulations, the polymer is a partially or completely water insoluble but water swellable polymer, e.g., a hydrogel. Polymers useful in this aspect of the invention are desirably water interactive and/or hydrophilic in nature to absorb significant quantities of water, and they often form hydrogels when placed in contact with water or aqueous media for a period of time sufficient to reach equilibrium with water. In more detailed embodiments, the polymer is a hydrogel which, when placed in contact with excess water, absorbs at least two times its weight of water at equilibrium when exposed to water at room temperature, U.S. Patent No. 6,004,583.

Drug delivery systems based on biodegradable polymers are preferred in many biomedical applications because such systems are broken down either by hydrolysis or by enzymatic reaction into non-toxic molecules. The rate of degradation is controlled by manipulating the composition of the biodegradable polymer matrix. These types of systems can therefore be employed in certain settings for long-term release of biologically active agents. Biodegradable polymers such as poly(glycolic acid) (PGA), poly-(lactic acid) (PLA), and poly(D,L-lactic-co-glycolic acid) (PLGA), have received considerable attention as possible drug delivery carriers, since the degradation products of these polymers have been found to have low toxicity. During the normal metabolic function of the body these polymers degrade into carbon dioxide and water. These polymers have also exhibited excellent biocompatibility.

For prolonging the biological activity of glucose-regulating peptide, analogs and mimetics, and other biologically active agents disclosed herein, as well as optional delivery-enhancing agents, these agents may be incorporated into polymeric matrices, e.g., polyorthoesters, polyanhydrides, or polyesters. This yields sustained activity and release of the active agent(s), e.g., as determined by the degradation of the polymer matrix. Although the encapsulation of biotherapeutic molecules inside synthetic polymers may stabilize them during storage and delivery, the largest obstacle of polymer-based release technology is the activity loss of the therapeutic molecules during the formulation processes that often involve heat, sonication or organic solvents.

Absorption-promoting polymers contemplated for use within the invention may include derivatives and chemically or physically modified versions of the foregoing types of polymers, in addition to other naturally occurring or synthetic polymers, gums, resins, and other agents, as well as blends of these materials with each other or other polymers, so long as the alterations, modifications or blending do not adversely affect the desired properties, such as water absorption, hydrogel formation, and/or chemical stability for useful application. In more detailed aspects of the invention, polymers such as nylon, acrylan and other normally hydrophobic synthetic polymers may be sufficiently modified by reaction to become water swellable and/or form stable gels in aqueous media.

Absorption-promoting polymers of the invention may include polymers from the group of homo- and copolymers based on various combinations of the following vinyl monomers: acrylic and methacrylic acids, acrylamide, methacrylamide, hydroxyethylacrylate or methacrylate, vinylpyrrolidones, as well as polyvinylalcohol and its co- and terpolymers, polyvinylacetate, its co- and terpolymers with the above listed monomers and 2-acrylamido-2-methyl-propanesulfonic acid (AMPS®). Very useful are copolymers of the above listed monomers with copolymerizable functional monomers such as acryl or methacryl amide acrylate or methacrylate esters where the ester groups are derived from straight or branched chain alkyl, aryl having up to four aromatic rings which may contain alkyl substituents of 1 to 6 carbons; steroidal, sulfates, phosphates or cationic monomers such as N,N-dimethylaminoalkyl(meth)acrylamide, dimethylaminoalkyl(meth)acrylate, (meth)acryloxyalkyltrimethylammonium chloride, (meth)acryloxyalkyldimethylbenzyl ammonium chloride.

Additional absorption-promoting polymers for use within the invention are those classified as dextrans, dextrins, and from the class of materials classified as natural gums and resins, or from the class of natural polymers such as processed collagen, chitin, chitosan, pullalan, zooglan, alginates and modified alginates such as "Kelcoloid" (a polypropylene glycol modified alginate) gellan gums such as "Kelocogel", Xanathan gums such as "Keltrol", estastin, alpha hydroxy butyrate and its copolymers, hyaluronic acid and its derivatives, polylactic and glycolic acids.

A very useful class of polymers applicable within the instant invention are olefinically-unsaturated carboxylic acids containing at least one activated carbon-to-carbon olefinic double bond, and at least one carboxyl group; that is, an acid or functional group readily converted to an acid containing an olefinic double bond which readily functions in polymerization because of its presence in the monomer molecule, either in the alpha-beta position with respect to a carboxyl group, or as part of a terminal methylene grouping. Olefinically-unsaturated acids of this class include such materials as the acrylic acids typified by the acrylic acid itself, alpha-cyano acrylic acid, beta methylacrylic acid (crotonic acid), alpha-phenyl acrylic acid, beta-acryloxy propionic acid, cinnamic acid, p-chloro cinnamic acid, 1-carboxy-4-phenyl butadiene-1,3, itaconic acid, citraconic acid, mesaconic acid, glutaconic acid, aconitic acid, maleic acid, fumaric acid, and tricarboxy ethylene. As used herein, the term "carboxylic acid" includes the polycarboxylic acids and those acid anhydrides, such as maleic anhydride, wherein the anhydride group is formed by the elimination of one molecule of water from two carboxyl groups located on the same carboxylic acid molecule.

Representative acrylates useful as absorption-promoting agents within the invention include methyl acrylate, ethyl acrylate, propyl acrylate, isopropyl acrylate, butyl acrylate, isobutyl acrylate, methyl methacrylate, methyl ethacrylate, ethyl methacrylate, octyl acrylate, heptyl acrylate, octyl methacrylate, isopropyl methacrylate, 2-ethylhexyl methacrylate, nonyl acrylate, hexyl acrylate, n-hexyl methacrylate, and the like. Higher alkyl acrylic esters are decyl acrylate, isodecyl methacrylate, lauryl acrylate, stearyl acrylate, behenyl acrylate and melissyl acrylate and methacrylate versions thereof. Mixtures of two or three or more long chain acrylic esters may be successfully polymerized with one of the carboxylic monomers. Other comonomers include olefins, including alpha olefins, vinyl ethers, vinyl esters, and mixtures thereof.

Other vinylidene monomers, including the acrylic nitriles, may also be used as absorption-promoting agents within the therapeutic uses and compositions of the invention to enhance delivery and absorption of one or more glucose-regulating peptide proteins, analogs and mimetics, and other biologically active agent(s), including to enhance delivery of the active agent(s) to a target tissue or compartment in the subject (e.g., the liver, hepatic portal vein, CNS tissue or fluid, or blood plasma). Useful alpha, beta-olefinically unsaturated nitriles are preferably monoolefinically unsaturated nitriles having from 3 to 10 carbon atoms such as acrylonitrile, methacrylonitrile, and the like. Most preferred are acrylonitrile and methacrylonitrile. Acrylic amides containing from 3 to 35 carbon atoms including monoolefinically unsaturated amides also may be used. Representative amides include acrylamide, methacrylamide, N-t-butyl acrylamide, N-cyclohexyl acrylamide, higher alkyl amides, where the alkyl group on the nitrogen contains from 8 to 32 carbon atoms, acrylic amides including N-alkylol amides of alpha, beta-olefinically unsaturated carboxylic acids including those having from 4 to 10 carbon atoms such as N-methylol acrylamide, N-propanol acrylamide, N-methylol methacrylamide, N-methylol maleimide, N-methylol maleamic acid esters, N-methylol-p-vinyl benzamide, and the like.

Yet additional useful absorption promoting materials are alpha-olefins containing from 2 to 18 carbon atoms, more preferably from 2 to 8 carbon atoms; dienes containing from 4 to 10 carbon atoms; vinyl esters and allyl esters such as vinyl acetate; vinyl aromatics such as styrene, methyl styrene and chloro-styrene; vinyl and allyl ethers and ketones such as vinyl methyl ether and methyl vinyl ketone; chloroacrylates; cyanoalkyl acrylates such as alpha-cyanomethyl acrylate, and the alpha-, beta-, and gamma-cyanopropyl acrylates; alkoxyacrylates such as methoxy ethyl acrylate; haloacrylates as chloroethyl acrylate; vinyl halides and vinyl chloride, vinylidene chloride and the like; divinyls, diacrylates and other polyfunctional monomers such as divinyl ether, diethylene glycol diacrylate, ethylene glycol dimethacrylate, methylene-bis-acrylamide, allylpentaerythritol, and the like; and bis (beta-haloalkyl) alkenyl phosphonates such as bis(beta-chloroethyl) vinyl phosphonate and the like as are known to those skilled in the art. Copolymers wherein the carboxy containing monomer is a minor constituent, and the other vinylidene monomers present as major components are readily prepared in accordance with the methods disclosed herein.

When hydrogels are employed as absorption promoting agents within the invention, these may be composed of synthetic copolymers from the group of acrylic and methacrylic acids, acrylamide, methacrylamide, hydroxyethylacrylate (HEA) or methacrylate (HEMA), and vinylpyrrolidones which are water interactive and swellable. Specific illustrative examples of useful polymers, especially for the delivery of peptides or proteins, are the following types of polymers: (meth)acrylamide and 0.1 to 99 wt. % (meth)acrylic acid; (meth)acrylamides and 0.1-75 wt % (meth)acryloxyethyl trimethyammonium chloride; (meth)acrylamide and 0.1-75 wt % (meth)acrylamide; acrylic acid and 0.1-75 wt % alkyl(meth) acrylates; (meth)acrylamide and 0.1-75 wt % AMPS.RTM. (trademark of Lubrizol Corp.); (meth)acrylamide and 0 to 30 wt % alkyl(meth)acrylamides and 0.1-75 wt % AMPS.RTM.; (meth)acrylamide and 0.1-99 wt. % HEMA; (metb)acrylamide and 0.1 to 75 wt % HEMA and 0.1 to 99%(meth)acrylic acid; (meth)acrylic acid and 0.1-99 wt % HEMA; 50 mole % vinyl ether and 50 mole % maleic anhydride; (meth)acrylamide and 0.1 to 75 wt % (meth)acryloxyalky dimethyl benzylammonium chloride; (meth)acrylamide and 0.1 to 99 wt % vinyl pyrrolidone; (meth)acrylamide and 50 wt % vinyl pyrrolidone and 0.1-99.9 wt % (meth)acrylic acid; (meth)acrylic acid and 0.1 to 75 wt % AMPS.RTM. and 0.1-75 wt % alkyl(meth) acrylamide. In the above examples, alkyl means $C_1$ to $C_{30}$, preferably $C_1$ to $C_{22}$, linear and branched and $C_4$ to $C_{16}$ cyclic; where (meth) is used, it means that the monomers with and without the methyl group are included. Other very useful hydrogel polymers are swellable, but insoluble versions of poly(vinyl pyrrolidone) starch, carboxymethyl cellulose and polyvinyl alcohol.

Additional polymeric hydrogel materials useful within the invention include (poly) hydroxyalkyl (meth)acrylate: anionic and cationic hydrogels: poly(electrolyte) complexes; poly(vinyl alcohols) having a low acetate residual: a swellable mixture of crosslinked agar and crosslinked carboxymethyl cellulose: a swellable composition comprising methyl cellulose mixed with a sparingly crosslinked agar; a water swellable copolymer produced by a dispersion of finely divided copolymer of maleic anhydride with styrene, ethylene, propylene, or isobutylene; a water swellable polymer of N-vinyl lactams; swellable sodium salts of carboxymethyl cellulose; and the like.

Other gelable, fluid imbibing and retaining polymers useful for forming the hydrophilic hydrogel for mucosal delivery of biologically active agents within the invention include pectin; polysaccharides such as agar, acacia, karaya, tragacenth, algins and guar and their crosslinked versions; acrylic acid polymers, copolymers and salt derivatives, polyacrylamides; water swellable indene maleic anhydride polymers; starch graft copolymers; acrylate type polymers and copolymers with water absorbability of about 2 to 400 times its original weight; diesters of polyglucan; a mixture of crosslinked poly(vinyl alcohol) and poly(N-vinyl-2-pyrrolidone); polyoxybutylene-polyethylene block copolymer gels; carob gum; polyester gels; poly urea gels; polyether gels; polyamide gels; polyimide gels; polypeptide gels; polyamino acid gels; poly cellulosic gels; crosslinked indene-maleic anhydride acrylate polymers; and polysaccharides.

Synthetic hydrogel polymers for use within the invention may be made by an infinite combination of several monomers in several ratios. The hydrogel can be crosslinked and generally possesses the ability to imbibe and absorb fluid and swell or expand to an enlarged equilibrium state. The hydrogel typically swells or expands upon delivery to the nasal mucosal surface, absorbing about 2-5, 5-10, 10-50, up to 50-100 or more times fold its weight of water. The optimum degree of swellability for a given hydrogel will be determined for different biologically active agents depending upon such factors as molecular weight, size, solubility and diffusion characteristics of the active agent carried by or entrapped or encapsulated within the polymer, and the specific spacing and cooperative chain motion associated with each individual polymer.

Hydrophilic polymers useful within the invention are water insoluble but water swellable. Such water-swollen polymers as typically referred to as hydro gels or gels. Such gels may be conveniently produced from water-soluble polymer by the process of crosslinking the polymers by a suitable crosslinking agent. However, stable hydrogels may also be formed from specific polymers under defined conditions of pH, temperature and/or ionic concentration, according to know methods in the art. Typically the polymers are cross-linked, that is, cross-linked to the extent that the polymers possess good hydrophilic properties, have improved physical integrity (as compared to non cross-linked polymers of the same or similar type) and exhibit improved ability to retain within the gel network both the biologically active agent of interest and additional compounds for coadministration therewith such as a cytokine or enzyme inhibitor, while retaining the ability to release the active agent(s) at the appropriate location and time.

Generally hydrogel polymers for use within the invention are crosslinked with a difunctional cross-linking in the amount of from 0.01 to 25 weight percent, based on the weight of the monomers forming the copolymer, and more preferably from 0.1 to 20 weight percent and more often from 0.1 to 15 weight percent of the crosslinking agent. Another useful amount of a crosslinking agent is 0.1 to 10 weight percent. Tri, tetra or higher multifunctional crosslinking agents may also be employed. When such reagents are utilized, lower amounts may be required to attain equivalent crosslinking density, *i.e.*, the degree of crosslinking, or network properties that are sufficient to contain effectively the biologically active agent(s).

The crosslinks can be covalent, ionic or hydrogen bonds with the polymer possessing the ability to swell in the presence of water containing fluids. Such crosslinkers and crosslinking reactions are known to those skilled in the art and in many cases are dependent upon the polymer system. Thus a crosslinked network may be formed by free radical copolymerization of unsaturated monomers. Polymeric hydrogels may also be formed by crosslinking preformed polymers by reacting functional groups found on the polymers such as alcohols, acids, amines with such groups as glyoxal, formaldehyde or glutaraldehyde, bis anhydrides and the like.

The polymers also may be cross-linked with any polyene, e.g. decadiene or trivinyl cyclohexane; acrylamides, such as N,N-methylene-bis (acrylamide); polyfunctional acrylates, such as trimethylol propane triacrylate; or polyfunctional vinylidene monomer containing at least 2 terminal $CH_2 <$ groups, including, for example, divinyl benzene, divinyl naphthlene, allyl acrylates and the like. In certain embodiments, cross-linking monomers for use in preparing the copolymers are polyalkenyl polyethers having more than one alkenyl ether grouping per molecule, which may optionally possess alkenyl groups in which an olefinic double bond is present attached to a terminal methylene grouping (e.g., made by the etherification of a polyhydric alcohol containing at least 2 carbon atoms and at least 2 hydroxyl groups). Compounds of this class may be produced by reacting an alkenyl halide, such as allyl chloride or allyl bromide, with a strongly alkaline aqueous solution of one or more polyhydric alcohols. The product may be a complex mixture of polyethers with varying numbers of ether groups. Efficiency of the polyether cross-linking agent increases with the number of potentially polymerizable groups on the molecule. Typically, polyethers containing an average of two or more alkenyl ether groupings per molecule are used. Other cross-linking monomers include for example, diallyl esters, dimethallyl ethers, allyl or methallyl acrylates and acrylamides, tetravinyl silane, polyalkenyl methanes, diacrylates, and dimethacrylates, divinyl compounds such as divinyl benzene, polyallyl phosphate, diallyloxy compounds and phosphite esters and the like. Typical agents are allyl pentaerythritol, allyl sucrose, trimethylolpropane triacrylate, 1,6-hexanediol diacrylate, trimethylolpropane diallyl ether, pentaerythritol triacrylate, tetramethylene dimethacrylate, ethylene diacrylate, ethylene dimethacrylate, triethylene glycol dimethacrylate, and the like. Allyl pentaerythritol, trimethylolpropane diallylether and allyl sucrose provide suitable polymers. When the cross-linking agent is present, the polymeric mixtures usually contain between about 0.01 to 20 weight percent, e.g., 1%, 5%, or 10% or more by weight of cross-linking monomer based on the total of carboxylic acid monomer, plus other monomers.

In more detailed aspects of the invention, mucosal delivery of glucose-regulating peptide, analogs and mimetics, and other biologically active agents disclosed herein, is enhanced by retaining the active agent(s) in a slow-release or enzymatically or physiologically protective carrier or vehicle, for example a hydrogel that shields the active agent from the action of the degradative enzymes. In certain embodiments, the active agent is bound by chemical means to the carrier or vehicle, to which may also be admixed or bound additional agents such as enzyme inhibitors, cytokines, etc. The active agent may alternately be immobilized through sufficient physical entrapment within the carrier or vehicle, e.g., a polymer matrix.

Polymers such as hydrogels useful within the invention may incorporate functional linked agents such as glycosides chemically incorporated into the polymer for enhancing intranasal bioavailability of active agents formulated therewith. Examples of such glycosides are glucosides, fructosides, galactosides, arabinosides, mannosides and their alkyl substituted derivatives and natural glycosides such as arbutin, phlorizin, amygdalin, digitonin, saponin, and indican. There are several ways in which a typical glycoside may be bound to a polymer. For example, the hydrogen of the hydroxyl groups of a glycoside or other similar carbohydrate may be replaced by the alkyl group from a hydrogel polymer to form an ether. Also, the hydroxyl groups of the glycosides may be reacted to esterify the carboxyl groups of a polymeric hydrogel to form polymeric esters *in situ.* Another approach is to employ condensation of acetobromoglucose with cholest-5-en-3beta-ol on a copolymer of maleic acid. N-substituted polyacrylamides can be synthesized by the reaction of activated polymers with omega-aminoalkylglycosides: (1) (carbohydrate-spacer)(n)-polyacrylamide, 'pseudopolysaccharides'; (2) (carbohydrate spacer)(n)-phosphatidylethanolamine(m)-polyacrylamide, neoglycolipids, derivatives of phosphatidylethanolamine; (3) (carbohydrate-spacer)(n)-biotin(m)-polyacrylamide. These biotinylated derivatives may attach to lectins on the mucosal surface to facilitate absorption of the biologically active agent(s), e.g., a polymer-encapsulated glucose-regulating peptide.

Within more detailed aspects of the invention, one or more glucose-regulating peptide, optionally including secondary active agents such as protease inhibitor(s), cytokine(s), additional modulator(s) of intercellular junctional physiology, etc., are modified and bound to a polymeric carrier or matrix. For example, this may be accomplished by chemically binding a peptide or protein active agent and other optional agent(s) within a crosslinked polymer network. It is also possible to chemically modify the polymer separately with an interactive agent such as a glycosidal containing molecule. In certain aspects, the biologically active agent(s), and optional secondary active agent(s), may be functionalized, i.e., wherein an appropriate reactive group is identified or is chemically added to the active agent(s). Most often an ethylenic polymerizable group is added, and the functionalized active agent is then copolymerized with monomers and a crosslinking agent using a standard polymerization method such as solution polymerization (usually in water), emulsion, suspension or dispersion polymerization. Often, the functionalizing agent is provided with a high enough concentration of functional or polymerizable groups to insure that several sites on the active agent(s) are functions. For example, in a polypeptide comprising 16 amine sites, it is generally desired to functionalize at least 2, 4, 5, 7, and up to 8 or more of the sites.

After functionalization, the functionalized active agent(s) is/are mixed with monomers and a crosslinking agent that comprise the reagents from which the polymer of interest is formed. Polymerization is then induced in this medium to create a polymer containing the bound active agent(s). The polymer is then washed with water or other appropriate solvents and otherwise purified to remove trace unreacted impurities and, if necessary, ground or broken up by physical means such as by stirring, forcing it through a mesh, ultrasonication or other suitable means to a desired particle size. The solvent, usually water, is then removed in such a manner as to not denature or otherwise degrade the active agent(s). One desired method is lyophilization (freeze drying) but other methods are available and may be used (e.g., vacuum drying, air drying, spray drying, etc.).

To introduce polymerizable groups in peptides, proteins and other active agents within the invention, it is possible to react available amino, hydroxyl, thiol and other reactive groups with electrophiles containing unsaturated groups. For example, unsaturated monomers containing N-hydroxy succinimidyl groups, active carbonates such as p-nitrophenyl carbonate, trichlorophenyl carbonates, tresylate, oxycarbonylimidazoles, epoxide, isocyanates and aldehyde, and unsaturated carboxymethyl azides and unsaturated orthopyridyl-disulfide belong to this category of reagents. Illustrative examples of unsaturated reagents are allyl glycidyl ether, allyl chloride, allylbromide, allyl iodide, acryloyl chloride, allyl isocyanate, allylsulfonyl chloride, maleic anhydride, copolymers of maleic anhydride and allyl ether, and the like.

All of the lysine active derivatives, except aldehyde, can generally react with other amino acids such as imidazole groups of histidine and hydroxyl groups of tyrosine and the thiol groups of cystine if the local environment enhances nucleophilicity of these groups. Aldehyde containing functionalizing reagents are specific to lysine. These types of reactions with available groups from lysines, cysteines, tyrosine have been extensively documented in the literature and are known to those skilled in the art.

In the case of biologically active agents that contain amine groups, it is convenient to react such groups with an acyloyl chloride, such as acryloyl chloride, and introduce the polymerizable acrylic group onto the reacted agent. Then during preparation of the polymer, such as during the crosslinking of the copolymer of acrylamide and acrylic acid, the functionalized active agent, through the acrylic groups, is attached to the polymer and becomes bound thereto.

In additional aspects of the invention, biologically active agents, including peptides, proteins, nucleosides, and other molecules which are bioactive *in vivo,* are conjugation-stabilized by covalently bonding one or more active agent(s) to a polymer incorporating as an integral part thereof both a hydrophilic moiety, e.g., a linear polyalkylene glycol, a lipophilic moiety (see, e.g., U.S. Patent No. 5,681,811). In one aspect, a biologically active agent is covalently coupled with a polymer comprising (i) a linear polyalkylene glycol moiety and (ii) a lipophilic moiety, wherein the active agent, linear polyalkylene glycol moiety, and the lipophilic moiety are conformationally arranged in relation to one another such that the active therapeutic agent has an enhanced *in vivo* resistance to enzymatic degradation (i.e., relative to its stability under similar conditions in an unconjugated form devoid of the polymer coupled thereto). In another aspect, the conjugation-stabilized formulation has a three-dimensional conformation comprising the biologically active agent covalently coupled with a polysorbate complex comprising (i) a linear polyalkylene glycol moiety and (ii) a lipophilic moiety, wherein the active agent, the linear polyalkylene glycol moiety and the lipophilic moiety are conformationally arranged in relation to one another such that (a) the lipophilic moiety is exteriorly available in the three-dimensional conformation, and (b) the active agent in the composition has an enhanced *in vivo* resistance to enzymatic degradation.

In a further related aspect, a multiligand conjugated complex is provided which comprises a biologically active agent covalently coupled with a triglyceride backbone moiety through a polyalkylene glycol spacer group bonded at a carbon atom of the triglyceride backbone moiety, and at least one fatty acid moiety covalently attached either directly to a carbon atom of the triglyceride backbone moiety or covalently joined through a polyalkylene glycol spacer moiety (see, e.g., U.S. Patent No. 5,681,811). In such a multiligand conjugated therapeutic agent complex, the alpha' and beta carbon atoms of the triglyceride bioactive moiety may have fatty acid moieties attached by covalently bonding either directly thereto, or indirectly covalently bonded thereto through polyalkylene glycol spacer moieties. Alternatively, a fatty acid moiety may be covalently attached either directly or through a polyalkylene glycol spacer moiety to the alpha and alpha' carbons of the triglyceride backbone moiety, with the bioactive therapeutic agent being covalently coupled with the gamma-carbon of the triglyceride backbone moiety, either being directly covalently bonded thereto or indirectly bonded thereto through a polyalkylene spacer moiety. It will be recognized that a wide variety of structural, compositional, and conformational forms are possible for the multiligand conjugated therapeutic agent complex comprising the triglyceride backbone moiety, within the scope of the invention. It is further noted that in such a multiligand conjugated therapeutic agent complex, the biologically active agent(s) may advantageously be covalently coupled with the triglyceride modified backbone moiety through alkyl spacer groups, or alternatively other acceptable spacer groups, within the scope of the invention. As used in such context, acceptability of the spacer group refers to steric, compositional, and end use application specific acceptability characteristics.

In yet additional aspects of the invention, a conjugation-stabilized complex is provided which comprises a polysorbate complex comprising a polysorbate moiety including a triglyceride backbone having covalently coupled to alpha, alpha and beta carbon atoms thereof functionalizing groups including (i) a fatty acid group; and (ii) a polyethylene glycol group having a biologically active agent or moiety covalently bonded thereto, e.g., bonded to an appropriate functionality of the polyethylene glycol group. Such covalent bonding may be either direct, e.g., to a hydroxy terminal functionality of the polyethylene glycol group, or alternatively, the covalent bonding may be indirect, e.g., by reactively capping the hydroxy terminus of the polyethylene glycol group with a terminal carboxy functionality spacer group, so that the resulting capped polyethylene glycol group has a terminal carboxy functionality to which the biologically active agent or moiety may be covalently bonded.

In yet additional aspects of the invention, a stable, aqueously soluble, conjugation-stabilized complex is provided which comprises one or more glucose-regulating peptide proteins, analogs and mimetics, and/or other biologically active agent(s)+ disclosed herein covalently coupled to a physiologically compatible polyethylene glycol (PEG) modified glycolipid moiety. In such complex, the biologically active agent(s) may be covalently coupled to the physiologically compatible PEG modified glycolipid moiety by a labile covalent bond at a free amino acid group of the active agent, wherein the labile covalent bond is scissionable *in vivo* by biochemical hydrolysis and/or proteolysis. The physiologically compatible PEG modified glycolipid moiety may advantageously comprise a polysorbate polymer, e.g., a polysorbate polymer comprising fatty acid ester groups selected from the group consisting of monopalmitate, dipalmitate, monolaurate, dilaurate, trilaurate, monoleate, dioleate, trioleate, monostearate, distearate, and tristearate. In such complex, the physiologically compatible PEG modified glycolipid moiety may suitably comprise a polymer selected from the group consisting of polyethylene glycol ethers of fatty acids, and polyethylene glycol esters of fatty acids, wherein the fatty acids for example comprise a fatty acid selected from the group consisting of lauric, palmitic, oleic, and stearic acids.

**Storage of Material**

In certain aspects of the invention, the combinatorial formulations and/or coordinate therapeutic uses herein incorporate an effective amount of peptides and proteins which may adhere to charged glass thereby reducing the effective concentration in the container. Silanized containers, for example, silanized glass containers, are used to store the finished product to reduce adsorption of the polypeptide or protein to a glass container.

In yet additional aspects of the invention, a kit comprises a stable pharmaceutical composition of one or more glucose-regulating peptide compound(s) formulated for mucosal delivery to the mammalian subject wherein the composition is effective to alleviate one or more symptom(s) of obesity, cancer, or malnutrition or washing related to cancer in a subject without unacceptable adverse side effects. The kit further comprises a pharmaceutical reagent vial to contain the one or more glucose-regulating peptide compounds. The pharmaceutical reagent vial is composed of pharmaceutical grade polymer, glass or other suitable material. The pharmaceutical reagent vial is, for example, a silanized glass vial. The kit further comprises an aperture for delivery of the composition to a nasal mucosal surface of the subject. The delivery aperture is composed of a pharmaceutical grade polymer, glass or other suitable material. The delivery aperture is, for example, a silanized glass.

A silanization technique combines a special cleaning technique for the surfaces to be silanized with a silanization process at low pressure. The silane is in the gas phase and at an enhanced temperature of the surfaces to be silanized. The method provides reproducible surfaces with stable, homogeneous and functional silane layers having characteristics of a monolayer. The silanized surfaces prevent binding to the glass of polypeptides or mucosal delivery enhancing agents of the present invention..

The procedure is useful to prepare silanized pharmaceutical reagent vials to hold glucose-regulating peptide compositions of the present invention. Glass trays are cleaned by rinsing with double distilled water (ddH$_2$O) before using. The silane tray is then be rinsed with 95% EtOH, and the acetone tray is rinsed with acetone. Pharmaceutical reagent vials are sonicated in acetone for 10 minutes. After the acetone sonication, reagent vials are washed in ddH$_2$O tray at least twice. Reagent vials are sonicated in 0.1M NaOH for 10 minutes. While the reagent vials are sonicating in NaOH, the silane solution is made under a hood. (Silane solution: 800 mL of 95% ethanol; 96L of glacial acetic acid; 25 mL of glycidoxypropyltrimethoxy silane). After the NaOH sonication, reagent vials are washed in ddH$_2$O tray at least twice. The reagent vials are sonicated in silane solution for 3 to 5 minutes. The reagent vials are washed in 100% EtOH tray. The reagent vials are dried with prepurified N$_2$ gas and stored in a 100°C oven for at least 2 hours before using.

**Bioadhesive Delivery Vehicles and Methods**

In certain aspects of the invention, the combinatorial formulations and/or coordinate therapeuctic uses herein may incorporate an effective amount of a nontoxic bioadhesive as an adjunct compound or carrier to enhance mucosal delivery of one or more biologically active agent(s). Bioadhesive agents in this context exhibit general or specific adhesion to one or more components or surfaces of the targeted mucosa. The bioadhesive maintains a desired concentration gradient of the biologically active agent into or across the mucosa to ensure penetration of even large molecules (e.g., peptides and proteins) into or through the mucosal epithelium. Typically, employment of a bioadhesive within the methods and compositions of the invention yields a two- to five- fold, often a five- to ten-fold increase in permeability for peptides and proteins into or through the mucosal epithelium. This enhancement of epithelial permeation often permits effective transmucosal delivery of large macromolecules, for example to the basal portion of the nasal epithelium or into the adjacent extracellular compartments or a blood plasma or CNS tissue or fluid.

This enhanced delivery provides for greatly improved effectiveness of delivery of bioactive peptides, proteins and other macromolecular therapeutic species. These results will depend in part on the hydrophilicity of the compound, whereby greater penetration will be achieved with hydrophilic species compared to water insoluble compounds. In addition to these effects, employment of bioadhesives to enhance drug persistence at the mucosal surface can elicit a reservoir mechanism for protracted drug delivery, whereby compounds not only penetrate across the mucosal tissue but also back-diffuse toward the mucosal surface once the material at the surface is depleted.

A variety of suitable bioadhesives are disclosed in the art for oral administration, U.S. Patent No.s 3,972,995; 4,259,314; 4,680,323; 4,740,365; 4,573,996; 4,292,299; 4,715,369; 4,876,092; 4,855,142; 4,250,163; 4,226,848; 4,948,580; U.S. Patent Reissue 33,093, which find use within the therapeutic uses and compositions of the invention. The potential of various bioadhesive polymers as a mucosal, e.g., nasal, delivery platform within the methods and compositions of the invention can be readily assessed by determining their ability to retain and release glucose-regulating peptide, as well as by their capacity to interact with the mucosal surfaces following incorporation of the active agent therein. In addition, well known methods will be applied to determine the biocompatibility of selected polymers with the tissue at the site of mucosal administration. When the target mucosa is covered by mucus (i.e., in the absence of mucolytic or mucus-clearing treatment), it can serve as a connecting link to the underlying mucosal epithelium. Therefore, the term "bioadhesive" as used herein also covers mucoadhesive compounds useful for enhancing mucosal delivery of biologically active agents within the invention. However, adhesive contact to mucosal tissue mediated through adhesion to a mucus gel layer may be limited by incomplete or transient attachment between the mucus layer and the underlying tissue, particularly at nasal surfaces where rapid mucus clearance occurs. In this regard, mucin glycoproteins are continuously secreted and, immediately after their release from cells or glands, form a viscoelastic gel. The luminal surface of the adherent gel layer, however, is continuously eroded by mechanical, enzymatic and/or ciliary action. Where such activities are more prominent or where longer adhesion times are desired, the coordinate therapeutic uses and combinatorial formulation methods of the invention may further corporate mucolytic and/or ciliostatic methods or agents as disclosed herein above.

Typically, mucoadhesive polymers which may be use within the invention are natural or synthetic macromolecules which adhere to wet mucosal tissue surfaces by complex, but non-specific, mechanisms. In addition to these mucoadhesive polymers, the invention also provides therapeutic uses and compositions incorporating bioadhesives that adhere directly to a cell surface, rather than to mucus, by means of specific, including receptor-mediated, interactions. One example of bioadhesives that function in this specific manner is the group of compounds known as lectins. These are glycoproteins with an ability to specifically recognize and bind to sugar molecules, e.g. glycoproteins or glycolipids, which form part of intranasal epithelial cell membranes and can be considered as "lectin receptors".

In certain aspects of the invention, bioadhesive materials for enhancing intranasal delivery of biologically active agents comprise a matrix of a hydrophilic, e.g., water soluble or swellable, polymer or a mixture of polymers that can adhere to a wet mucous surface. These adhesives may be formulated as ointments, hydro gels (see above) thin films, and other application forms. Often, these adhesives have the biologically active agent mixed therewith to effectuate slow release or local delivery of the active agent. Some are formulated with additional ingredients to facilitate penetration of the active agent through the nasal mucosa, e.g., into the circulatory system of the individual.

Various polymers, both natural and synthetic ones, show significant binding to mucus and/or mucosal epithelial surfaces under physiological conditions. The strength of this interaction can readily be measured by mechanical peel or shear tests. When applied to a humid mucosal surface, many dry materials will spontaneously adhere, at least slightly. After such an initial contact, some hydrophilic materials start to attract water by adsorption, swelling or capillary forces, and if this water is absorbed from the underlying substrate or from the polymer-tissue interface, the adhesion may be sufficient to achieve the goal of enhancing mucosal absorption of biologically active agents. Such 'adhesion by hydration' can be quite strong, but formulations adapted to employ this mechanism must account for swelling which continues as the dosage transforms into a hydrated mucilage. This is projected for many hydrocolloids useful within the invention, especially some cellulose-derivatives, which are generally non-adhesive when applied in pre-hydrated state. Nevertheless, bioadhesive drug delivery systems for mucosal administration are effective within the invention when such materials are applied in the form of a dry polymeric powder, microsphere, or film-type delivery form.

Other polymers adhere to mucosal surfaces not only when applied in dry, but also in fully hydrated state, and in the presence of excess amounts of water. The selection of a mucoadhesive thus requires due consideration of the conditions, physiological as well as physico-chemical, under which the contact to the tissue will be formed and maintained. In particular, the amount of water or humidity usually present at the intended site of adhesion, and the prevailing pH, are known to largely affect the mucoadhesive binding strength of different polymers.

Several polymeric bioadhesive drug delivery systems have been fabricated and studied in the past 20 years, not always with success. A variety of such carriers are, however, currently used in clinical applications involving dental, orthopedic, ophthalmological, and surgical uses. For example, acrylic-based hydrogels have been used extensively for bioadhesive devices. Acrylic-based hydrogels are well suited for bioadhesion due to their flexibility and nonabrasive characteristics in the partially swollen state, which reduce damage-causing attrition to the tissues in contact. Furthermore, their high permeability in the swollen state allows unreacted monomer, un-crosslinked polymer chains, and the initiator to be washed out of the matrix after polymerization, which is an important feature for selection of bioadhesive materials for use within the invention. Acrylic-based polymer devices exhibit very high adhesive bond strength. For controlled mucosal delivery of peptide and protein drugs, the methods and compositions of the invention optionally include the use of carriers, e.g., polymeric delivery vehicles, that function in part to shield the biologically active agent from proteolytic breakdown, while at the same time providing for enhanced penetration of the peptide or protein into or through the nasal mucosa. In this context, bioadhesive polymers have demonstrated considerable potential for enhancing oral drug delivery. As an example, the bioavailability of 9-desglycinamide, 8-arginine vasopressin (DGAVP) intraduodenally administered to rats together with a 1% (w/v) saline dispersion of the mucoadhesive poly(acrylic acid) derivative polycarbophil, is 3-5-fold increased compared to an aqueous solution of the peptide drug without this polymer.

Mucoadhesive polymers of the poly(acrylic acid)-type are potent inhibitors of some intestinal proteases. The mechanism of enzyme inhibition is explained by the strong affinity of this class of polymers for divalent cations, such as calcium or zinc, which are essential cofactors of metallo-proteinases, such as trypsin and chymotrypsin. Depriving the proteases of their cofactors by poly(acrylic acid) is reported to induce irreversible structural changes of the enzyme proteins which were accompanied by a loss of enzyme activity. At the same time, other mucoadhesive polymers (e.g., some cellulose derivatives and chitosan) may not inhibit proteolytic enzymes under certain conditions. In contrast to other enzyme inhibitors contemplated for use within the invention (e.g. aprotinin, bestatin), which are relatively small molecules, the trans-nasal absorption of inhibitory polymers is likely to be minimal in light of the size of these molecules, and thereby eliminate possible adverse side effects. Thus, mucoadhesive polymers, particularly of the poly(acrylic acid)-type, may serve both as an absorption-promoting adhesive and enzyme-protective agent to enhance controlled delivery of peptide and protein drugs, especially when safety concerns are considered.

In addition to protecting against enzymatic degradation, bioadhesives and other polymeric or non-polymeric absorption-promoting agents for use within the invention may directly increase mucosal permeability to biologically active agents. To facilitate the transport of large and hydrophilic molecules, such as peptides and proteins, across the nasal epithelial barrier, mucoadhesive polymers and other agents have been postulated to yield enhanced permeation effects beyond what is accounted for by prolonged premucosal residence time of the delivery system. The time course of drug plasma concentrations reportedly suggested that the bioadhesive microspheres caused an acute, but transient increase of insulin permeability across the nasal mucosa. Other mucoadhesive polymers for use within the invention, for example chitosan, reportedly enhance the permeability of certain mucosal epithelia even when they are applied as an aqueous solution or gel. Another mucoadhesive polymer reported to directly affect epithelial permeability is hyaluronic acid and ester derivatives thereof. A particularly useful bioadhesive agent within the coordinate administration, and/or combinatorial formulation methods and compositions of the invention is chitosan, as well as its analogs and derivatives. Chitosan is a non-toxic, biocompatible and biodegradable polymer that is widely used for pharmaceutical and medical applications because of its favorable properties of low toxicity and good biocompatibility. It is a natural polyaminosaccharide prepared from chitin by N-deacetylation with alkali. As used within the methods and compositions of the invention, chitosan increases the retention of glucose-regulating peptide proteins, analogs and mimetics, and other biologically active agents disclosed herein at a mucosal site of application. This mode of administration can also improve patient compliance and acceptance. As further provided herein, the methods and compositions of the invention will optionally include a novel chitosan derivative or chemically modified form of chitosan. One such novel derivative for use within the invention is denoted as a β-[1→4]-2-guanidino-2-deoxy-D-glucose polymer (poly-GuD). Chitosan is the N-deacetylated product of chitin, a naturally occurring polymer that has been used extensively to prepare microspheres for oral and intra-nasal formulations. The chitosan polymer has also been proposed as a soluble carrier for parenteral drug delivery. Within one aspect of the invention, o-methylisourea is used to convert a chitosan amine to its guanidinium moiety. The guanidinium compound is prepared, for example, by the reaction between equi-normal solutions of chitosan and o-methylisourea at pH above 8.0.

Additional compounds classified as bioadhesive agents for use within the present invention act by mediating specific interactions, typically classified as "receptor-ligand interactions" between complementary structures of the bioadhesive compound and a component of the mucosal epithelial surface. Many natural examples illustrate this form of specific binding bioadhesion, as exemplified by lectin-sugar interactions. Lectins are (glyco) proteins of non-immune origin which bind to polysaccharides or glycoconjugates.

Several plant lectins have been investigated as possible pharmaceutical absorption-promoting agents. One plant lectin, Phaseolus vulgaris hemagglutinin (PHA), exhibits high oral bioavailability of more than 10% after feeding to rats. Tomato (*Lycopersicon esculeutum*) lectin (TL) appears safe for various modes of administration.

In summary, the foregoing bioadhesive agents are useful in the combinatorial formulations and coordinate therapeutic uses of the instant invention, which optionally incorporate an effective amount and form of a bioadhesive agent to prolong persistence or otherwise increase mucosal absorption of one or more glucose-regulating peptide proteins, analogs and mimetics, and other biologically active agents. The bioadhesive agents may be coordinately administered as adjunct compounds or as additives within the combinatorial formulations of the invention. In certain embodiments, the bioadhesive agent acts as a 'pharmaceutical glue', whereas in other embodiments adjunct delivery or combinatorial formulation of the bioadhesive agent serves to intensify contact of the biologically active agent with the nasal mucosa, in some cases by promoting specific receptor-ligand interactions with epithelial cell "receptors", and in others by increasing epithelial permeability to significantly increase the drug concentration gradient measured at a target site of delivery (e.g., liver, blood plasma, or CNS tissue or fluid). Yet additional bioadhesive agents for use within the invention act as enzyme (e.g., protease) inhibitors to enhance the stability of mucosally administered biotherapeutic agents delivered coordinately or in a combinatorial formulation with the bioadhesive agent.

**Liposomes and Micellar Delivery Vehicles**

The coordinate therapeutic uses and combinatorial formulations of the instant invention optionally incorporate effective lipid or fatty acid based carriers, processing agents, or delivery vehicles, to provide improved formulations for mucosal delivery of glucose-regulating peptide proteins, analogs and mimetics, and other biologically active agents. For example, a variety of formulations and therapeutic uses are provided for mucosal delivery which comprise one or more of these active agents, such as a peptide or protein, admixed or encapsulated by, or coordinately administered with, a liposome, mixed micellar carrier, or emulsion, to enhance chemical and physical stability and increase the half life of the biologically active agents (e.g., by reducing susceptibility to proteolysis, chemical modification and/or denaturation) upon mucosal delivery.

Within certain aspects of the invention, specialized delivery systems for biologically active agents comprise small lipid vesicles known as liposomes. These are typically made from natural, biodegradable, non-toxic, and non-immunogenic lipid molecules, and can efficiently entrap or bind drug molecules, including peptides and proteins, into, or onto, their membranes. The attractiveness of liposomes as a peptide and protein delivery system within the invention is increased by the fact that the encapsulated proteins can remain in their preferred aqueous environment within the vesicles, while the liposomal membrane protects them against proteolysis and other destabilizing factors. Even though not all liposome preparation methods known are feasible in the encapsulation of peptides and proteins due to their unique physical and chemical properties, several methods allow the encapsulation of these macromolecules without substantial deactivation.

A variety of methods are available for preparing liposomes for use within the invention, U.S. Patent Nos. 4,235,871, 4,501,728, and 4,837,028. For use with liposome delivery, the biologically active agent is typically entrapped within the liposome, or lipid vesicle, or is bound to the outside of the vesicle.

Like liposomes, unsaturated long chain fatty acids, which also have enhancing activity for mucosal absorption, can form closed vesicles with bilayer-like structures (so called "ufasomes"). These can be formed, for example, using oleic acid to entrap biologically active peptides and proteins for mucosal, e.g., intranasal, delivery within the invention.

Other delivery systems for use within the invention combine the use of polymers and liposomes to ally the advantageous properties of both vehicles such as encapsulation inside the natural polymer fibrin. In addition, release of biotherapeutic compounds from this delivery system is controllable through the use of covalent crosslinking and the addition of antifibrinolytic agents to the fibrin polymer.

More simplified delivery systems for use within the invention may include the use of cationic lipids as delivery vehicles or carriers, which can be effectively employed to provide an electrostatic interaction between the lipid carrier and such charged biologically active agents as proteins and polyanionic nucleic acids. This allows efficient packaging of the drugs into a form suitable for mucosal administration and/or subsequent delivery to systemic compartments.

Additional delivery vehicles for use within the invention include long and medium chain fatty acids, as well as surfactant mixed micelles with fatty acids. Most naturally occurring lipids in the form of esters have important implications with regard to their own transport across mucosal surfaces. Free fatty acids and their monoglycerides which have polar groups attached have been demonstrated in the form of mixed micelles to act on the intestinal barrier as penetration enhancers. This discovery of barrier modifying function of free fatty acids (carboxylic acids with a chain length varying from 12 to 20 carbon atoms) and their polar derivatives has stimulated extensive research on the application of these agents as mucosal absorption enhancers.

For use within the invention, long chain fatty acids, especially fusogenic lipids (unsaturated fatty acids and monoglycerides such as oleic acid, linoleic acid, linoleic acid, monoolein, etc.) provide useful carriers to enhance mucosal delivery of glucose-regulating peptide, analogs and mimetics, and other biologically active agents disclosed herein. Medium chain fatty acids (C6 to C12) and monoglycerides have also been shown to have enhancing activity in intestinal drug absorption and can be adapted for use within the mocosal delivery formulations and therapeutic uses of the invention. In addition, sodium salts of medium and long chain fatty acids are effective delivery vehicles and absorption-enhancing agents for mucosal delivery of biologically active agents within the invention. Thus, fatty acids can be employed in soluble forms of sodium salts or by the addition of non-toxic surfactants, e.g., polyoxyethylated hydrogenated castor oil, sodium taurocholate, etc. Other fatty acid and mixed micellar preparations that are useful within the invention include, but are not limited to, Na caprylate (C8), Na caprate (C10), Na laurate (C12) or Na oleate (C18), optionally combined with bile salts, such as glycocholate and taurocholate.

**Pegylation**

Additional therapeutic uses and compositions provided within the invention may involve chemical modification of biologically active peptides and proteins by covalent attachment of polymeric materials, for example dextrans, polyvinyl pyrrolidones, glycopeptides, polyethylene glycol and polyamino acids. The resulting conjugated peptides and proteins retain their biological activities and solubility for mucosal administration. In alternate embodiments, glucose-regulating peptide proteins, analogs and mimetics, and other biologically active peptides and proteins, are conjugated to polyalkylene oxide polymers, particularly polyethylene glycols (PEG). U.S. Patent No. 4,179,337.

Amine-reactive PEG polymers for use within the invention include SC-PEG with molecular masses of 2000, 5000, 10000,12000, and 20 000; U-PEG-10000; NHS-PEG-3400-biotin; T-PEG-5000; T-PEG-12000; and TPC-PEG-5000. PEGylation of biologically active peptides and proteins may be achieved by modification of carboxyl sites (e.g., aspartic acid or glutamic acid groups in addition to the carboxyl terminus). The utility of PEG-hydrazide in selective modification of carbodiimide-activated protein carboxyl groups under acidic conditions has been described. Alternatively, bifunctional PEG modification of biologically active peptides and proteins can be employed. In some procedures, charged amino acid residues, including lysine, aspartic acid, and glutamic acid, have a marked tendency to be solvent accessible on protein surfaces.

**Other Stabilizing Modifications of Active Agents**

In addition to PEGylation, biologically active agents such as peptides and proteins for use within the invention can be modified to enhance circulating half-life by shielding the active agent via conjugation to other known protecting or stabilizing compounds, for example by the creation of fusion proteins with an active peptide, protein, analog or mimetic linked to one or more carrier proteins, such as one or more immunoglobulin chains.

**Formulation and Administration**

Mucosal delivery formulations of the present invention comprise glucose-regulating peptide, typically combined together with one or more pharmaceutically acceptable carriers and, optionally, other therapeutic ingredients. The carrier (s) must be "pharmaceutically acceptable" in the sense of being compatible with the other ingredients of the formulation and not eliciting an unacceptable deleterious effect in the subject. Such carriers are described herein above or are otherwise well known to those skilled in the art of pharmacology. Desirably, the formulation should not include substances such as enzymes or oxidizing agents with which the biologically active agent to be administered is known to be incompatible. The formulations may be prepared by any of the methods well known in the art of pharmacy.

Within the compositions and therapeutic uses of the invention, the glucose-regulating peptide proteins, and other biologically active agents disclosed herein may be administered to subjects by a intranasal. In other alternative embodiments, the biologically active agent(s) can be administered *ex vivo* by direct exposure to cells, tissues or organs originating from a mammalian subject, for example as a component of an *ex vivo* tissue or organ treatment formulation that contains the biologically active agent in a suitable, liquid or solid carrier.

Compositions according to the present invention are often administered in an aqueous solution as a nasal spray and may be dispensed in spray form by a variety of methods known to those skilled in the art. Preferred systems for dispensing liquids as a nasal spray are disclosed in U.S. Patent No. 4,511,069. The formulations may be presented in multi-dose containers, for example in the sealed dispensing system disclosed in U.S. Patent No. 4,511,069. Additional aerosol delivery forms may include, e.g., compressed air-, jet-, ultrasonic-, and piezoelectric nebulizers, which deliver the biologically active agent dissolved or suspended in a pharmaceutical solvent, e.g., water, ethanol, or a mixture thereof.

Nasal spray solutions of the present invention typically comprise the drug or drug to be delivered, optionally formulated with a surface-active agent, such as a nonionic surfactant (e.g., polysorbate-80), and one or more buffers. In some embodiments of the present invention, the nasal spray solution further comprises a propellant. The pH of the nasal spray solution is optionally between about pH 2.0 and 8, preferably 4.5 $\pm$0.5. Suitable buffers for use within these compositions are as described above or as otherwise known in the art. Other components may be added to enhance or maintain chemical stability, including preservatives, surfactants, dispersants, or gases. Suitable preservatives include, but are not limited to, phenol, methyl paraben, paraben, m-cresol, thiomersal, chlorobutanol, benzylalkonimum chloride, sodium benzoate, and the like. Suitable surfactants include, but are not limited to, oleic acid, sorbitan trioleate, polysorbates, lecithin, phosphotidyl cholines, and various long chain diglycerides and phospholipids. Suitable dispersants include, but are not limited to, ethylenediaminetetraacetic acid, and the like. Suitable gases include, but are not limited to, nitrogen, helium, chlorofluorocarbons (CFCs), hydrofluorocarbons (HFCs), carbon dioxide, air, and the like.

Within alternate embodiments, mucosal formulations are to be administered as dry powder formulations comprising the biologically active agent in a dry, usually lyophilized, form of an appropriate particle size, or within an appropriate particle size range, for intranasal delivery. Minimum particle size appropriate for deposition within the nasal or pulmonary passages is often about 0.5 µ mass median equivalent aerodynamic diameter (MMEAD), commonly about 1 µ MMEAD, and more typically about 2 µ MMEAD. Maximum particle size appropriate for deposition within the nasal passages is often about 10 µ MMEAD, commonly about 8 µ MMEAD, and more typically about 4 µ MMEAD. Intranasally respirable powders within these size ranges can be produced by a variety of conventional techniques, such as jet milling, spray drying, solvent precipitation, supercritical fluid condensation, and the like. These dry powders of appropriate MMEAD can be administered to a patient via a conventional dry powder inhaler (DPI), which rely on the patient's breath, upon pulmonary or nasal inhalation, to disperse the power into an aerosolized amount. Alternatively, the dry powder may be administered via air-assisted devices that use an external power source to disperse the powder into an aerosolized amount, e.g., a piston pump.

Dry powder devices typically require a powder mass in the range from about 1 mg to 20 mg to produce a single aerosolized dose ("puff"). If the required or desired dose of the biologically active agent is lower than this amount, the powdered active agent will typically be combined with a pharmaceutical dry bulking powder to provide the required total powder mass. Preferred dry bulking powders include sucrose, lactose, dextrose, mannitol, glycine, trehalose, human serum albumin (HSA), and starch. Other suitable dry bulking powders include cellobiose, dextrans, maltotriose, pectin, sodium citrate, sodium ascorbate, and the like.

To formulate compositions for mucosal delivery within the present invention, the biologically active agent can be combined with various pharmaceutically acceptable additives, as well as a base or carrier for dispersion of the active agent(s). Desired additives include, but are not limited to, pH control agents, such as arginine, sodium hydroxide, glycine, hydrochloric acid, citric acid, acetic acid, *etc.* In addition, local anesthetics (e.g., benzyl alcohol), isotonizing agents (e.g., sodium chloride, mannitol, sorbitol), adsorption inhibitors (e.g., Tween 80), solubility enhancing agents (e.g., cyclodextrins and derivatives thereof), stabilizers (e.g., serum albumin), and reducing agents (e.g., glutathione) can be included. When the composition for mucosal delivery is a liquid, the tonicity of the formulation, as measured with reference to the tonicity of 0.9% (w/v) physiological saline solution taken as unity, is typically adjusted to a value at which no substantial, irreversible tissue damage will be induced in the nasal mucosa at the site of administration. Generally, the tonicity of the solution is adjusted to a value of about 1/3 to 3, more typically 1/2 to 2, and most often 3/4 to 1.7.

The biologically active agent may be dispersed in a base or vehicle, which may comprise a hydrophilic compound having a capacity to disperse the active agent and any desired additives. The base may be selected from a wide range of suitable carriers, including but not limited to, copolymers of polycarboxylic acids or salts thereof, carboxylic anhydrides (e.g. maleic anhydride) with other monomers (e.g. methyl (meth)acrylate, acrylic acid, etc.), hydrophilic vinyl polymers such as polyvinyl acetate, polyvinyl alcohol, polyvinylpyrrolidone, cellulose derivatives such as hydroxymethylcellulose, hydroxypropylcellulose, etc., and natural polymers such as chitosan, collagen, sodium alginate, gelatin, hyaluronic acid, and nontoxic metal salts thereof. Often, a biodegradable polymer is selected as a base or carrier, for example, polylactic acid, poly(lactic acid-glycolic acid) copolymer, polyhydroxybutyric acid, poly(hydroxybutyric acid-glycolic acid) copolymer and mixtures thereof. Alternatively or additionally, synthetic fatty acid esters such as polyglycerin fatty acid esters, sucrose fatty acid esters, etc. can be employed as carriers. Hydrophilic polymers and other carriers can be used alone or in combination, and enhanced structural integrity can be imparted to the carrier by partial crystallization, ionic bonding, crosslinking and the like. The carrier can be provided in a variety of forms, including, fluid or viscous solutions, gels, pastes, powders, microspheres and films for direct application to the nasal mucosa. The use of a selected carrier in this context may result in promotion of absorption of the biologically active agent.

The biologically active agent can be combined with the base or carrier according to a variety of methods, and release of the active agent may be by diffusion, disintegration of the carrier, or associated formulation of water channels. In some circumstances, the active agent is dispersed in microcapsules (microspheres) or nanocapsules (nanospheres) prepared from a suitable polymer, e.g., isobutyl 2-cyanoacrylate and dispersed in a biocompatible dispersing medium applied to the nasal mucosa, which yields sustained delivery and biological activity over a protracted time.

To further enhance mucosal delivery of pharmaceutical agents within the invention, formulations comprising the active agent may also contain a hydrophilic low molecular weight compound as a base or excipient. Such hydrophilic low molecular weight compounds provide a passage medium through which a water-soluble active agent, such as a physiologically active peptide or protein, may diffuse through the base to the body surface where the active agent is absorbed. The hydrophilic low molecular weight compound optionally absorbs moisture from the mucosa or the administration atmosphere and dissolves the water-soluble active peptide. The molecular weight of the hydrophilic low molecular weight compound is generally not more than 10000 and preferably not more than 3000. Exemplary hydrophilic low molecular weight compound include polyol compounds, such as oligo-, di- and monosaccharides such as sucrose, mannitol, sorbitol, lactose, L-arabinose, D-erythrose, D-ribose, D-xylose, D-mannose, trehalose, D-galactose, lactulose, cellobiose, gentibiose, glycerin and polyethylene glycol. Other examples of hydrophilic low molecular weight compounds useful as carriers within the invention include N-methylpyrrolidone, and alcohols (e.g. oligovinyl alcohol, ethanol, ethylene glycol, propylene glycol, etc.) These hydrophilic low molecular weight compounds can be used alone or in combination with one another or with other active or inactive components of the intranasal formulation.

The compositions of the invention may alternatively contain as pharmaceutically acceptable carriers substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. For solid compositions, conventional nontoxic pharmaceutically acceptable carriers can be used which include, for example, pharmaceutical grades of mannitol, lactose, starch, magnesium stearate, sodium saccharin, talcum, cellulose, glucose, sucrose, magnesium carbonate, and the like.

Therapeutic compositions for administering the biologically active agent can also be formulated as a solution, microemulsion, or other ordered structure suitable for high concentration of active ingredients. The carrier can be a solvent or dispersion medium containing, for example, water, ethanol, polyol (for example, glycerol, propylene glycol, and liquid polyethylene glycol, and the like), and suitable mixtures thereof. Proper fluidity for solutions can be maintained, for example, by the use of a coating such as lecithin, by the maintenance of a desired particle size in the case of dispersible formulations, and by the use of surfactants. In many cases, it will be desirable to include isotonic agents, for example, sugars, polyalcohols such as mannitol, sorbitol, or sodium chloride in the composition. Prolonged absorption of the biologically active agent can be brought about by including in the composition an agent which delays absorption, for example, monostearate salts and gelatin.

In certain embodiments of the invention, the biologically active agent is to be administered in a time-release formulation, for example in a composition which includes a slow release polymer. The active agent can be prepared with carriers that will protect against rapid release, for example a controlled release vehicle such as a polymer, microencapsulated delivery system or bioadhesive gel. Prolonged delivery of the active agent, in various compositions of the invention can be brought about by including in the composition agents that delay absorption, for example, aluminum monosterate hydrogels and gelatin. When controlled release formulations of the biologically active agent is desired, controlled release binders suitable for use in accordance with the invention include any biocompatible controlled-release material which is inert to the active agent and which is capable of incorporating the biologically active agent. Numerous such materials are known in the art. Useful controlled-release binders are materials that are metabolized slowly under physiological conditions following their intranasal delivery (e.g., at the nasal mucosal surface, or in the presence of bodily fluids following transmucosal delivery). Appropriate binders include but are not limited to biocompatible polymers and copolymers previously used in the art in sustained release formulations. Such biocompatible compounds are non-toxic and inert to surrounding tissues, and do not trigger significant adverse side effects such as nasal irritation, immune response, inflammation, or the like. They are metabolized into metabolic products that are also biocompatible and easily eliminated from the body.

Exemplary polymeric materials for use in this context include, but are not limited to, polymeric matrices derived from copolymeric and homopolymeric polyesters having hydrolysable ester linkages. A number of these are known in the art to be biodegradable and to lead to degradation products having no or low toxicity. Exemplary polymers include polyglycolic acids (PGA) and polylactic acids (PLA), poly(DL-lactic acid-co-glycolic acid)(DL PLGA), poly(D-lactic acid-coglycolic acid)(D PLGA) and poly(L-lactic acid-coglycolic acid)(L PLGA). Other useful biodegradable or bioerodable polymers include but are not limited to such polymers as poly(epsilon-caprolactone), poly(epsilon-aprolactone-CO-lactic acid), poly(ε-aprolactone-CO-glycolic acid), poly(beta-hydroxy butyric acid), poly(alkyl-2-cyanoacrilate), hydrogels such as poly(hydroxyethyl methacrylate), polyamides, poly(amino acids) (i.e., L-leucine, glutamic acid, L-aspartic acid and the like), poly (ester urea), poly (2-hydroxyethyl DL-aspartamide), polyacetal polymers, polyorthoesters, polycarbonate, polymaleamides, polysaccharides and copolymers thereof. Many methods for preparing such formulations are generally known to those skilled in the art. Other useful formulations include controlled-release compositions e.g., microcapsules, U.S. Patent Nos. 4,652,441 and 4,917,893, lactic acid-glycolic acid copolymers useful in making microcapsules and other formulations, U.S. Patent Nos. 4,677,191 and 4,728,721, and sustained-release compositions for water-soluble peptides, U.S. Patent No. 4,675,189.

Sterile solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders, methods of preparation include vacuum drying and freeze-drying which yields a powder of the active ingredient plus any additional desired ingredient from a previously sterile-filtered solution thereof. The prevention of the action of microorganisms can be accomplished by various antibacterial and antifungal agents, for example, parabens, chlorobutanol, phenol, sorbic acid, thimerosal, and the like.

Mucosal administration according to the invention allows effective self-administration of treatment by patients, provided that sufficient safeguards are in place to control and monitor dosing and side effects. Mucosal administration also overcomes certain drawbacks of other administration forms, such as injections, that are painful and expose the patient to possible infections and may present drug bioavailability problems. For nasal and pulmonary delivery, systems for controlled aerosol dispensing of therapeutic liquids as a spray are well known. In one embodiment, metered doses of active agent are to be delivered by means of a specially constructed mechanical pump valve, U.S. Patent No. 4,511,069.

**Dosage**

For prophylactic and treatment purposes, the biologically active agent(s) disclosed herein may be administered to the subject in a single bolus delivery, via continuous delivery (mucosal) over an extended time period, or in a repeated administration protocol (e.g., by an hourly, daily or weekly, repeated administration protocol). In this context, a therapeutically effective dosage of the glucose-regulating peptide may include repeated doses within a prolonged prophylaxis or treatment regimen that will yield clinically significant results to alleviate one or more symptoms or detectable conditions associated with a targeted disease or condition as set forth above. Determination of effective dosages in this context is typically based on animal model studies followed up by human clinical trials and is guided by determining effective dosages and administration protocols that significantly reduce the occurrence or severity of targeted disease symptoms or conditions in the subject. Suitable models in this regard include, for example, murine, rat, porcine, feline, non-human primate, and other accepted animal model subjects known in the art. Alternatively, effective dosages can be determined using in vitro models (e.g., immunologic and histopathologic assays). Using such models, only ordinary calculations and adjustments are typically required to determine an appropriate concentration and dose to administer a therapeutically effective amount of the biologically active agent(s) (e.g., amounts that are intranasally effective, transdermally effective, intravenously effective, or intramuscularly effective to elicit a desired response).

The invention provides compositions and therapeutic uses for intranasal delivery of glucose-regulating peptide, wherein the glucose-regulating peptide compound(s) is/are repeatedly administered through an intranasal effective dosage regimen that involves multiple administrations of the glucose-regulating peptide to the subject during a daily or weekly schedule to maintain a therapeutically effective elevated and lowered pulsatile level of glucose-regulating peptide during an extended dosing period. The compositions and method provide glucose-regulating peptide compound(s) that are self-administered by the subject in a nasal formulation between one and six times daily to maintain a therapeutically effective elevated and lowered pulsatile level of glucose-regulating peptide during an 8 hour to 24 hour extended dosing period.

The application also includes kits, packages and multicontainer units containing the above described pharmaceutical compositions, active ingredients, and/or means for administering the same for use in the prevention and treatment of diseases and other conditions in mammalian subjects. Briefly, these kits include a container or formulation that contains one or more glucose-regulating peptide proteins, analogs or mimetics, and/or other biologically active agents in combination with mucosal delivery enhancing agents disclosed herein formulated in a pharmaceutical preparation for mucosal delivery.

The intranasal formulations of the present invention can be administered using any spray bottle or syringe, or by instillation. An example of a nasal spray bottle is the, "Nasal Spray Pump w/ Safety Clip, Pfeiffer SAP # 60548, which delivers a dose of 0.1mL per squirt and has a diptube length of 36.05 mm. It can be purchased from Pfeiffer of America of Princeton, NJ.

**Aerosol Nasal Administration of a Glucose-regulating Peptide**

We have discovered that the GRPs can be administered intranasally using a nasal spray or aerosol. This is surprising because many proteins and peptides have been shown to be sheared or denatured due to the mechanical forces generated by the actuator in producing the spray or aerosol. In this area the following definitions are useful.

1. Aerosol - A product that is packaged under pressure and contains therapeutically active ingredients that are released upon activation of an appropriate valve system.

2. Metered aerosol - A pressurized dosage form comprised of metered dose valves, which allow for the delivery of a uniform quantity of spray upon each activation.

3. Powder aerosol - A product that is packaged under pressure and contains therapeutically active ingredients in the form of a powder, which are released upon activation of an appropriate valve system.

4. Spray aerosol - An aerosol product that utilizes a compressed gas as the propellant to provide the force necessary to expel the product as a wet spray; it generally applicable to solutions of medicinal agents in aqueous solvents.

5. Spray - A liquid minutely divided as by a jet of air or steam. Nasal spray drug products contain therapeutically active ingredients dissolved or suspended in solutions or mixtures of excipients in nonpressurized dispensers.

6. Metered spray - A non-pressurized dosage form consisting of valves that allow the dispensing of a specified quantity of spray upon each activation.

7. Suspension spray - A liquid preparation containing solid particles dispersed in a liquid vehicle and in the form of course droplets or as finely divided solids.

The fluid dynamic characterization of the aerosol spray emitted by metered nasal spray pumps as a drug delivery device ("DDD"). Spray characterization is an integral part of the regulatory submissions necessary for Food and Drug Administration ("FDA") approval of research and development, quality assurance and stability testing procedures for new and existing nasal spray pumps.

Thorough characterization of the spray's geometry has been found to be the best indicator of the overall performance of nasal spray pumps. In particular, measurements of the spray's divergence angle (plume geometry) as it exits the device; the spray's cross-sectional ellipticity, uniformity and particle/droplet distribution (spray pattern); and the time evolution of the developing spray have been found to be the most representative performance quantities in the characterization of a nasal spray pump. During quality assurance and stability testing, plume geometry and spray pattern measurements are key identifiers for verifying consistency and conformity with the approved data criteria for the nasal spray pumps.

Definitions

Plume Height - the measurement from the actuator tip to the point at which the plume angle becomes non-linear because of the breakdown of linear flow. Based on a visual examination of digital images, and to establish a measurement point for width that is consistent with the farthest measurement point of spray pattern, a height of 30 mm is defined for this study

Major Axis - the largest chord that can be drawn within the fitted spray pattern that crosses the COMw in base units (mm)

Minor Axis - the smallest chord that can be drawn within the fitted spray pattern that crosses the COMw in base units (mm)

Ellipticity Ratio - the ratio of the major axis to the minor axis, preferably between 1.0 and 1.5, and most preferably between 1.0 and 1.3.

$D_{10}$ - the diameter of droplet for which 10% of the total liquid volume of sample consists of droplets of a smaller diameter ($\mu$m)

$D_{50}$ - the diameter of droplet for which 50% of the total liquid volume of sample consists of droplets of a smaller diameter ($\mu$m), also known as the mass median diameter

$D_{90}$ - the diameter of droplet for which 90% of the total liquid volume of sample consists of droplets of a smaller diameter ($\mu$m)

Span - measurement of the width of the distribution, The smaller the value, the narrower the distribution. Span is calculated as $\dfrac{(D_{90} - D_{10})}{D_{50}}$ .

% RSD - percent relative standard deviation, the standard deviation divided by the mean of the series and multiplied by 100, also known as % CV.

Volume- the volume of liquid or powder discharged from the delivery device with each actuation, preferably between 0.01 mL and about 2.5 mL and most preferably between 0.02 mL and 0.25 mL.

The following examples are provided by way of illustration, not limitation.

## EXAMPLE 1

(Prophetic)

**Composition of GRP-containing Formulations**

An exemplary, prophetic formulation for enhanced nasal mucosal delivery of glucose-regulating peptides following the teachings of the instant specification is prepared and evaluated as follows:

**Table 1: GRP formulation composition***

| Formulations | Mucosal Delivery Enhancing Agent |
|---|---|
| A | Phosphate-buffered saline (0.8%) pH 7.4 (Control 1) |
| B | Phosphate-buffered saline (0.8%) pH 5.0 (Control 2) |
| C | L-Arginine (10% w/v) |
| D | Poly-L-Arginine (0.5% w/v) |
| E | Gamma-Cyclodextrin (1% w/v) |
| F | $\alpha$-Cyclodextrin (5% w/v) |
| G | Methyl-$\beta$-Cyclodextrin (3% w/v) |

(continued)

| Formulations | Mucosal Delivery Enhancing Agent |
|---|---|
| H | n-Capric Acid Sodium (0.075% w/v) |
| I | Chitosan (0.5% w/v) |
| J | L-α-phosphatidilcholine didecanyl (3.5% w/v) |
| K | S-Nitroso-N-Acetyl-Penicillamine (0.5% w/v) |
| L | Palmotoyl-DL-Carnitine (0.02% w/v) |
| M | Pluronic-127 (0.3% w/v) |
| N | Sodium Nitroprusside (0.3% w/v) |
| O | Sodium Glycocholate (1% w/v) |
| P | F1: Gelatin, DDPC, MBCD, EDTA |
| F 1 | L-α-phosphatidilcholine didecanyl (0.5% w/v) Methyl β Cyclodextrin (3% w/v) EDTA (0.1 % w/v, Inf. Conc. 0.5 M) Gelatin (0.5 % w/v) |
| • An amount of a GRP is added to the formulation to produce a concentration sufficient to produce a therapeutic effect. | |

## EXAMPLE 2

(Prophetic)

### Preparation of a Amylin Formulation Free of a Stabilizer that is a Protein

A amylin formulation suitable for intranasal administration of amylin, which is substantially free of a stabilizer that is a protein is prepared having the formulation listed below.

1. About ¾ of the water is added to a beaker and stirred with a stir bar on a stir plate and the sodium citrate is added until it is completely dissolved.
2. The EDTA is then added and stirred until it is completely dissolved.
3. The citric acid is then added and stirred until it is completely dissolved.
4. The methyl-β-cyclodextrin is added and stirred until it is completely dissolved.
5. The DDPC is then added and stirred until it is completely dissolved.
6. The lactose is then added and stirred until it is completely dissolved.
7. The sorbitol is then added and stirred until it is completely dissolved.
8. The chlorobutanol is then added and stirred until it is completely dissolved.
9. The amylin is added and stirred gently until it dissolved.
10. 11 Check the pH to make sure it is 5.0 $\pm$ 0.25. Add dilute HCl or dilute NaOH to adjust the pH.
11. Add water to final volume.

### Table 2

| Reagent | mg/mL | % |
|---|---|---|
| Cholorbutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |

(continued)

| Reagent | mg/mL | % |
|---|---|---|
| Amylin | 1 | 0.1 |
| Purified Water | | |
| Formulation pH 5 +/- 0.25 Osmolarity ~250 | | |

## EXAMPLE 3

(Prophetic)

**Preparation of a Amylin Formulation Free of a Stabilizer that is a Protein and the Concentration of Amylin is 15 mg/mL**

A second formulation is prepared as above, except the concentration of amylin is 15 mg/mL as shown below in Table 3.

**Table 3**

| Reagent | mg/ml | % |
|---|---|---|
| Cholorbutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| amylin | 15 | 0.1 |
| Purified Water | | |
| Formulation pH 4-6 | | |

## EXAMPLE 4

(Prophetic)

**Preparation of a Pramlintide Formulation Free of a Stabilizer that is a Protein and is Endotoxin-free**

The following enodotoxin-free pramlintide acetate Nasal formulation can be made.

**Table 4**

| Reagent | mg/ml | % |
|---|---|---|
| Cholorbutanol, anhydrous | 2.5 | 0.25 |
| Methyl-β-Cyclodextrin | 45 | 4.5 |
| L-α-Phosphatidylcholine Didecanoyl | 1 | 0.1 |
| Edetate Disodium (EDTA) | 1 | 0.1 |
| Sodium Citrate, Dihydrate | 1.6 | 0.16 |

(continued)

| Reagent | mg/ml | % |
|---|---|---|
| Citric Acid, Anhydrous | 0.9 | 0.09 |
| Pramlintide, endotoxin-free | 2 | 0.2 |
| Purified Water | | |
| Formulation pH 5 +/- 0.25 | | |

## EXAMPLE 5

(Prophetic)

### Buccal formulation of a GRP

Bilayer tablets are prepared in the following manner. An adhesive layer is prepared by weighing 70 parts by weight polyethylene oxide (Polyox 301N; Union Carbide), 20 parts by weight polyacrylic acid (Carbopol 934P; B.F. Goodrich), and 10 parts by weight of a compressible xylitol/carboxymethyl cellulose filler (Xylitab 200; Xyrofin). These ingredients are mixed by rolling in a jar for 3 minutes. The mixture is then transferred to an evaporating dish and quickly wet granulated with absolute ethanol to a semi-dough-like consistency. This mass is immediately and rapidly forced through a 14 mesh (1.4 mm opening) stainless steel screen, to which the wet granules adhered. The screen is covered with perforated aluminum foil, and the wet granules are dried overnight at 30° C. The dried granules are removed from the screen and then passed through a 20 mesh (0.85 mm opening) screen to further reduce the size of the granules. Particles that do not pass through the 20 mesh screen are ground briefly with a mortar and pestle to minimize the amount of fines and then passed through the 20 mesh screen. The resulting granules are then placed in a mixing jar, and 0.25 parts by weight stearic acid and 0.06 parts by weight mint flavor (Universal Flavors) are added and blended to the granules. The final percentages by weight of the ingredients are thus 69.78% polyethylene oxide, 9.97% compressible xylitol/carboxymethyl cellulose filler, 19.94% polyacrylic acid, 0.25% stearic acid, and 0.06% mint flavor. A 50 mg amount of this mixture is placed on a 0.375 inch diameter die and precompressed on a Carver Press Model C with 0.25 metric ton pressure for a 3 second dwell time to form the adhesive layer.

The active layer is prepared by weighing 49.39 parts by weight of mannitol, 34.33 parts by weight of hydroxypropyl cellulose (Klucel L F; Aqualon, Wilmington, Del.) and 15.00 parts by weight of sodium taurocholate (Aldrich, Milwaukee, Wis.), and mixing by rolling in a jar for 3 minutes. The mixture is then transferred to an evaporating dish and quickly wet granulated with absolute ethanol to a semi-dough-like consistency. This mass is immediately and rapidly forced through a 14 mesh stainless steel screen, to which the wet granules adher. The screen is covered with perforated aluminum foil, and the granules dried at 30° C. The dried granulation is then passed sequentially through 20, 40 (0.425 mm opening), and 60 (0.25 mm opening) mesh screens to reduce particle size further. Particles that do not pass through a screen are briefly ground with a mortar and pestle to minimize fines and then passed through the screen. The screened particles were weighed, and then 0.91 parts by weight of GRP and 0.06 parts by weight of FD&C yellow #6HT aluminum lake dye are sequentially blended with the dry granulation by geometric dilution. The dyed granulation is then placed in a mixing jar and blended with 0.25 parts by weight magnesium stearate (lubricant) and 0.06 parts by weight mint flavor by rolling for 3 minutes. A 50 mg sample of this material is placed on top of the partially compressed adhesive layer and both layers are then compressed at 1.0 ton pressure for a 3 second dwell time to yield a bilayer tablet suitable for buccal delivery.

This procedure results in a gingival tablet wherein the active layer contains 0.91% by weight of GRP , 15% by weight of NaTC, and 84.09% by weight of filler, lubricant, colorant, formulation aids, or flavoring agents.

## EXAMPLE 6 (not part of the invention)

(Prophetic)

### Pulmonary Delivery of GRP

The carrier compounds, prepared as described below may be used directly as a delivery carrier by simply mixing one or more compound or salt, poly amino acid or peptide with an endotoxin-free glucose-regulating peptide for pulmonary delivery.

The administration mixtures are prepared by mixing an aqueous solution of the carrier with an aqueous solution of the active ingredient, just prior to administration. Alternatively, the carrier and the biologically or chemically active ingredient can be admixed during the manufacturing process. The solutions may optionally contain additives such as phosphate buffer salts, citric acid, acetic acid, gelatin, and gum acacia.

A number of known pulmonary delivery methods can use endotoxin-free glucose-regulating peptides, especially GRP , to improve the delivery of GRP to the lungs. The following non-limiting patent applications are incorporated herein by reference for pulmonary delivery: US Patent applications Nos. 20030223939, 20030215514, 20030215512, 20030209243, 20030203036, 20030198601, 20030183228, 200301885765, 20030150454, 20030124193, 20030094173.

**EXAMPLE 7** (not part of the invention)

(Prophetic)

**Preparation of Carriers for Pulmonary Delivery**

Preparation of 2-(4-(N-salicyloyl)aminophenyl) propionic acid (Carrier B)

A slurry of 58.6 g (0.355 mol) of 2-(4-aminophenyl)propionic acid and 500 ml of methylene chloride is treated with 90.11 ml (77.13 g. 0-710 mol) of trimethylsilyl chloride and is heated to reflux for 120 min. The reaction mixture is cooled to 0°C. and treated with 184.44 ml (107.77 g, 1.065 mol) of triethylamine. After stirring for 5 minutes, this mixture is treated with a solution of 70.45 g (0.355 mol) of O-acetylsalicyloyl chloride and 150 ml of methylene chloride. The reaction mixture is warmed to 25° C. and stirred for 64 hr. The volatiles are removed in vacuo. The residue is stirred in 2N aqueous sodium hydroxide for one hour and acidified with 2 M aqueous sulfuric acid. The solid is recrystallized twice from ethanol/water to give a tan solid. Isolation by filtration results in an expected yield of 53.05 g (52% yield) of 2-(4-(N-salicyloyl)aminophenyl)propionic acid. Properties. Solubility: 200 mg/m: 200 mg+350 .μL 2N NaOH+650 .μL $H_2O$-pH-7.67. Analysis: C, 67.36; H, 5.3; N, 4.91..

Preparation of Sodium 2-(4-(N-salicyloyl)aminophenyl)propionate (Sodium Salt of Carrier B)

A solution of 53.05 g (0.186 mol) of 2-(4-(N-salicyloyl)aminophenyl- )propionic acid and 300 ml of ethanol is treated with 7.59 g (0.190 mol) of NaOH dissolved in 22 ml of water. The reaction mixture is stirred for 30 min at 25°C and for 30 min at 0° C. The resulting pale yellow solid is isolated by filtration to give 52.61 g of sodium 2-(4-(N-salicyloyl) aminophenyl)propionate. Properties. Solubility: 200 mg/ml clear solution, pH=6.85. Analysis C, 60.45; H, 5.45; N, 3.92; Na, 6.43. Melting point 236-238° C.

Preparation of the Sodium Salt of Carrier C

A 2L round bottom flask equipped with a magnetic stirrer and a reflux condenser is charged with a suspension of 3-(4-aminophenyl)propio- nic acid (15.0 g. 0.084 moles. 1.0 equiv.) in dichloromethane (250 ml). Chlorotrimethylsilane (18.19 g, 0.856 moles, 2.0 equiv.) is added in one portion, and the mixture is heated to reflux for 1.5 h under argon. The reaction is allowed to cool to room temperature and is placed in an ice bath (internal temperature <10° C). The reflux condenser is replaced with an addition funnel containing triethylamine (25.41 g, 0.251 moles, 3.0 equiv.). The triethylamine is added dropwise over 15 min, and a yellow solid forms during the addition. The funnel is replaced by another addition funnel containing a solution of 2,3-dimethoxybenzoylchlo- ride (I 8.31 g, 0.091 moles, 1.09 equiv.) in dichloromethane (100 mL). The solution is added dropwise over 30 nm. The reaction is stirred in the ice bath for another 30 min and at ambient temperature for 3 h. The dicholoromethane is evaporated in vacuo to give a brown oil. The brown oil is cooled in an ice bath, and an ice-cold solution of saturated sodium bicarbonate (250 ml) is added. The ice bath is removed, and the reaction is stirred 1 h to afford a clear brown solution. The solution is acidified with concentrated HCl and stored at ca SC for 1 hour. The mixture is extracted with dichloromethane (3.times.100 mL), dried over sodium sulfate, the salts filtered off and the dichloromethane removed in vacuo. The resulting solid is recrystallized from 50% ethyl acetate/water (v/v) to afford Carrier C acid as off white needles (25.92 g. 90%). Analysis for $C_{19}H_{21}NO_5$: C, 66.46; H, 6.16; N, 4.08. mp=99-102°C.

12 grams of the Carrier C acid is dissolved in ethanol, 75 mL, with warming. To this solution a 8.5 M Sodium hydroxide (1.02 molar equivalents, 1.426 grams in 4.5 mL water) solution is added. The mixture is stirred for 15 minutes. Approximately three quarters of the ethanol is remove in vacuo and n-heptane, 100 mL, is added to the resulting oil causing a precipitate to form. The solids are dried in vacuo at 50° C. Analysis: $C_{19}H_{20}NO_5Na0.067H_2O$: C, 62.25; H, 5.54; N, 3.82; Na, 6.27.

Preparation of N-(4-methylsalicyloyl)-8-aminocaprylic acid (Carrier D)

(a) Preparation of Oligo(4-methylsalicylate)

Acetic anhydride (32 mL, 34.5 g, 0.338 mol, 1.03 eq), 4-methylsalicylic acid (50 g, 0.329 mmol, 1.00 eq), and xylenes (100 mL) are added to a 1 L, four-neck flask fitted with a magnetic stir bar, a thermometer, and a condenser. The flask is placed in a sand bath and heating of the cloudy white mixture begun. The reaction mixture clears to a yellow solution around 90° C. Most of the volatile organics (xylenes and acetic acid) are distilled into the Dean-Stark trap over three hours (135-146° C.). Distillation is continued for another hour (a total of 110 mL distilled), during which the pot temperature slowly rises to 204° C. and the distillate slows to a trickle. The residue is poured off while still hot into an aluminum tray. Upon cooling a brittle yellow glass forms. The solid is ground to a fine powder. The oligo(4-methylsalicylate) received is used without further purification.

(b) Preparation of N-(4-methylsalicyloyl)-8-aminocaprylic acid

A 7M solution of potassium carbonate (45 mL, 43.2 g, 0.313 mol, 0.95 eq), 8-aminocaprylic acid (41.8 g, 262 mol, 798 eq), and water (20 mL) are added to a 1 L round bottom flask equipped with a magnetic stir bar, condenser, and an addition fuel. The white cloudy mixture is treated with a solution of oligo(4-methylsalicylate) (44.7 g, 0.329 mmol 1.0 eq) and dioxane (250 mL), added over thirty minutes. The reaction mixture is heated to 90°C. for 3 hours (at which time the reaction is determined to have finished, by HPLC). The clear orange reaction mixture is cooled to 30°C. and acidified to pH=2 with 50% aqueous sulfuric acid (64 g). The resulting solid is isolated by filtration. The white solid is recrystallized from 1170 mL of 50% ethanol-water. The solid is recovered by filtration and is dried over 18 hours in a 50°C. vacuum oven. The N-(4-methylsalicyloyl)-8-ami- nocaprylic acid is isolated as a white solid (30.88 g, 52%); mp=113-114°. Analysis: $C_6H_{23}NO_4$: C, 65.51; H, 7.90; N, 4.77.

An aqueous solution of a GRP is then prepared and mixed with one or more of the carrier to produce a GRP composition, which then can be sprayed into the lungs. A suitable concentration of GRP for the resultant composition should be about 400 $\mu$g/mL. See U.S. Patent Application No. 20030072740.

### EXAMPLE 8

(Prophetic)

**Preparation of an GLP-1 Formulation Free of a Stabilizer that is a Protein**

A GLP-1 formulation suitable for intranasal administration of GLP-1, which is substantially free of a stabilizer that is a polypeptide or a protein is prepared having the formulation listed below.

1. About ¾ of the water is added to a beaker and stirred with a stir bar on a stir plate and the sodium citrate is added until it is completely dissolved.
2. The EDTA is then added and stirred until it is completely dissolved.
3. The citric acid is then added and stirred until it is completely dissolved.
4. The methyl-β-cyclodextrin is added and stirred until it is completely dissolved.
5. The DDPC is then added and stirred until it is completely dissolved.
6. The lactose is then added and stirred until it is completely dissolved.
7. The sorbitol is then added and stirred until it is completely dissolved.
8. The chlorobutanol is then added and stirred until it is completely dissolved.
9. The GLP-1 is added and stirred gently until it dissolved.
10. Check the pH to make sure it is 4.5 ±0.5. Add dilute HCl or dilute NaOH to adjust the pH.
11. Add water to final volume.

**Table 5**

| Reagent | mg/mL | % |
|---|---|---|
| Chlorobutanol, anhydrous | 5.0 | 0.50 |
| Methyl-β-Cyclodextrin | 45 | 4.5 |
| L-α-Phosphatidylcholine Didecanoyl | 1 | 0.1 |

(continued)

| Reagent | mg/mL | % |
|---|---|---|
| Edetate Disodium (EDTA) | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| GLP-1 | 1 | 0.1 |
| Purified Water | | |
| Formulation pH 4.5 ±0.5 Osmolarity ~250 | | |

## EXAMPLE 9

(Prophetic)

**Preparation of an GLP-1 Formulation Free of a Stabilizer that is a Protein**

A second formulation is prepared as above, except the concentration of GLP-1 is 15 mg/mL as shown below in Table 6.

**Table 6**

| Reagent | mg/ml | % |
|---|---|---|
| chlorobutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| GLP-1 | 15 | 0.1 |
| Purified Water | | |
| Formulation pH 4.5 ±0.5 | | |

## EXAMPLE 10

(Prophetic)

**Preparation of Exendin-4 Formulation Free of a Stabilizer that is a Protein**

An exendin-4 formulation suitable for intranasal administration of exendin, which is substantially free of a stabilizer that is a protein is prepared having the formulation listed below.

1. About ¾ of the water is added to a beaker and stirred with a stir bar on a stir plate and the sodium citrate is added until it is completely dissolved.
2. The EDTA is then added and stirred until it is completely dissolved.

3. The citric acid is then added and stirred until it is completely dissolved.
4. The methyl-β-cyclodextrin is added and stirred until it is completely dissolved.
5. The DDPC is then added and stirred until it is completely dissolved.
6. The lactose is then added and stirred until it is completely dissolved.
7. The sorbitol is then added and stirred until it is completely dissolved.
8. The chlorobutanol is then added and stirred until it is completely dissolved.
9. The exendin-4 is added and stirred gently until it dissolved.
10. 11 Check the pH to make sure it is 5.0 $\pm$ 0.25. Add dilute HCl or dilute NaOH to adjust the pH.
11. Add water to final volume.

**Table 7**

| Reagent | mg/mL | % |
|---|---|---|
| chlorobutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| Exendin-4 | 1 | 0.1 |
| Purified Water | | |
| Formulation pH 4.5 $\pm$0.5 Osmolarity -250 | | |

### EXAMPLE 11

A second formulation is prepared as above, except the concentration of exendin-4 is 15 mg/mL as shown below in Table 8.

**Table 8**

| Reagent | mg/ml | % |
|---|---|---|
| chlorobutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| Exendin-4 | 15 | 0.1 |
| Purified Water | | |
| Formulation pH 5 +/- 0.25 | | |

EXAMPLE 12

**Increased Permeability of Fluorescein-labeled Exenatide across a Cellular Barrier using Permeation Enhancers.**

**Samples:**

**Formulation #1:**

    1 mg/mL Fluorescein-exendin 4 (AnaSpec, Inc, San Jose, CA)
    10 mM sodium citrate/citric acid buffer system, pH 4.5
    45 mg/mL methyl-beta-cyclodextrin
    1 mg/mL EDTA
    1 mg/mL DDPC
    25 mM lactose
    100 mM sorbitol
    0.5% chlorobutanol

**Formulation #2** (Saline Formulation)

    1 mg/mL Fluorescein-exendin 4 (AnaSpec, Inc, San Jose, CA)
    10 mM sodium citrate/citric acid buffer system, pH 4.5
    140 mM NaCl

**Methods:**

*Cell Cultures*

The cell line MatTek Corp. (Ashland, MA) was used as the source of normal, human-derived tracheal/bronchial epithelial cells (EpiAirway™ Tissue Model). The cells were provided as inserts grown to confluent on Millipore Milicell-CM filters comprised of transparent hydrophilic Teflon (PTFE). Upon receipt, the membranes were cultured in 1 mL basal media (phenol red-free and hydrocortisone-free Dulbecco's Modified Eagle's Medium (DMEM)) at 37°C with 5% $CO_2$ for 24-48 hours before use. Inserts were feed for each day of recovery.

*Tissue Assays*

Each tissue insert was placed in an individual well containing 1 mL of basal media. On the apical surface of the inserts, 100 ul of test formulation was be applied, and the samples placed on a shaker (~100 rpm) for 1 h at 37 °C. The underlying culture media samples were stored at 4°C for up to 48 hours for lactate dehydrogenase (LDH, cytotoxicity) and sample permeation evaluations. Transepithelial electrical resistence (TER) was measured before and after the 1-h incubation. Following the incubation, the cell inserts were analyzed for cell viability via the mitochondrial dehydrogenase (MDH) assay.

*Measurement of Transepithelial Electrical Resistance (TER)*

TEER measurements was accomplished using the Endohm-12 Tissue Resistance Measurement Chamber connected to the EVOM Epithelial Voltohmmeter (World Precision Instruments, Sarasota, FL) with the electrode leads. The electrodes and a tissue culture blank insert were equilibrated for at least 20 minutes in phosphate buffered solution with the power off prior to checking calibration. The background resistance was measured with 1.5 mL PBS in the Endohm tissue chamber and 250 $\mu$L PBS in the blank insert. For each TER determination, -250 $\mu$L of PBS was added to the insert followed by placement in the Endohm chamber. Resistance is expressed as (resistance measured - blank ) X 0.6 cm$^2$.

*LDH Assay*

The amount of cell death was assayed by measuring the loss of lactate dehydrogenase (LDH) from the cells using a CytoTox 96 Cytoxicity Assay Kit (Promega Corp., Madison, WI). Fresh, cell-free culture medium was used as a blank. Fifty microliters of substrate solution was added to each well and the plates incubated for 30 minutes at room temperature in the dark. Following incubation, 50 μL of stop solution was added to each well and the plates read on an optical density plate reader at 490 nm.

*MTT Assay*

Cell viability was assessed using the MTT assay (MTT-100, MatTek kit). Thawed and diluted MTT concentrate was pipetted (300 μL) into a 24-well plate. Tissue inserts were gently dried, placed into the plate wells, and incubated at 37°C for 3 hours. After incubation, each insert was removed from the plate, blotted gently, and placed into a 24-well extraction plate. The cell culture inserts were then immersed in 2.0 mL of the extractant solution per well (to completely cover the sample). The extraction plate was covered and sealed to reduce evaporation of extractant. After an overnight incubation at room temperature in the dark, the liquid within each insert was decanted back into the well from which it was taken, and the inserts discarded. The extractant solution (200 μL in at least duplicate) was pipetted into a 96-well microtiter plate, along with extract blanks. The optical density of the samples was measured at 550 nm on a plate reader (Molecular Devices, Palo Alto, CA).

*Quantitation of Fluorescein-exenatide permeated across the tissue barrier*

The amount Fluorescein-Exendin 4 that permeated across the cellular barrier *in vitro* was quantitated using a Bio-Tek Microplate Fluorescence Plate Reader, FLC 800 (Bioteck Instruments Inc, Winooski, VT). Basolateral samples form each well were collected after one hour of incubation and read undiluted with the fluorescent plate reader, using a standard made from the same stock of Fluorescein-Exendin 4 and PBS that was used for the permeation experiment. A standard curve was generated over the relevant quantitation range. Excitation used was 485 and emission was 528.

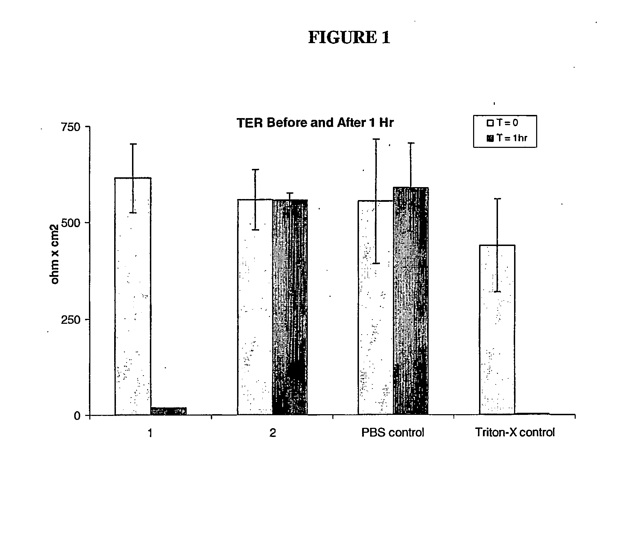

The data shown in Figure 1 indicate that the addition of the permeation enhancers in formulation #1 greatly reduced the TER. In this case, the reduction in TER was comparable to the Triton-X control sample. In contrast, formulation #2, which was absent of any permeation enhancers did not show a reduction in TER, a behavior similar to the PBS (phosphate-buffered-saline) control.

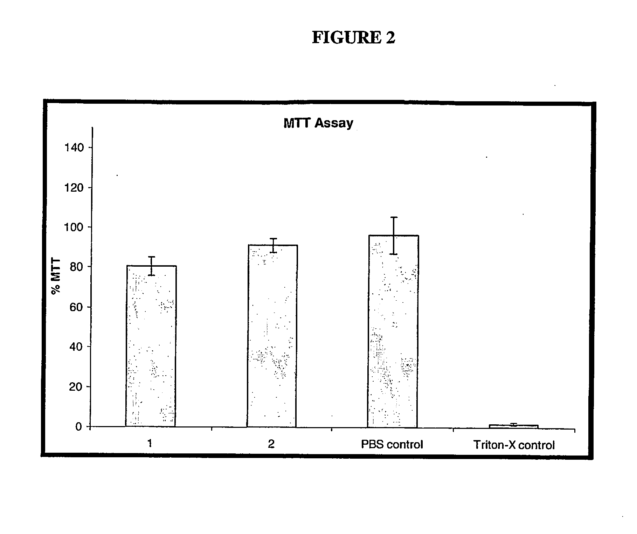

Figure 2 depicts data for the MTT assay (cell viability). It can be seen that both formulation #1 and #2 exhibited a high viability, at least 80% of greater compared to the PBS control. As expected, the Triton control had drastically decreased cell viability.

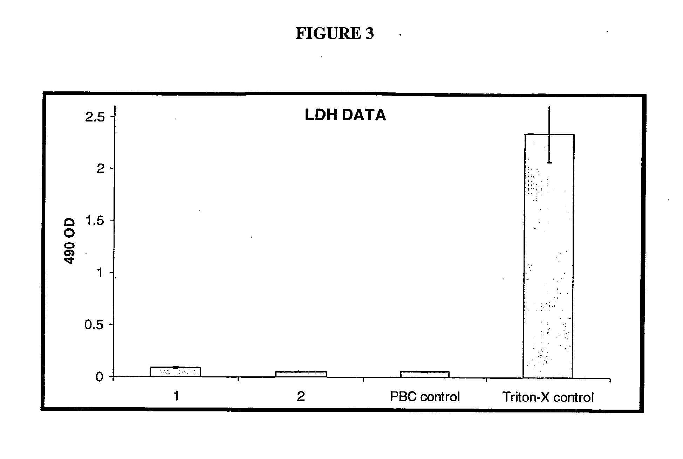

Figure 3 presented data for the LDH assay (cytotoxicty). It can be seen that both formulation #1 and #2 exhibited a low cytotoxicity, similar to the PBS control. As expected, the Triton control had drastically increased cell viability.

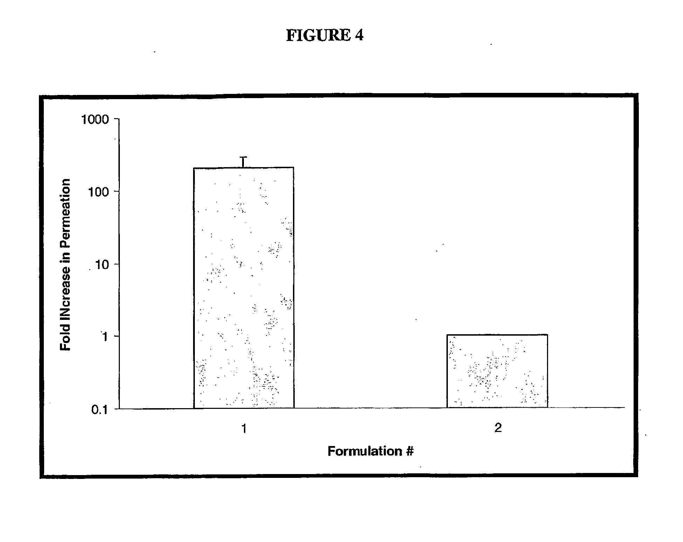

Finally, the data for permeation of Fluorescein -exenatide in formulation #1 and #2 are given in Figure 4 (note the y-axis is shown as a log scale). Formulation #1, with the addition of permeation enhancers to reversibly open the tight junctions, exhibited a dramatically increased permeation compared to simple formulation #2 (over a 200-fold increase).

## Claims

1. A pharmaceutical formulation for intranasal delivery, comprising:

   a glucose-regulating peptide selected from an amylin peptide, pramlintide, a GLP-1 peptide or an extendin;
   water;
   a cyclodextrin; and
   a phospholipid.

2. The formulation according to claim 1, wherein said cyclodextrin is selected from the group consisting of hydroxypropyl-β-cyclodextran, sulfobutylether-β-cyclodextran, and methyl-β-cyclodextrin.

3. The formulation according to claim 2, wherein said cyclodextrin is methyl-β-cyclodextrin.

4. The formulation according to any of claims 1-3, wherein the formulation further comprises a chelating agent; preferably wherein said chelating agent is ethylenediamine tetraacetic acid (EDTA) or ethylene glycol tetraacetic acid (EGTA).

5. The formulation according to claim 1, wherein the glucose-regulating peptide is selected from an amylin peptide of SEQ ID NO: 6-47, pramlintide, glucagon-like peptide-1 (GLP), or exendin-4.

6. The formulation according to claim 1, wherein the phospholipid is L-α-didecanoyl phosphatidylcholine (DDPC).

7. The formulation according to any of claims 1-6, wherein the formulation has a pH of 3 to 6.

8. The formulation according to claim 1, further comprising a hydrogel.

9. The formulation according to any of claims 1-8, wherein the formulation is free of a stabilizer that is a polypeptide or a protein.

10. The formulation according to Claim 1, wherein the glucose-regulating peptide is exendin-4.

11. The formulation according to Claim 1, wherein said formulation contains:

| Reagent | mg/mL | % |
|---|---|---|
| chlorobutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| Exendin-4 | 1 | 0.1 |
| Purified Water | Added to make 100% volume | |
| Formulation pH 4.5 ±0.5 Osmolarity -250 | | |

Or

| Reagent | mg/mL | % |
|---|---|---|
| chlorobutanol, anhydrous | 5.0 | 0.50 |
| Methyl-β-Cyclodextrin | 45 | 4.5 |
| L-α-Phosphatidylcholine Didecanoyl | 1 | 0.1 |
| Edetate Disodium | 1 | 0.1 |
| Sodium Citrate, Dihydrate | 1.62 | 0.162 |
| Citric Acid, Anhydrous | 0.86 | 0.086 |
| α-Lactose monohydrate | 9 | 0.9 |
| Sorbitol | 18.2 | 1.82 |
| Exendin-4 | 15 | 0.1 |
| Purified Water | Added to make 100 % volume | |
| Formulation pH 5 +/- 0.25 | | |

12. The formulation according to any preceding claim, for use in the prevention or treatment of a disease or condition selected from the group consisting of obesity, diabetes mellitus, suppressing appetite, promoting weight loss, decreasing food intake, hyperglycemia, and dyslipidemia.

13. Use of the formulation according to any of claims 1-11, for the manufacture of a medicament for preventing or treating a disease or condition selected from the group consisting of obesity, diabetes mellitus, suppressing appetite, promoting weight loss, decreasing food intake, hyperglycemia, and dyslipidemia.

**Patentansprüche**

1. Pharmazeutische Formulierung zur intranasalen Abgabe, umfassend:

ein Glucose regulierendes Peptid, ausgewählt aus einem Amylin-Peptid, Pramlintid, einem GLP-1-Peptid oder einem Exendin;

Wasser;
ein Cyclodextrin; und
ein Phospholipid.

**2.** Formulierung nach Anspruch 1, wobei das Cyclodextrin aus der Gruppe, bestehend aus Hydroxypropyl-β-cyclodextran, Sulfobutylether-β-cyclodextran und Methyl-β-cyclodextrin, ausgewählt ist.

**3.** Formulierung nach Anspruch 2, wobei das Cyclodextrin Methyl-β-cyclodextrin ist.

**4.** Formulierung nach einem von Ansprüchen 1-3, wobei die Formulierung weiterhin ein Chelatisierungsmittel umfasst; wobei das Chelatisierungsmittel vorzugsweise Ethylen-diamin-tetraessigsäure (EDTA) oder Ethylenglycol-tetraessigsäure (EGTA) ist.

**5.** Formulierung nach Anspruch 1, wobei das Glucose regulierende Peptid aus einem Amylin-Peptid von SEQ ID Nr.: 6-47, Pramlintid, Glucagon-like Peptide-1 (GLP), oder Exendin-4 ausgewählt ist.

**6.** Formulierung nach Anspruch 1, wobei das Phospholipid L-α-Didecanoyl-phosphatidylcholin (DDPC) ist.

**7.** Formulierung nach einem von Ansprüchen 1-6, wobei die Formulierung einen pH-Wert von 3 bis 6 aufweist.

**8.** Formulierung nach Anspruch 1, weiterhin umfassend ein Hydrogel.

**9.** Formulierung nach einem von Ansprüchen 1-8, wobei die Formulierung frei von einem Stabilisator ist, der ein Polypeptid oder ein Protein ist.

**10.** Formulierung nach Anspruch 1, wobei das Glucoseregulierende Peptid Exendin-4 ist.

**11.** Formulierung nach Anspruch 1, wobei die Formulierung enthält:

| Reagenz | mg/ml | % |
|---|---|---|
| Chlorbutanol, wasserfrei | 5,0 | 0,50 |
| Methyl-β-cyclodextrin | 45 | 4,5 |
| L-α-Didecanoyl-phosphatidylcholin | 1 | 0,1 |
| Dinatriumedetat | 1 | 0,1 |
| Natriumcitrat, Dihydrat | 1,62 | 0,162 |
| Zitronensäure, wasserfrei | 0,86 | 0,086 |
| α-Lactose-monohydrat | 9 | 0,9 |
| Sorbit | 18,2 | 1,82 |
| Exendin-4 | 1 | 0,1 |
| Gereinigtes Wasser | Zugegeben, um 100 Volumen-% herzustellen | |
| Formulierung pH 4,5 ± 0,5 Osmolarität ~ 250 | | |

oder

| Reagenz | mg/ml | % |
|---|---|---|
| Chlorbutanol, wasserfrei | 5,0 | 0,50 |
| Methyl-β-cyclodextrin | 45 | 4,5 |
| L-α-Didecanoyl-phosphatidylcholin | 1 | 0,1 |

(fortgesetzt)

| Reagenz | mg/ml | % |
|---|---|---|
| Dinatriumedetat | 1 | 0,1 |
| Natriumcitrat, Dihydrat | 1,62 | 0,162 |
| Zitronensäure, wasserfrei | 0,86 | 0,086 |
| α-Lactose-monohydrat | 9 | 0,9 |
| Sorbit | 18,2 | 1,82 |
| Exendin-4 | 15 | 0, 1 |
| Gereinigtes Wasser | Zugegeben, um 100 Volumen-% herzustellen | |
| Formulierung pH 5 $\pm$ 0,25. | | |

**12.** Formulierung nach einem vorangehenden Anspruch, zur Verwendung bei der Prävention oder Behandlung von einer Krankheit oder einem Zustand, ausgewählt aus der Gruppe, bestehend aus Fettsucht, Diabetes mellitus, verminderndem Appetit, zunehmendem Gewichtsverlust, verringerter Nahrungsaufnahme, Hyperglykämie und Dyslipidämie.

**13.** Verwendung von der Formulierung nach einem von Ansprüchen 1-11, zur Herstellung von einem Medikament zur Prävention oder Behandlung von einer Krankheit oder einem Zustand, ausgewählt aus der Gruppe, bestehend aus Fettsucht, Diabetes mellitus, verminderndem Appetit, zunehmendem Gewichtsverlust, verringerter Nahrungsaufnahme, Hyperglykämie und Dyslipidämie.

**Revendications**

**1.** Formulation pharmaceutique pour une délivrance intranasale, comprenant :

un peptide régulant le glucose choisi parmi un peptide amyline, le pramlintide, un peptide GLP-1 ou une exendine ;
de l'eau ;
une cyclodextrine ; et
un phospholipide.

**2.** Formulation selon la revendication 1, dans laquelle ladite cyclodextrine est choisie dans le groupe constitué d'hydroxypropyl-β-cyclodextrane, sulfobutyléther-β-cyclodextrane et méthyl-β-cyclodextrine.

**3.** Formulation selon la revendication 2, dans laquelle ladite cyclodextrine est la méthyl-β-cyclodextrine.

**4.** Formulation selon l'une quelconque des revendications 1 à 3, où la formulation comprend en outre un agent chélateur ; de préférence où ledit agent chélateur est l'acide éthylène-diamine-tétra-acétique (EDTA) ou l'acide éthylène-glycol-tétra-acétique (EGTA).

**5.** Formulation selon la revendication 1, dans laquelle le peptide régulant le glucose est choisi parmi un peptide amyline de SEQ ID NO : 6 à 47, le pramlintide, le peptide de type glucagon 1 (GLP) ou l'exendine 4.

**6.** Formulation selon la revendication 1, dans laquelle le phospholipide est la L-α-didécanoylphosphatidylcholine (DDPC).

**7.** Formulation selon l'une quelconque des revendications 1 à 6, où la formulation a un pH de 3 à 6.

**8.** Formulation selon la revendication 1, comprenant en outre un hydrogel.

**9.** Formulation selon l'une quelconque des revendications 1 à 8, où la formulation est dépourvue de stabilisant qui est un polypeptide ou une protéine.

**10.** Formulation selon la revendication 1, dans laquelle le peptide régulant le glucose est l'exendine 4.

**11.** Formulation selon la revendication 1, où ladite formulation continent :

| Réactif | mg/ml | % |
|---|---|---|
| Chlorobutanol, anhydre | 5,0 | 0,50 |
| Méthyl-5-cyclodextrine | 45 | 4,5 |
| L-$\alpha$-didécanoyl-phosphatidylcholine | 1 | 0,1 |
| Edétate disodique | 1 | 0,1 |
| Citrate de sodium, dihydraté | 1,62 | 0,162 |
| Acide citrique, anhydre | 0,86 | 0,086 |
| $\alpha$-Lactose monohydraté | 9 | 0,9 |
| Sorbitol | 18,2 | 1,82 |
| Exendine 4 | 1 | 0,1 |
| Eau purifiée | Ajoutée pour constituer un volume de 100 % | |
| pH de la formulation : 4,5 $\pm$ 0,5<br>Osmolarité : ~ 250 | | |

ou

| Réactif | mg/ml | % |
|---|---|---|
| Chlorobutanol, anhydre | 5,0 | 0,50 |
| Méthyl-5-cyclodextrine | 45 | 4,5 |
| L-$\alpha$-didécanoyl-phosphatidylcholine | 1 | 0,1 |
| Edétate disodique | 1 | 0,1 |
| Citrate de sodium, dihydraté | 1,62 | 0,162 |
| Acide citrique, anhydre | 0,86 | 0,086 |
| $\alpha$-Lactose monohydraté | 9 | 0,9 |
| Sorbitol | 18,2 | 1,82 |
| Exendine 4 | 15 | 0,1 |
| Eau purifiée | Ajoutée pour constituer un volume de 100 % | |
| pH de la formulation : 5 +/- 0,25 | | |

**12.** Formulation selon l'une quelconque des revendications précédentes, pour une utilisation dans la prévention ou le traitement d'une maladie ou d'une affection choisie dans le groupe constitué par l'obésité, le diabète sucré, la suppression de l'appétit, la favorisation de la perte de poids, la diminution de la prise d'aliments, l'hyperglycémie et la dyslipidémie.

**13.** Utilisation de la formulation selon l'une quelconque des revendications 1 à 11, pour la fabrication d'un médicament destiné à la prévention ou au traitement d'une maladie ou d'une affection choisie dans le groupe constitué par l'obésité, le diabète sucré, la suppression de l'appétit, la favorisation de la perte de poids, la diminution de la prise d'aliments, l'hyperglycémie et la dyslipidémie.

# FIGURE 1

**FIGURE 2**

## FIGURE 3

## FIGURE 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6506724 B
- US 20030036504 A1
- EP 1083924 B1
- WO 9830231 A1
- WO 0073331 A2
- US 5424286 A
- WO 8706941 A
- WO 9011296 A
- WO 9111457 A
- EP 0708179 A2
- EP 0699686 A2
- US 4839343 A
- US 6190894 B
- US 5908825 A
- US 6004583 A
- US 5681811 A
- US 3972995 A
- US 4259314 A
- US 4680323 A
- US 4740365 A
- US 4573996 A
- US 4292299 A
- US 4715369 A
- US 4876092 A
- US 4855142 A
- US 4250163 A
- US 4226848 A
- US 4948580 A
- US 33093 A
- US 4235871 A
- US 4501728 A
- US 4837028 A
- US 4179337 A
- US 4511069 A
- US 4652441 A
- US 4917893 A
- US 4677191 A
- US 4728721 A
- US 4675189 A
- US 20030223939 A
- US 20030215514 A
- US 20030215512 A
- US 20030209243 A
- US 20030203036 A
- US 20030198601 A
- US 20030183228 A
- US 200301885765 A
- US 20030150454 A
- US 20030124193 A
- US 20030094173 A
- US 20030072740 A
- WO 60532337 A

### Non-patent literature cited in the description

- **Eng, J. et al.** *J. Biol. Chem.,* 1990, vol. 265, 20259-62
- **Eng., J. et al.** *J. Biol. Chem.,* 1992, vol. 267, 7402-05
- **Goke et al.** *J. Biol. Chem.,* 1993, vol. 268, 19650-55
- **Orskov et al.** *Diabetes,* 1993, vol. 42, 658-61
- **D'Alessio et al.** *J. Clin. Invest.,* 1996, vol. 97, 133-38
- **Williams B et al.** *J Clin Encocrinol Metab,* 1996, vol. 81 (1), 327-32
- **Wettergren A et al.** *Dig Dis Sci,* 1993, vol. 38 (4), 665-73
- **Schjoldager B T et al.** *Dig Dis Sci,* 1989, vol. 34 (5), 703-8
- **O'Halloran D J et al.** *J Endocrinol,* 1990, vol. 126 (1), 169-73
- **Thorens.** *Proc. Natl. Acad Sci. USA,* 1992, vol. 89, 8641-45
- **Schmidt et al.** *Diabetologia,* 1985, vol. 28, 704-707
- **Thorton et al.** *Biochemistry,* 1994, vol. 33, 3532-3539
- *U.S Pharmacopeia National Formulary,* 1990, 1857-1859
- **Laursen et al.** *Eur. J. Endocrinology,* 1996, vol. 135, 309-315
- **Fasano et al.** *Proc. Nat. Acad. Sci., U.S.A.,* 1991, vol. 8, 5242-5246